# EUROPÄISCHE PATENTSCHRIFT

(11) **EP 0 811 623 B1**
(45) Veröffentlichungstag und Bekanntmachung des Hinweises auf die Patenterteilung: **04.04.2001**
(21) Anmeldenummer: 97108871.1
(22) Anmeldetag: 03.06.1997
(51) Int. Cl.: C07D 473/04, A61K 31/52

(54) **Xanthinverbindungen mit terminal aminierten Alkinol-Seitenketten**
Xanthine derivatives with end-aminated alkynol side chains
Dérivés de la xanthine avec des chaines alcynoles latérales aminées en bout

(30) Priorität: 07.06.1996 DE 19622734; 11.09.1996 DE 19636882
(43) Veröffentlichungstag der Anmeldung: 10.12.1997
(73) Patentinhaber: HOECHST AKTIENGESELLSCHAFT, 65929 Frankfurt am Main (DE)
(72) Erfinder: Gebert, Ulrich, Dr., 61479 Glashütten (DE); Defossa, Elisabeth, Dr., 65510 Idstein (DE); Heinelt, Uwe, Dr., 65187 Wiesbaden (DE); Rudolphi, Karl, DR., 55116 Mainz (DE); Grome, John, J., Dr., 65207 Wiesbaden (DE)

(56) Entgegenhaltungen:
- EP-A- 0 260 127
- EP-A- 0 369 744
- WO-A-87/00523
- WO-A-94/11001
- CHEMICAL ABSTRACTS, vol. 88, no. 11, 13.März 1978 Columbus, Ohio, US; abstract no. 68922z, XP002038565 & ARCHIV DER PHARMAZIE, Bd. 310, Nr. 11, 1977, WEINHEIM DE, Seiten 888-893, J. REISCH ET AL.:
- CHEMICAL ABSTRACTS, vol. 70, no. 9, 3.März 1969 Columbus, Ohio, US; abstract no. 37787h, XP002038566 & ARZNEIMITTEL FORSCHUNG DRUG RESEARCH., Bd. 18, Nr. 11, 1968, AULENDORF DE, Seiten 1485-1486, J. REISCH:
- : "", ARCH.PHARM.,1977, , , Band 310, Nr. 11, Seiten 888 - 893
- : "", ARZNEIM.-FORCH, 1968, , , Band 18, Nr. 11, Seiten 1485 - 1486

## Beschreibung

Die Erfindung betrifft neue Xanthinderivate mit mindestens einer Alkinol-Seitenkette in 1- oder 7-Stellung des Xanthingerüstes, Verfahren zu deren Herstellung und deren Verwendung als Wirkstoffe in Arzneimitteln insbesondere für die Behandlung und/oder Prophylaxe zerebrovaskulärer Erkrankungen, die durch ischämisch bedingte Schädigung und nachfolgenden nekrotischen Untergang von Nervenzellen (Neuronen) gekennzeichnet sind.

Der postischämische neuronale Zelltod und die durch ihn bedingten fatalen Funktionsausfälle mit entsprechend schwerwiegenden neurologischen und/oder psychischen Symptomen sind das gemeinsame klinische Krankheitsbild einer Vielzahl zerebrovaskulärer Erkrankungen. Hierzu zählen beispielsweise Schlaganfall; transitorische ischämische Attacken (TIA); Multiinfarktdemenz, Demenz des gemischten Typs mit vaskulärer und degenerativer (Alzheimer) Komponente; Rückenmarkschädigungen; Hirntrauma infolge von Kopfverletzungen; und neuronale Schäden nach Herzstillstand, (neonataler) Asphyxie und Reanimation sowie gefäßchirurgischen Eingriffen (z.B. Bypass-Operationen) im Bereich der das Gehirn versorgenden Hauptarterien.

In der klinischen Praxis dominiert der Schlaganfall, synonym auch Gehirn- oder Hirnschlag, Apoplexie, Apoplexia cerebri oder apoplektischer Insult genannt. Er liegt rund 15% aller Todesfälle zugrunde (Pschyrembel, Klinisches Wörterbuch, Walter de Gruyter-Verlag, 255. Auflage, 1986, Seite 105) und steht damit in der Todesursachenstatistik nach Herz- und Krebskrankheiten an dritter Stelle (Pharmazeutische Zeitung 1994, 139/31: 2482 - 2483). Frauen und Männer sind gleichermaßen betroffen, wobei ab dem 6. Dezennium eine drastische Morbiditätszunahme zu verzeichnen ist. Die Inzidenzrate beträgt derzeit weltweit etwa 0,8% der Bevölkerung mit kontinuierlich steigender Prävalenz insbesondere in den industrialisierten Ländern, da hier die mittlere Lebenserwartung stetig zunimmt.

Wird ein Schlaganfall überlebt, so hinterläßt er in der Regel bleibende Schäden, beispielsweise Lähmungen, Sprachstörungen und/oder Krampfanfälle, die eine fortdauernde intensive Pflege der Patienten mit enormem Leidensdruck auch für die Angehörigen und immensem Kostendruck auf das Gesundheitswesen erforderlich machen. So wird der Kostenaufwand für die Behandlung und Nachsorge von Schlaganfallpatienten allein in den USA gegenwärtig auf jährlich 20 Milliarden US Dollar veranschlagt. Darüber hinaus erleiden ungefähr 10% aller überlebenden Schlaganfallopfer im Laufe des ersten Folgejahres erneut einen Hirninfarkt mit erheblich verschlechterter Prognosis vitam.

Mithin stellen Entwicklung und klinische Etablierung einer effektiven medikamentösen Therapie, die sowohl die akute Mortalität als auch das Ausmaß der neurologischen Defizite und die Rezidivrate reduziert und damit die Lebensqualität nach überwundenem Schlaganfall deutlich verbessert, für die pharmazeu-tische Forschung eine gewaltige Herausforderung von sozialmedizinischer Tragweite dar.

Schlaganfallursache ist stets eine mit Sauerstoffmangel einhergehende Kreislaufstörung im Bereich einer umschriebenen Hirnregion. Die klinische Symptomatik wird bestimmt durch Bewußtseinsstörungen bis zum Koma, häufig spastische Hemiplegie, verschiedenste zentrale motorische, sensible und sensorische Ausfallerscheinungen und fokale oder generalisierte Krampfanfälle. Ätiologisch hat man zu unterscheiden zwischen der mit hoher Letalität behafteten Hirnblutung oder Enzephalorrhagie (primär blutiger Insult; etwa 15% der Fälle; häufig als Massenblutung) nach Gefäßruptur vornehmlich der striolentikulären Arterien infolge von Hypertonie, Arteriosklerose oder intrakraniellem Aneurysma als Grundleiden und dem Hirninfarkt oder Enzephalomalazie (primär unblutiger Insult; ca. 85% der Fälle) mit Ausbildung eines ischämischen Erweichungsherdes (Nekrose), verursacht entweder durch funktionelle Ischämie unter anderem infolge einer zumeist kardial bedingten Blutdruckabfallkrise oder vorwiegend durch progrediente oder persistierende Ischämie aufgrund von stenosierenden bzw. obliterierenden Gefäßprozessen arteriosklerotischer, thrombotischer und embolischer Genese im Bereich der extra- und/oder intrakraniellen Arterien mit bevorzugter Lokalisation in der Arteria carotis interna, cerebri media und vertebralis. Die seltene, sich langsam entwickelnde Symptomatik einer Enzephalomalazie bezeichnet man als "progressive stroke".

Als Vorboten eines drohenden Hirninfarkts gelten die häufig rezidivierenden transitorischen ischämischen Attacken (TIA) von 2- bis 15-minütiger Dauer mit passager auftretenden neurologischen Ausfallsymptomen, denen eine vorübergehende, stenotisch bedingte oder durch Mikroembolien verursachte umschriebene Durchblutungsstörung zugrunde liegt und die sich innerhalb von Minuten bis spätestens 24 Stunden unter vollständiger Restitution zurückbilden. Einer wirkungsvollen Behandlung dieser ischämischen Attacken käme daher große Bedeutung für die Prophylaxe des Schlaganfalls zu.

Epidemiologisch gesicherte Risikofaktoren, die die Entstehung zerebraler Ischämien begünstigen, stellen beispielsweise arterielle Hypertonie, Hyperlipidämie, Hyperurikämie, Diabetes mellitus, rheologische Störungen des Blutes, Herzinsuffizienz und die Einnahme von hormonellen Kontrazeptiva dar (Pschyrembel, Klinisches Wörterbuch, Walter de Gruyter-Verlag, 255. Auflage, 1986, Seite 1840).

Die heute praktizierte Therapie der zerebrovaskulären Erkrankungen beschränkt sich auf Maßnahmen ohne direkten Einfluß auf die zerebrale Ischämie (Schweiz. Med. Wochenschr. 1994, 124/45: 2005 - 2012). Therapeutisches Ziel ist allein die Aufrechterhaltung einer ausreichenden Perfusion im noch intakten Randgebiet des Ischämieherdes, um so bestensfalls die fortschreitende Infarzierung des Hirngewebes zu begrenzen. Eine vorrangige Rolle spielen bei gegebener Indikation gefäßchirurgische Maßnahmen wie die intramurale Desobliteration oder die Überbrückung von Gefäßstenosen mittels extra-intrakraniellem Bypass, die allerdings mit einem relativ hohen Operationsrisiko verbunden sind. Insbesondere die derzeit verfügbaren medikamentösen Maßnahmen gestatten keine kausale Behandlung, sondern sind ausschließlich auf die Behebung klinischer Symptome gerichtet. Hierzu zählen in erster Linie die Sicherstellung einer ausreichenden Herzfunktion durch Verabreichung von Digitalisglykosiden und Antiarrhythmika, die Regulierung des Blutdruckes, die Beseitigung von Stoffwechselstörungen vornehmlich im Elektrolyt- und Glukose-Haushalt und die Verhinderung weiterer Thromboseherde durch antithrombotische Therapie mit Acetylsalicylsäure oder Heparin, während Antikoagulantien vom Typ der Vitamin-K-Antagonisten (Cumarine) wegen erhöhter Blutungsgefahr kontraindiziert sind. Daneben wird auch der Ausschaltung vorgenannter Risikofaktoren therapeutische Bedeutung beigemessen.

Die medikamentöse Akutbehandlung der zerebralen Ischämie stellt somit ein noch ungelöstes klinisches Problem dar (Ann. Radiol. 1994, 37/1-2: 132 - 135). Zu diesem Ergebnis kommt auch eine kürzlich publizierte kritische Analyse aller bislang durchgeführten größeren klinischen Therapiestudien (Lancet 1992, 339/8792: 537 - 539), wobei nochmals hervorgehoben wird, daß Mortalitätssenkung und Begrenzung der neurologischen Folgeschäden bei den Überlebenden gleichrangige Bewertungskriterien für den Behandlungserfolg sind.

Von Klinikern werden daher neue, mehr kausal ausgerichtete Therapiekonzepte gefordert. Erfolgversprechende Ansätze hierfür bieten die komplexen pathophysiologischen Prozesse auf vaskulärer und zellulärer Ebene, die in Form von Circuli vitiosi dem progressiven Verlauf der akuten zerebralen Ischämie zugrunde liegen. Nach gegenwärtigem Kenntnisstand ist die pathogenetische Strecke zwischen Zellischämie und Zelltod durch eine Kaskade physiologischer und biochemischer Vorgänge unter Beteiligung einer Vielzahl von Mediatorsystemen gekennzeichnet, die mit Mangelversorgung, Aufzehrung der energiereichen Verbindungen und Zusammenbruch des Energiestoffwechsels beginnt und über exzessive Ausschüttung exzitatorischer Neurotransmitter, wie Glutamat und Aspartat, bei eingeschränkter oder fehlender Wiederaufnahme zum pathologischen Konzentrationsanstieg des intrazellulären Kalziums als Hauptträger der Zytotoxizität führt. Hand in Hand mit der fatalen Störung der Kalziumhomöostase tragen weitere deletäre Prozesse zum Verlust der Zellintegrität bei. Hierzu gehören unter anderem die Aktivierung membranständiger Phospholipasen und des Arachidonsäure-Stoffwechsels unter Bildung freier Fettsäuren und deren Abbau über den Cyclooxygenase- und Lipoxygenase-Reaktionsweg zu Prostaglandinen bzw. Leukotrienen als Entzündungsmediatoren, die Produktion aggressiver Sauerstoffradikale mit ausgeprägtem Zellmembran-schädigendem Potential, der drastische Anstieg der Membranpermeabilität, die Ausbildung vasogener und zytotoxischer Hirnödeme und die durch Kalziumionen getriggerte Proteolyse der zelleigenen Eiweißstrukturen. Da alle diese Mechanismen zeitabhängig sind, besteht zwischen dem Auftreten der Ischämie und dem Zelluntergang eine Latenzzeit von etwa 6 bis höchstens 12 Stunden, und nur in diesem Zeitfenster können medikamentöse Interventionen überhaupt Aussicht auf Erfolg haben (Rev. Med. Interne 1994, 15/5: 350-356).

Neue kausale Therapieversuche konzentrieren sich nun darauf, durch gezielte Eingriffe in die pathogenetische Reaktionskaskade den progredienten Verlauf der akuten zerebralen Ischämie so frühzeitig wie möglich zu unterbrechen und damit den postischämischen neuronalen Zellverlust nachhaltig einzudämmen. Derzeit werden im wesentlichen zwei Strategien verfolgt (Stroke 1990, 21/8 Suppl. I: I-130 - I-131); zum einen die Thrombolyse von thromboembolischen und atherothrombotischen Verschlüssen mit Fibrinolytika, wie Streptokinase, Urokinase oder dem rekombinanten Gewebe-Plasminogen-Aktivator r-tPA, zwecks frühzeitiger Rekanalisation der arteriellen Strombahn und zum anderen die Zytoprotektion, die auf das Überleben der Neuronen unter ischämischen Bedingungen abzielt.

Zu den neuroprotektiven Therapieprinzipien, die besonders pharmakologisch, teilweise aber auch bereits klinisch intensiv untersucht werden, gehören z.B. die Unterdrückung des neuronalen Kalziumeinstroms mit Kalzium-Antagonisten (z.B. Nimodipin, Nicardipin, Flunarizin und Levemopamil), EAA (excitatory amino acid)-Antagonisten (z.B. kompetitiven und nicht-kompetitiven NMDA(N-Methyl-D-Aspartat)- sowie Nicht-NMDA-Antagonisten) oder Gangliosiden (z.B. GM-1); die Blockade der Arachidonsäure-Kaskade sowie die Ausschaltung ihrer schädlichen Stoffwechselprodukte mit Phospholipase-, Cyclooxygenase- und Lipoxygenase-Inhibitoren bzw. PAF(plättchenaktivierender Faktor)-, Thromboxan- und Leukotrien-Antagonisten; die Hemmung der Zellmembranschädigenden Lipidperoxidation mit Sauerstoffradikalfängern (z.B. Superoxiddismutase, Katalase, alpha-Tocopherol, Ascorbinsäure, Ginkgo-Folium, Allopurinol, Tirilazad und Melatonin) oder Schwermetall-Chelatoren (z.B. Deferoxamin); die Begrenzung der Ödemausbreitung mit antiödematösen Wirkstoffen (z.B. Kortikosteroiden); die Herabsetzung der Thromboseneigung mit Antikoagulantien (z.B. Heparin) und Thrombozyten-Aggregationshemmern (z.B. ASS, Ticlopidin, Prostazyklin und dessen stabileren synthetischen Derivaten); und die Unterstützung endogener Schutzfaktoren mit Serotonin-1A-Agonisten (z.B. Urapidil und Ipsapiron), Adenosin-Modulatoren (z.B. Propentofyllin und Vinpocetin) oder neurotrophen Wachstumsfaktoren (z.B. dem Transforming Growth Factor TGF-β1 und dem Brain-Derived Neurotrophic Factor) und deren Freisetzungsaktivatoren (Prog. Neuro-Psychopharmacol. Biol. Psychiatry 1993, 17/1: 21-70; Clin. Neuropharmacol. 1990, 13 Suppl 3: S9-S25). Die größten Erfolgschancen werden dabei naturgemäß einem multifaktoriellen Eingriff in die pathogenetische Reaktionskaskade mit ihrem komplexen Netzwerk von einander wechselseitig amplifizierenden Mediatorsystemen eingeräumt (Drugs 1988, 35/4: 468 - 476), sei es nun durch Kombination verschiedener selektiv wirkender Pharmaka oder vorteilhafter durch ein Monopharmakon mit möglichst breitem pharmakologischem Wirkungsspektrum.

Neben dem bereits erwähnten Propentofyllin (3-Methyl-1-(5-oxohexyl)-7-propylxanthin) sind auch andere Xanthine, wie die in der Natur weitverbreiteten Methylxanthine Theophyllin (1,3-Dimethylxanthin), Theobromin (3,7-Dimethylxanthin) und Koffein (1,3,7-Trimethylxanthin) sowie die synthetischen 1,3,7-Trialkyl-Derivate Pentoxifyllin (3,7-Dimethyl-1-(5-oxohexyl)-xanthin; Drugs & Aging 1995, 7/6: 480-503) und Denbufyllin (1,3-Dibutyl-7-(2-oxopropyl)xanthin), mehr oder weniger breit pharmakologisch und größtenteils auch klinisch untersucht worden, ohne daß sich bislang ein eindeutiger therapeutischer Nutzen bei der Prophylaxe und Behandlung des akuten ischämischen Schlaganfalls nachweisen ließ. Die natürlichen Methylxanthine können im Gegenteil sogar zu einer Verschlechterung der klinischen Situation führen (Schweiz. Rundsch. Med. Prax. 1989, 78/23: 663-666) und sollten daher kontraindiziert sein. Allein Propentofyllin scheint jedoch aufgrund seines exklusiven pharmakologischen Wirkprofils (Gen. Pharmac. 1994, 25/6: 1053-1058; Drug Dev. Res. 1993, 28/3: 438-444) eine gewisse Ausnahmestellung einzunehmen; allerdings sind weitere kontrollierte klinische Studien mit ausreichend großer Patientenzahl erforderlich, um die therapeutische Wertigkeit des Präparates sicher beurteilen zu können (J. Cereb. Blood. Flow Metab. 1993, 13/3: 526-530).

Überraschend wurde nun gefunden, daß die Einführung von AlkinolSeitenketten mit terminaler Aminofunktion in die 1- und/oder 7-Stellung des Xanthingerüstes zu Verbindungen führt, die in klinisch relevanten experimentellen Modellen dem Propentofyllin deutlich überlegen sind und daher ein größeres therapeutisches Potential für Prophylaxe und Behandlung zerebrovaskulärer Erkrankungen haben.

Die Erfindung betrifft somit neue Xanthinverbindungen der Formel I, 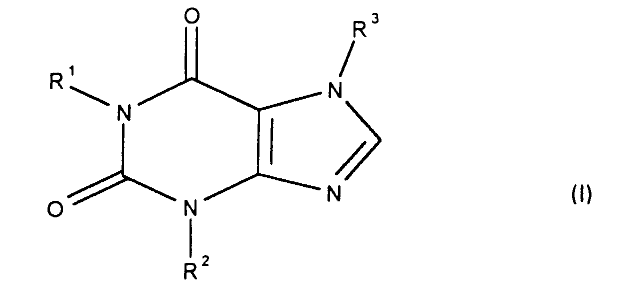 wobei
1)
   - R¹ und R³: für einen Alkinolrest der Formel Ia oder Ib stehen, 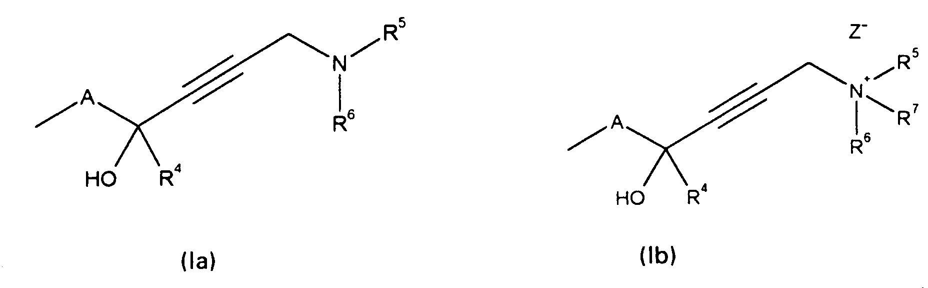
   - R²: für
   a) geradkettiges oder verzweigtes (C₁-C₅)-Alkyl,
   b) (C₃-C₆)-Cycloalkyl oder
   c) (C₄-C₈)-Cycloalkyl-alkyl steht,
   - R⁴: für ein Wasserstoffatom oder (C₁-C₃)-Alkyl steht,
   - R⁵, R⁶ und R⁷: unabhängig voneinander für
   a) ein Wasserstoffatom,
   b) (C₁-C₆)-Alkyl,
   c) (C₃-C₆)-Cycloalkyl,
   d) (C₄-C₈)-Cycloalkyl-alkyl,
   e) Ar-(C₁-C₂)-alkyl oder
   f) Tri-(C₁-C₄)-alkylsilyl stehen, oder
   - R⁵ und R⁶: zusammen mit dem Stickstoffatom, an das sie gebunden sind, einen 4- bis 7-gliedrigen gesättigten Ring bilden, der unsubstituiert oder ein- bis vierfach mit (C₁-C₄)-Alkyl substituiert ist, oder
   - R⁵ und R⁶: zusammen mit dem Stickstoffatom, an das sie gebunden sind, einen 4- bis 7-gliedrigen gesättigten Ring bilden, worin eine -CH₂-Gruppe des Ringes durch einen Rest aus der Gruppe O, S, SO, SO₂ und NR¹³ ersetzt ist,
   - R¹³: für ein Wasserstoffatom, (C₁-C₃)-Alkylcarbonyl oder (C₁-C₄)-Alkyl steht,
   und der Ring unsubstituiert oder ein- bis vierfach mit (C₁-C₄)-Alkyl substituiert ist,
   - A: für unverzweigtes oder verzweigtes (C₁-C₆)-Alkylen steht, und
   - Z⁻: für das Anion einer physiologisch verträglichen anorganischen oder organischen Säure steht, oder
2) R¹ oder R³ für einen Alkinolrest der Formel la oder Ib steht und der andere Rest R³ oder R¹ für
   a) ein Wasserstoffatom oder
   b) R⁸ steht,
      worin R⁸ geradkettiges oder verzweigtes (C₁-C₆)-Alkyl, (C₃-C₆)-Cycloalkyl oder (C₄-C₈)-Cycloalkyl-alkyl bedeutet,
   und R², R⁴, R⁵, R⁶, R⁷, A und Z⁻ wie unter 1) definiert sind.

Bevorzugt sind Verbindungen der Formel I, bei denen nur einer der beiden Reste R¹ oder R³ einen Alkinolrest der Formel la oder Ib darstellt und der andere Rest ein Wasserstoffatom oder R⁸ bedeutet.
Weiterhin bevorzugt sind Verbindungen der Formel I, bei denen R¹ für einen Alkinolrest der Formel la oder Ib und R³ für ein Wasserstoffatom oder R⁸ stehen.
Ferner sind Verbindungen der Formel I bevorzugt, bei denen
- R¹: für einen Alkinolrest der Formel la oder Ib steht,
- R²: für geradkettiges (C₁-C₄)-Alkyl, Cyclopropyl oder Cyclopropylmethyl steht,
- R³: für a) ein Wasserstoffatom oder b) R⁸ steht, worin R⁸ geradkettiges oder verzweigtes (C₁-C₆)-Alkyl, Cyclopropyl oder Cyclopropylmethyl bedeutet,
- R⁴: für ein Wasserstoffatom, Methyl oder Ethyl steht,
- R⁵, R⁶ und R⁷: unabhängig voneinander für ein Wasserstoffatom, (C₁-C₄)-Alkyl, Cyclopropyl, Cyclopropylmethyl oder Benzyl stehen, oder
- R⁵ und R⁶: zusammen mit dem Stickstoffatom, an das sie gebunden sind, einen 5- bis 6-gliedrigen gesättigten Ring aus der Gruppe Morpholin, 4-(C₁-C₃)-Alkylcarbonylpiperazin, 4-(C₁-C₂)-Alkylpiperazin, Piperazin, Piperidin, Pyrrolidin und Thiomorpholin bilden,
- A: für unverzweigtes (C₁-C₅)-Alkylen steht und
- Z⁻: für das Anion einer physiologisch verträglichen anorganischen oder organischen Säure steht.

Besonders bevorzugt sind Verbindungen der Formel I, bei denen
- R¹: für einen Alkinolrest der Formel la oder Ib steht,
- R²: für (C₁-C₄)-Alkyl steht,
- R³: für geradkettiges (C₂-C₄)-Alkyl oder Cyclopropyl steht,
- R⁴: für ein Wasserstoffatom oder Methyl steht,
- R⁵, R⁶ und R⁷: unabhängig voneinander für ein Wasserstoffatom, (C₁-C₄)-Alkyl oder Benzyl stehen, oder
- R⁵ und R⁶: zusammen mit dem Stickstoffatom, an das sie gebunden sind, den Morpholin-, Pyrrolidin-, Piperidin-, 4-Methyl-piperazin- oder 4-Acetylpiperazinring bilden,
- A: für unverzweigtes (C₂-C₄)-Alkylen steht und
- Z⁻: für das Anion einer physiologisch verträglichen anorganischen oder organischen Säure steht.

Insbesondere bevorzugt sind die Verbindungen 1-(8-Diethylamino-5-hydroxy-5-methyl-oct-6-inyl)-3-methyl-, 1-(5-Hydroxy-5-methyl-8-pyrrolidino-oct-6-inyl)-3-methyl-, 3-Butyl-1-(5-hydroxy-5-methyl-8-piperidino-oct-6-inyl)-, 1-(5-Diethylamino-2-hydroxy-2-methyl-pent-3-inyl)-3-propyl-,1-(6-Dimethylamino-3-hydroxy-3-methyl-hex-4-inyl)-3-ethyl-, 1-(7-Diethylamino-4-hydroxy-4-methyl-hept-5-inyl)-3-ethyl-, 1-[8-(4-Acetyl-piperazino)-5-hydroxy-5-methyl-oct-6-inyl]-3-methyl-7-propylxanthin sowie deren physiologisch verträgliche Säureadditionssalze und N,N-Diethyl-N-[4-hydroxy-4-methyl-8-(3-methyl-7-propyl-xanthin-1-yl)-oct-2-inyl]-N-methylammoniumiodid.

Der Ausdruck "(C₄-C₈)-Cycloalkyl-alkyl" definiert solche Alkylreste, die mit (C₃-C₆)-Cycloalkyl substituiert sind, wobei die Summe aller C-Atome kleiner oder gleich 8 ist. Dazu gehören beispielsweise der Cyclopropyl-methyl bis -pentyl-, Cyclobutyl-methyl- bis -butyl-, Cyclopentyl-methyl- bis -propyl- sowie Cyclohexyl-methyl- und -ethyl-Rest. "Ar" bezeichnet Reste, die sich von Benzol oder Naphthalin ableiten. Geeignete 4- bis 7-gliedrige gesättigte Ringe für das Strukturelement -NR⁵R⁶ stellen beispielsweise 4-(C₁-C₄)-Alkylpiperazin, Azetidin, 2,5-Dimethylpyrrolidin, 2,6-Dimethylpiperidin, Morpholin, Perhydroazepin (Azepan), Piperazin, Piperidin, Pyrrolidin, 2,2,6,6-Tetramethylpiperidin, Thiomorpholin und dessen Sulfoxid oder Sulfon dar.

Für die Bildung physiologisch verträglicher Säureadditions- und quartärer Ammoniumsalze gemäß Formel I mit dem Strukturelement der Formel Ib eignen sich unter anderen Halogenwasserstoffsäuren, wie Chlor-, Brom- und lodwasserstoffsäure, Schwefel-, Phosphor-, Essig-, Milch-, Malein-, Fumar-, Oxal-, Wein-, Zitronen-, D-Glucon-, 4-Toluolsulfon-, Methansulfon-, Benzolsulfon- und Cyclohexylsulfamidsäure oder deren jeweiliges Anion Z⁻.

Die erfindungsgemäßen Verbindungen der Formel I weisen aufgrund der sekundären oder tertiären Alkoholstruktur im Alkinolrest der Formel la oder lb immer ein Chiralitätszentrum auf und existieren somit in enantiomeren Formen. Darüber hinaus liegen bei unsymmetrisch verzweigtem Alkylrest in den Positionen von R² und/oder R⁵ bis R⁸ und/oder bei unsymmetrisch verzweigter Alkylengruppe A weitere asymmetrische C-Atome vor, so daß die Verbindungen der Formel I nunmehr in diastereomeren Formen auftreten. Die Erfindung schließt daher sowohl alle stereoisomerenreinen Verbindungen als auch deren Gemische mit ein.

Die Erfindung betrifft weiterhin ein Verfahren zur Herstellung der Verbindungen der Formel I. Gemäß Verfahrensvariante A wird ein 3-Alkylxanthin der Formel II, 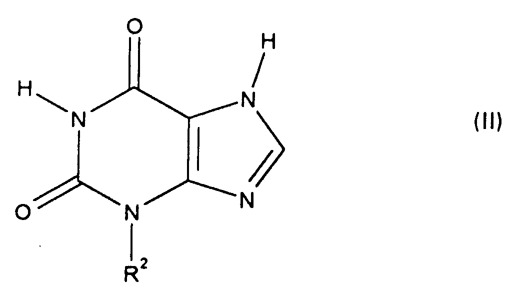 worin R² wie in Formel I definiert ist, ohne Kondensationsmittel oder in Gegenwart eines basischen Kondensationsmittels oder aber ein Salz der Verbindung der Formel II mit einer Verbindung der Formel III, 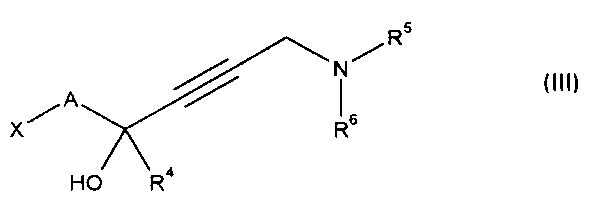 worin X für ein Halogenatom, vorzugsweise Chlor, Brom oder Iod, oder einen Sulfonsäureester- oder Phosphorsäureester-Rest steht und A, R⁴, R⁵ und R⁶ wie in Formel I definiert sind, zu einer Verbindung der Formel Ic 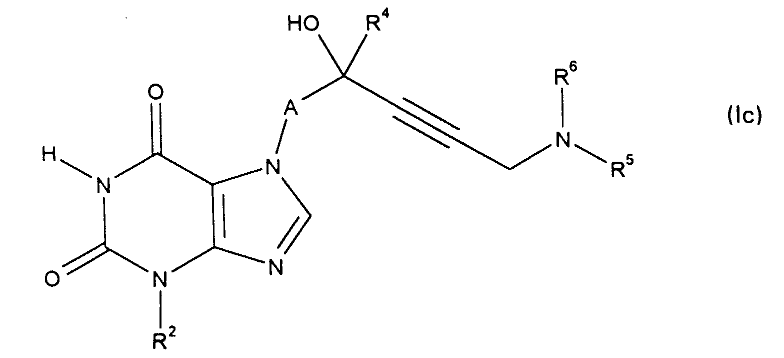 mit einem Alkinolrest der Formel Ia für R³ und einem Wasserstoffatom für R¹ gemäß Formel I umgesetzt und anschließend die Verbindung der Formel Ic ohne Kondensationsmittel oder in Gegenwart eines basischen Kondensationsmittels oder aber ein Salz der Verbindung der Formel Ic entweder wiederum mit einer Verbindung der Formel III zu einer Verbindung der Formel Id 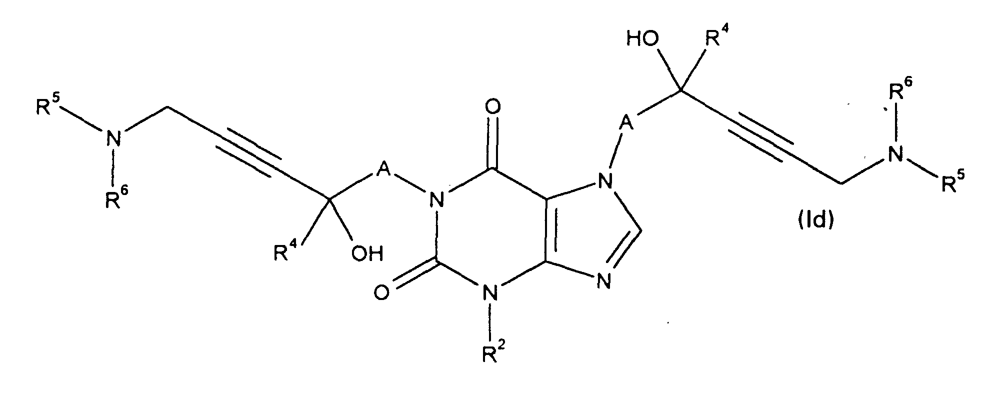 mit zwei gleichen oder verschiedenen Alkinolresten der Formel la für R¹ und R³ gemäß Formel I oder
mit einer Verbindung der Formel IV,

R⁸-X (IV)

worin R⁸ wie in Formel I und X wie in Formel III definiert sind,
zu einer Verbindung der Formel Ie 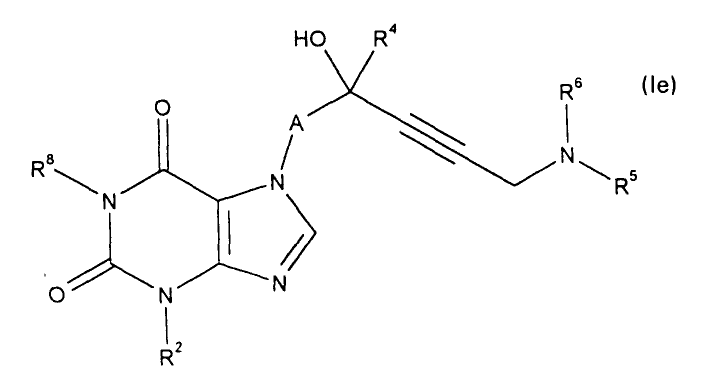 mit dem Rest R⁸ für R¹ und dem Alkinolrest der Formel Ia für R³ gemäß Formel I alkyliert, oder
ein 1,3-Dialkylxanthin der Formel V, 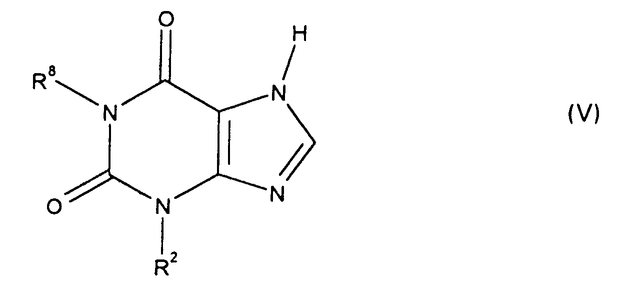 worin R² und R⁸ wie in Formel I definiert sind, ohne Kondensationsmittel oder in Gegenwart eines basischen Kondensationsmittels oder aber ein Salz der Verbindung der Formel V mit einer Verbindung der Formel III zu einer Verbindung der Formel le umgesetzt, oder
ein 3,7-Dialkylxanthin der Formel VI, 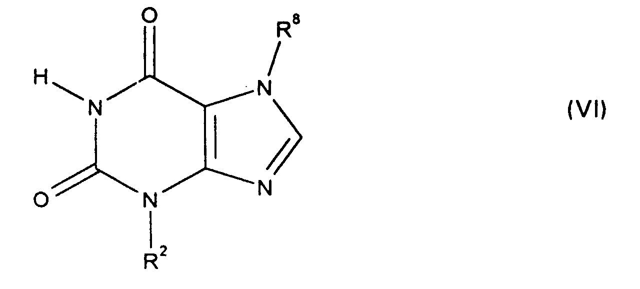 worin R² und R⁸ wie in Formel I definiert sind, ohne Kondensationsmittel oder in Gegenwart eines basischen Kondensationsmittels oder aber ein Salz der Verbindung der Formel VI mit einer Verbindung der Formel III zu einer Verbindung der Formel If 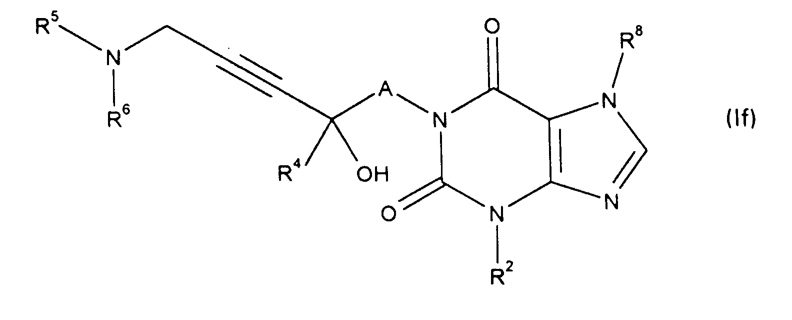 mit einem Alkinolrest der Formel Ia für R¹ und dem Rest R⁸ für R³ gemäß Formel I umgesetzt.

Gemäß Verfahrensvariante B wird eine Verbindung der Formel II, V oder VI analog Verfahrensvariante A mit einer Verbindung der Formel VIII, 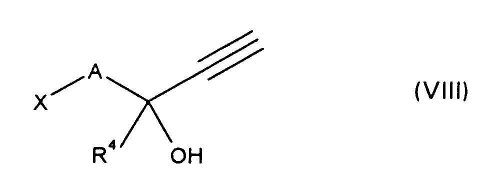 worin A und R⁴ wie in Formel I und X wie in Formel III definiert sind,
zu einer Verbindung der Formel IX 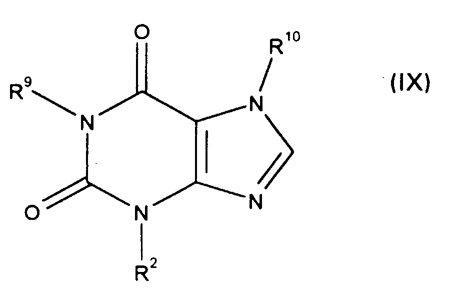 alkyliert, worin entweder R⁹ und R¹⁰ zwei gleiche oder verschiedene Reste der Formel IXa 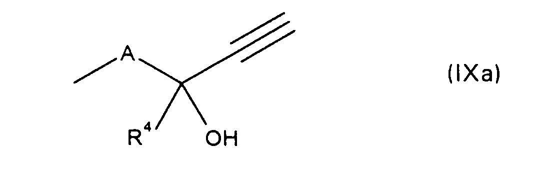 darstellen oder aber nur R⁹ oder R¹⁰ einen Rest der Formel IXa bedeutet und der andere Rest R¹⁰ oder R⁹ ein Wasserstoffatom oder R⁸ darstellt, wobei R², A, R⁴ und R⁸ wie in Formel I definiert sind, und
anschließend die Verbindung der Formel IX unter den Bedingungen der Mannich-Reaktion (RÖMPP Chemie Lexikon, 9. Auflage, Band 4 (1991), Seite 2632) mit Formaldehyd und einem Amin der Formel X, 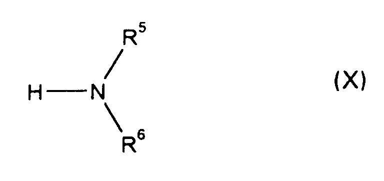 worin R⁵ und R⁶ wie in Formel I definiert sind, an der (oder den) terminalen Ethinylgruppe(n) zu einer Verbindung der Formel Ic, Id, le oder If aminomethyliert.

Gemäß Verfahrensvariante C wird ein 1,3- oder 3,7-di- oder 1,3,7-trisubstituiertes Xanthin der Formel XI, 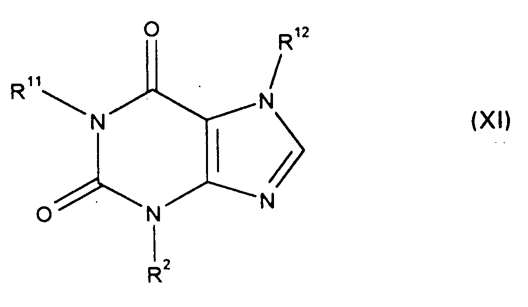 worin entweder R¹¹ und R¹² zwei gleiche oder verschiedene Reste der Formel XIa 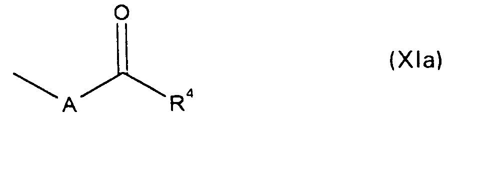 darstellen oder aber nur R¹¹ oder R¹² einen Rest der Formel Xla bedeutet und der andere Rest R¹² oder R¹¹ ein Wasserstoffatom oder R⁸ darstellt, wobei R², A, R⁴ und R⁸ wie in Formel I definiert sind,
mit einer Organometall-Verbindung der Formel XII, 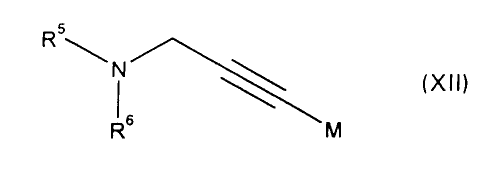 worin R⁵ und R⁶ wie in Formel I definiert sind und M ein Alkalimetall wie Natrium, Kalium oder insbesondere Lithium; Erdalkalimetall wie Kalzium oder insbesondere Magnesium, beispielsweise in Form einer Grignard-Verbindung (-Mg-Halogenid]; oder Schwermetall wie Cer, Kupfer oder Silber; bedeutet, unter reduktiver Alkinylierung der Carbonylgruppe(n) zu einer Verbindung der Formel Ic, Id, Ie, If oder Ig 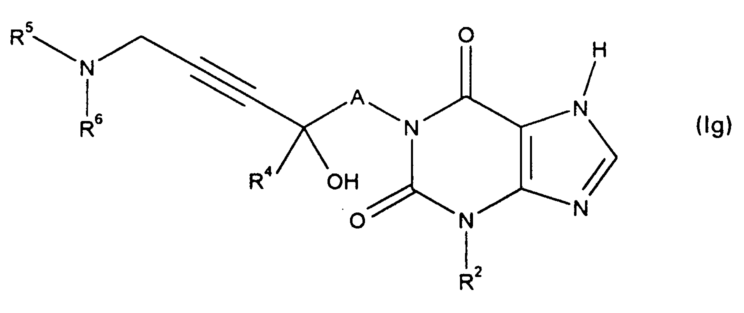 mit einem Alkinolrest der Formel Ia für R¹ und einem Wasserstoffatom für R³ gemäß Formel I umgesetzt.

Gemäß Verfahrensvariante D wird ein Xanthin der Formel XI, worin R¹¹ und/oder R¹² für den Rest der Formel XIa stehen, nach Art der Nef-Reaktion (RÖMPP Chemie Lexikon, 9. Auflage, Band 4 (1991), Seite 2954) entweder mit einem Acetylid der Formel XIII,

HC≡C-M (XIII)

worin M wie in Formel XII definiert ist, oder aber
mit einem endständig geschützten Acetylid der Formel XIV,

R^{a}-C≡C-M (XIV)

worin M wie in Formel XII definiert ist und R^{a} eine nachträglich leicht eliminierbare Abgangsgruppe darstellt, beispielsweise die unter Fluorid-Katalyse abspaltbare Trimethylsilylgruppe (TMS), unter Ethinylierung der Carbonylgruppe(n) zu einer Verbindung der Formel IX umgesetzt, worin R⁹ und/oder R¹⁰ für den Rest der Formel IXa stehen, und anschließend die erhaltene Verbindung IX durch Mannich-Reaktion mit Formaldehyd und einem Amin der Formel X analog Verfahrensvariante B zu einer Verbindung der Formel Ic, Id, Ie, If oder Ig aminomethyliert.

Gemäß Verfahrensvariante E wird eine nach den Verfahrensvarianten A bis D hergestellte Verbindung der Formel Ic, Id, Ie oder If oder eine nach den Verfahrensvarianten C oder D hergestellte Verbindung der Formel Ig, in der R⁵ und/oder R⁶ jeweils ein Wasserstoffatom darstellen, ein- oder zweifach mit einem Oxoderivat (Aldehyd oder Keton) von (C₁-C₆)-Alkanen, (C₃-C₆)-Cycloalkanen, (C₄-C₈)-Cycloalkyl-alkanen oder Ar-(C₁-C₂)-alkanen reduktiv alkyliert.

Gemäß Verfahrensvariante F wird eine nach den Verfahrensvarianten A bis E hergestellte Verbindung mit einer physiologisch verträglichen anorganischen oder organischen Säure HZ in ein Säureadditionssalz der Formel I umgewandelt, wobei R¹ und/oder R³ den Alkinolrest der Formel Ib darstellen, worin R⁷ für ein Wasserstoffatom steht, und R² wie in Formel I definiert ist.

Gemäß Verfahrensvariante G wird eine nach den Verfahrensvarianten A bis E hergestellte Verbindung mit einem Alkylierungsmittel der Formel VII,

R⁷-Z (VII)

wobei R⁷ wie in Formel I mit Ausnahme von Wasserstoff und Z wie in Formel III für X definiert sind, in ein quartäres Ammoniumsalz der Formel I umgewandelt,
wobei R¹ und/oder R³ für den Alkinolrest der Formel Ib stehen und R² wie in Formel I definiert ist.

Gemäß Verfahrensvariante H wird eine nach den Verfahrensvarianten A bis G hergestellte Verbindung chromatographisch oder durch fraktionierende Kristallisation in die reinen Stereoisomeren aufgetrennt.

Die bei den Verfahrensvarianten A bis D als Ausgangsstoffe verwendeten Xanthine der Formel II, V, VI oder XI; Alkylierungsmittel der Formel III, IV, VII oder VIII; Organometall-Verbindungen der Formel XII, XIII oder XIV; und Amine der Formel X sind bekannt oder lassen sich nach bekannten Methoden herstellen.
So können die basisch substituierten Alkinole der Formel III beispielsweise durch metallorganische Synthese erhalten werden, indem man die sterisch nicht gehinderten Halogenaldehyde oder -ketone der Formel Hal-A-CO-R⁴ in einer Aufbaureaktion unter reduktiver Alkinylierung der Carbonylfunktion mit den 2-Propinylamin-Metallverbindungen der Formel XII (R⁵R⁶N-CH₂-C≡C-M), vorzugsweise in Form der Lithium- oder Halogenmagnesium(Grignard)-Verbindungen, unter Standardbedingungen (wie nachfolgend bei den Verfahrensvarianten C und D detaillierter beschrieben) umsetzt.
Gleichartige Reaktion der Halogenaldehyde und -ketone mit Acetyliden der Formel XIII (HC≡C-M) oder XIV (R^{a}-C≡C-M) führt - nach Abspaltung der Schutzgruppe R^{a} bei Einsatz von XIV - zu den Alkinolen der Formel VIII.

Die den Organometall-Verbindungen der Formel XII zugrunde liegenden 2-Propinylamine (R⁵R⁶N-CH₂-C≡CH) lassen sich aus 2-Propinylbromid und den Aminen der Formel X durch unmittelbaren Halogen-Amin-Austausch oder auf dem Umweg über die intermediär erzeugten Metallamide in folgender, aus der Literatur (Tetrahedron 1992, 48/30: 6231 - 6244) bekannten Eintopfreaktion problemlos aufbauen:

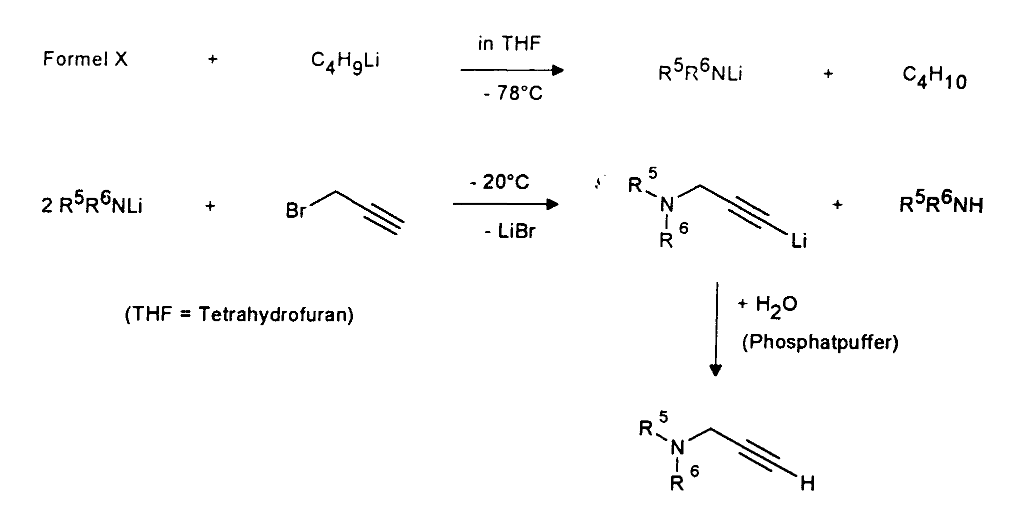

Die Umsetzung der mono- und disubstituierten Xanthinderivate II oder Ic, Ig, V, VI und IX mit den betreffenden Reagenzien der Formel III, IV oder VIII erfolgt gewöhnlich in einem gegenüber den Reaktionsteilnehmern inerten Verteilungs- oder Lösungsmittel. Als solche kommen vor allem dipolare, aprotische Solventien, beispielsweise Dimethylformamid, Dimethylacetamid, N-Methylpyrrolidon, Tetramethylharnstoff, Hexamethylphosphorsäuretriamid oder Dimethylsulfoxid in Frage; es können aber auch Formamid, Acetonitril, Aceton, Butanon oder Alkohole, wie Methanol, Ethylenglykol und dessen Mono- bzw. Di(C₁-C₄)alkylether, Ethanol, Propanol, Isopropanol und die verschiedenen Butanole; Kohlenwasserstoffe, wie Benzol, Toluol oder Xylole; halogenierte Kohlenwasserstoffe, wie Dichlormethan oder Chloroform; Pyridin sowie Mischungen der genannten Lösungsmittel oder deren Gemische mit Wasser Verwendung finden.
Die Reaktion wird zweckmäßig in Gegenwart eines basischen Kondensationsmittels durchgeführt. Hierfür eignen sich beispielsweise Alkali- oder Erdalkalihydroxide, -carbonate, -hydride, -alkoholate und organische Basen, wie Trialkylamine, etwa Triethyl- oder Tributylamin, quartäre Ammonium- oder Phosphoniumhydroxide und vernetzte Harze mit fixierten, gegebenenfalls substituierten Ammonium- oder Phosphoniumgruppen. Die Xanthinderivate können aber auch unmittelbar in Form ihrer gesondert hergestellten Salze, etwa der Alkali-, Erdalkali- oder gegebenenfalls substituierten Ammonium- oder Phosphoniumsalze, eingesetzt werden. Weiterhin lassen sich die Xanthinverbindungen sowohl in Gegenwart der vorgenannten anorganischen Kondensationsmittel als auch in Form ihrer Alkali- oder Erdalkalisalze unter Mithilfe von Phasentransferkatalysatoren, z.B. tertiären Aminen, quartären Ammonium- oder Phosphoniumsalzen oder auch Kronenethern, bevorzugt in einem zweiphasigen System unter den Bedingungen einer Phasentransferkatalyse, problemlos alkylieren. Geeignete, zumeist kommerziell erhältliche Phasentransferkatalysatoren sind unter anderen Tetra-(C₁-C₄)-alkyl- und Methyltrioctylammonium- und -phosphonium-, Methyl-, Myristyl-, Phenyl- und Benzyl-tri-(C₁-C₄)-alkyl- und Cetyltrimethylammonium- oder (C₁-C₁₂)-Alkyl- und Benzyl-triphenylphosphoniumsalze, wobei sich in der Regel jene Verbindungen, die das größere und symmetrischer gebaute Kation besitzen, als effektiver erweisen.
Hierbei wird im allgemeinen bei Reaktionstemperaturen zwischen 0°C und dem Siedepunkt des jeweils verwendeten Reaktionsmediums gearbeitet, vorzugsweise zwischen 20°C und 130°C, gegebenenfalls bei erhöhtem oder vermindertem Druck, aber gewöhnlich bei Atmosphärendruck, wobei die Reaktionszeit von weniger als einer Stunde bis zu mehreren Stunden betragen kann.

Die wahlfreie reduktive Alkylierung von Verbindungen der Formeln Ic bis Ig mit terminaler primärer (R⁵ und R⁶ = H) oder sekundärer (R⁵ oder R⁶ = H) Aminogruppe in der Alkinol-Seitenkette zu sekundären bzw. tertiären Aminen erfolgt durch Umsetzung mit einem der durchweg literaturbekannten Oxoderivate (Aldehyde oder Ketone) von (C₁-C₆)Alkanen, (C₃-C₆)Cycloalkanen, (C₄-C₈)Cycloalkyl-alkanen oder Ar(C₁-C₂)alkanen in Gegenwart eines geeigneten Reduktionsmittels. Die Reduktion der intermediär aus Oxoverbindung und Amin gebildeten Azomethine gelingt beispielsweise mit Ameisensäure und deren Derivaten; bevorzugt ist aber die Hydrierung mit komplexen Metallhydriden, wie Lithiumalanat, Lithium- oder Natriumboranat und insbesondere Natriumcyanoboranat. Hierbei wird zweckmäßig in einem gegenüber den Reaktanten inerten Verteilungs- oder Lösungsmittel, beispielsweise einem Ether, wie Diethylether, Dioxan oder Tetrahydrofuran; einem niederen Alkohol, vorzugsweise Methanol oder Ethanol; Wasser oder deren Mischungen untereinander bei Temperaturen zwischen 20°C und dem Siedepunkt des Reaktionsgemisches gearbeitet.

Die Überführung der Xanthine Ic bis Ig mit den Säuren HZ in die physiologisch verträglichen Säureadditionssalze gehört zum Stand der Technik.
Zur Herstellung der physiologisch verträglichen quartären Ammoniumsalze aus den Xanthinen Ic bis Ig durch Alkylierung mit den Reagenzien der Formel VII, vorzugsweise in Form von Alkylhalogeniden (R⁷Hal), insbesondere den lodiden R⁷I, oder Dialkylsulfaten (R⁷₂SO₄), arbeitet man zweckmäßigerweise in inerten Verteilungs- oder Lösungsmitteln, wie Di(C₁-C₄)alkylethern, cyclischen Ethern, aromatischen oder halogenierten Kohlenwasserstoffen oder Ketonen (z.B. Aceton), oder auch in Gemischen aus diesen Lösungsmitteln oder unter Zusatz von dipolar aprotischen Lösungsmitteln (z.B. Dimethylformamid) bei Temperaturen von 20°C bis zum Siedepunkt des betreffenden Reaktionsmediums, wobei häufig mehrere Stunden bis zur vollständigen Umsetzung benötigt werden. Die quartären Salze fallen hierbei gewöhnlich in kristalliner Form an. Falls erwünscht, läßt sich deren Anion Z⁻ nachträglich mit Hilfe von Anionenaustauschern beliebig variieren.

Die Dreikomponenten-Kondensation nach Mannich zur Aminomethylierung (Weygand/Hilgetag: Organisch-chemische Experimentierkunst, 4. Auflage, 1970, Seiten 990-993) der Verbindung IX an der terminalen, CH-aciden Acetylengruppe kann prinzipiell mit Ammoniak, primären oder vorzugsweise sekundären Aminen der Formel X in Gegenwart von Formaldehyd als Carbonylkomponente (eingesetzt entweder in wäßriger Lösung oder vorteilhafter in fester Form als Paraformaldehyd) unter dem katalytischen Einfluß sowohl von Basen als auch Säuren durchgeführt werden. Bevorzugt ist jedoch das Säure-katalysierte Verfahren, bei dem man die Amine X in Form ihrer Salze, etwa der Hydrochloride oder Acetate, zur Reaktion bringt. Häufig bewährt sich der Zusatz katalytischer Mengen an Metallsalzen, wie etwa Zink(II)-, Eisen(III)- oder insbesondere Kupfer(I)chlorid (J. Med. Chem. 1990, 33: 3182 - 3189).
Als Reaktionsmedium werden im allgemeinen niedere Alkohole, Di-(C₁-C₄)alkylether oder bevorzugt cyclische Ether, vor allem Dioxan, verwendet. Die Reaktionstemperatur liegt gewöhnlich zwischen 20°C und dem Siedepunkt des Reaktionsgemisches, vorzugsweise zwischen 30°C und 70°C, wobei Reaktionszeiten bis zu mehreren Stunden die Regel sind.

Die bei den metallorganischen Umsetzungen gemäß den Verfahrensvarianten C und D als Ausgangsstoffe eingesetzten 3-alkylierten Mono- oder Dioxoalkylxanthine der Formel XI sind unter anderem aus den deutschen Druckschriften DOS 23 30742 und DOS 24 02908 zumeist bekannt oder lassen sich aus den Mono- oder Dialkylxanthinen der Formel II bzw. V oder VI und den Halogenaldehyden oder -ketonen der Formel Hal-A-CO-R⁴, gegebenenfalls auch in Form ihrer offenkettigen oder ringförmigen Acetale bzw. Ketale, unter den vorangehend ausführlich geschilderten Alkylierungsbedingungen leicht herstellen. Dabei sind solche Verbindungen XI, die in der Position von R¹² ein Wasserstoffatom und in der Position von R¹¹ einen Oxoalkylrest der Formel XIa tragen, auf dem Umweg über 1-Oxoalkyl-3,7-dialkylxanthine, in denen der 7-ständige Alkylrest eine leicht eliminierbare Abgangsgruppe etwa in Form der reduktiv entfernbaren Benzylgruppe oder des hydrolytisch abspaltbaren Meth-, Eth-, Prop- oder Butoxymethylrestes darstellt, nach der in der WO 87/00523 detailliert beschriebenen Methodik ohne Schwierigkeiten zugänglich.
Unter den für die Alkinylierung der Carbonylgruppen geeigneten Organometall-Verbindungen der Formel XII, XIII oder XIV nehmen die Lithium- und Halogen-magnesium(Grignard)-Derivate wegen ihrer leichten Zugänglichkeit und Handhabung eine Vorzugsstellung ein. So lassen sich die voranstehend beschriebenen 2-Propinylamine der Formel R⁵R⁶N-CH₂-C≡CH und die einseitig geschützten Acetylene der Formel R^{a}-C≡CH, vorzugsweise Ethinyltrimethylsilan, mit (C₁-C₄)Alkyllithium-Verbindungen, bevorzugt Butyllithium, in einem der nachstehend aufgezählten Lösungsmittel, vornehmlich wasserfreiem Tetrahydrofuran, bei tiefen Temperaturen zwischen -50°C und -80°C oder mit (C₁-C₄)Alkylmagnesiumhalogeniden, beispielsweise Methyl- oder Ethylmagnesiumchlorid oder -bromid, in einem niedrig siedenden Ether, in der Regel Diethylether, bei Siedetemperatur quantitativ zu den Verbindungen der Formel XII bzw. XIV metallieren, die ohne Zwischenisolierung mit den Carbonylverbindungen XI umgesetzt werden. Als Reagenz der Formel XIII kann vorteilhaft kommerziell erhältliches Lithiumacetylid in Form des stabilen Ethylendiamin-Komplexes eingesetzt werden, wobei zur Reaktivitätssteigerung der Zusatz von trockenem Cer(III)chlorid in mindestens stöchiometrischer Menge empfohlen wird (Tetrahedron Letters 1984, 25/38: 4233 - 4236). Die stark nucleophilen metallorganischen Verbindungen sind sehr hydrolyse- und oxidationsempfindlich; ihre sichere Handhabung erfordert daher den konsequenten Ausschluß von Feuchtigkeit und gegebenenfalls ein Arbeiten unter Schutzgasatmosphäre.
Übliche Lösungs- oder Verteilungsmittel für die Alkinylierungsreaktion sind vornehmlich jene, die sich auch für die Herstellung der Organometall-Verbindungen eignen. Als solche kommen vor allem Ether mit einem oder mehreren Ethersauerstoffatomen, beispielsweise Diethyl-, Dipropyl-, Diisopropyl- oder Dibutylether, 1,2-Dimethoxyethan, Tetrahydrofuran, Dioxan, Tetrahydropyran, Furan und Anisol, und aliphatische oder aromatische Kohlenwasserstoffe, wie Petrolether, Cyclohexan, Benzol, Toluol, Xylole, Diethylbenzole und Tetrahydronaphthalin in Frage; es können aber auch tertiäre Amine, wie Triethylamin, oder dipolare, aprotische Solventien, etwa Dimethylformamid, Dimethylacetamid, N-Methylpyrrolidon, Hexamethylphosphorsäuretriamid und Dimethylsulfoxid, sowie Mischungen der genannten Lösungsmittel verwendet werden.
Die Alkinylierungsreaktion wird in der Regel bei Temperaturen zwischen -40°C und + 100°C, vorzugsweise zwischen -20°C und +70°C oder bei Raumtemperatur ohne Außenkühlung, durchgeführt, wobei man üblicherweise die jeweilige metallorganische Verbindung in geringfügigem Überschuß anwendet. Die Reaktionszeiten reichen dabei gewöhnlich von einigen Minuten bis zu mehreren Stunden. Die Zersetzung der gebildeten Alkoholate erfolgt vorzugsweise mit Wasser, wäßriger Ammoniumchlorid-Lösung oder verdünnter Salz- oder Essigsäure.

Die Desilylierung sowohl der aus den Carbonylverbindungen XI durch Umsetzung mit Lithiumtrimethylsilylacetylid (XIV) gewonnenen ethinylständig geschützten Alkinole zu den Zwischenverbindungen der Formel IX als auch der erfindungsgemäßen Verbindungen der Formel I mit N-trialkylsilylierten Alkinolseitenketten kann vorteilhaft durch Methanolyse in Gegenwart katalytischer Mengen Kaliumfluorid vorgenommen werden, die bei Temperaturen zwischen 20°C und dem Siedepunkt des Methanols innerhalb weniger Stunden quantitativ verläuft.

Zur Darstellung der erfindungsgemäßen Verbindungen I in stereoisomerenreiner Form kann man entweder von sterisch einheitlichen Ausgangsstoffen der Formel III oder VIII (gegebenenfalls auch II, IV, V, VI, VII, X und/oder XI) und Zwischenverbindungen der Formel IX ausgehen oder bei den Verfahrensvarianten C und D die Alkinolbildung aus den prochiralen Carbonylverbindungen XI mit den Organometall-Verbindungen XII, XIII oder XIV durch asymmetrische Induktion in Gegenwart chiraler Hilfsstoffe enantioselektiv gestalten.
Bevorzugt ist jedoch die nachträgliche Trennung der stereoisomeren Formen mit Hilfe an sich bekannter Methoden. Da Diastereomere im Gegensatz zu Enantiomeren unterschiedliche physikalische und chemische Eigenschaften aufweisen, bereitet die Trennung ihrer Gemische, beispielsweise durch fraktionierende Kristallisation oder chromatographische Verfahren, in der Regel keine Schwierigkeiten. Demgegenüber erfordert die physikalische Racematspaltung in die enantiomeren Formen (Antipoden) zusätzliche Vorkehrungen; so gelingt die fraktionierende Kristallisation erst nach Bildung diastereomerer Salze mit einer optisch aktiven Säure HZ und die chromatographische Trennung nur bei Verwendung chiraler stationärer Phasen, die zu den Enantiomeren eine unterschiedliche räumliche Affinität zeigen.

Die Alkinole der Formel IX stellen wertvolle Zwischenprodukte zum Aufbau der erfindungsgemäßen Verbindungen der Formel I dar, und weisen eine geringere Wasserlöslichkeit auf.

Die Verbindungen der Formel I eignen sich aufgrund ihrer wertvollen pharmakologischen Eigenschaften in hervorragender Weise für die Verwendung als Wirkstoffe in Arzneimitteln, insbesondere in solchen, die eine effektive kurative und prophylaktische Behandlung von ischämiebedingten zerebrovaskulären Erkrankungen, wie Schlaganfall; transitorischen ischämischen Attacken (TIA); Multiinfarktdemenz; Demenz des gemischten Typs mit vaskulärer und degenerativer (Alzheimer) Komponente; Rückenmarkschädigungen; Hirntrauma infolge von Kopfverletzungen; und neuronalen Schäden nach Herzstillstand, (neonataler) Asphyxie und Reanimation sowie gefäßchirurgischen Eingriffen (z.B. Bypass-Operationen) im Bereich der das Gehirn versorgenden Hauptarterien, gestatten. Dabei können die Verbindungen der Formel I entweder für sich allein, beispielsweise in Form von Mikrokapseln, in Mischungen untereinander oder in Kombination mit geeigneten Trägerstoffen verabreicht werden.

Die Erfindung betrifft folglich auch Arzneimittel, die mindestens eine Verbindung der Formel I als Wirkstoff enthalten.

Ferner betrifft die vorliegende Erfindung einerseits den Einsatz der erfindungsgemäßen Arzneimittel im Rahmen aller gegenwärtig bei zerebrovaskulären Erkrankungen praktizierten Therapieformen (Schweiz. Med. Wochenschr. 1994, 124/45: 2005 -2012), wie der Primärprävention zur Unterdrückung drohender Ischämie-Attacken, der Akutbehandlung zur Begrenzung der Infarzierung des Gewebes nach Ischämie-Eintritt und der Sekundärprophylaxe zur Senkung der Rezidivrate nach überstandener Ischämie-Episode, und andererseits die Anwendung der Arzneimittel in Form von pharmazeutischen Zubereitungen vor allem für die parenterale und orale, gegebenenfalls aber auch rektale oder transdermale Verabreichung.

Geeignete feste oder flüssige galenische Zubereitungsformen sind beispielsweise Granulate, Pulver, Tabletten, Dragees, (Mikro)Kapseln, Sirupe, Emulsionen, Suspensionen, Gele, Präparate mit protrahierter Wirkstoff-Freigabe, Zäpfchen, wirkstoffabgebende Pflaster, Aerosole, Tropfen und vor allem injizierbare Lösungen in Form von Ampullen oder Injektionsflaschen für die Dauerinfusion, bei deren Herstellung üblicherweise Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, physiologische Kochsalzlösung, Alkohole, Glycerin und andere mehrwertige Alkohole (Polyole) genannt.

Vorzugsweise werden die pharmazeutischen Präparate in Dosierungseinheiten hergestellt und verabreicht, wobei jede Einheit als aktiven Bestandteil eine bestimmte Dosis einer Verbindung gemäß Formel I enthält. Bei festen Dosierungseinheiten, wie Tabletten, Kapseln und Suppositorien, kann diese Dosis bis zu 1000 mg, bevorzugt jedoch 100 bis 600 mg, und bei Injektionslösungen in Ampullenform bis zu 300 mg, vorzugsweise aber 20 bis 200 mg, betragen.

Für die Behandlung eines erwachsenen Patienten sind - je nach Wirksamkeit der Verbindungen gemäß Formel I am Menschen und Schweregrad der lebensbedrohlichen Erkrankung - Tagesdosen von 100 bis 5000 mg Wirkstoff, vorzugsweise 300 bis 3000 mg, bei oraler Verabreichung und von 30 bis 3000 mg, bevorzugt 50 bis 2000 mg, bei intravenöser Applikation indiziert. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Zeitintervallen erfolgen.
Bei intravenöser Dauerinfusion beträgt die Tagesdosis 100 bis 5000 mg, vorzugsweise 500 bis 2000 mg, entsprechend einer Infusionsgeschwindigkeit von 0,1 bis 3 mg pro kg Körpergewicht und Stunde (h), bevorzugt von 0,3 bis 1 mg/kg/h.
Bei allen Applikationsformen können jedoch unter Umständen auch höhere oder niedrigere Tagesdosen angebracht sein.

Die Verbindungen der Formel I können auch zusammen mit anderen geeigneten Wirkstoffen, insbesondere mit solchen, die ebenfalls regulierend in die pathogenetische Reaktionskaskade der akuten zerebralen Ischämie eingreifen; z.B. mit Fibrinolytika, Kalzium-Antagonisten, EAA(excitatory amino acids)-Antagonisten, Gangliosiden, Phospholipase-, Cyclooxygenase- und Lipoxygenase-Inhibitoren, PAF(plättchenaktivierender Faktor)-, Thromboxan- und Leukotrien-Antagonisten, Sauerstoffradikalfängern, Schwermetall-Chelatoren, antiödematösen Wirkstoffen, Antikoagulantien, Thrombozytenaggregationshemmern, Serotonin-1A-Agonisten, Adenosin-Modulatoren oder neurotrophen Wachstumsfaktoren und deren Freisetzungsaktivatoren; verabreicht oder bei der Herstellung der galenischen Zubereitungsformen gemeinsam formuliert werden.

Nachfolgend wird anhand repräsentativer Herstellungsbeispiele der Aufbau der nach Strukturgesichtspunkten in Tabelle 1 zusammengefaßten Verbindungen gemäß Formel I näher erläutert. In Tabelle 2 sind die Verbindungen der Formel IX zusammengestellt. Für alle präparativ hergestellten Zwischen- und Endprodukte wurde die Struktur sowohl ¹H-NMR-spektroskopisch als auch durch Elementaranalyse oder Massenspektrum gesichert.

### Beispiel 1: 1-(8-Diethylamino-5-hydroxy-5-methyl-oct-6-inyl)-3-methyl-7-propylxanthin Hydrochlorid

### nach Verfahrensvarianten D und F:

### D1) 1-(5-Hydroxy-5-methyl-hept-6-inyl)-3-methyl-7-propylxanthin

Zu einer Suspension von 75,5 g (0,82 mol) Lithiumacetylid als Ethylendiamin-Komplex in 500 ml Dioxan wurde unter Feuchtigkeitsausschluß und Rühren bei Raumtemperatur eine Lösung von 153,2 g (0,5 mol) 3-Methyl-1-(5-oxohexyl)-7-propylxanthin in 750 ml Dioxan tropfenweise hinzugefügt. Die dabei einsetzende, schwach exotherme Reaktion beendete man durch 6-stündiges Rühren und Erwärmen auf 70°C. Anschließend wurde bei Raumtemperatur mit Wasser versetzt, das organische Lösungsmittel unter vermindertem Druck weitestgehend abdestilliert, die wäßrige Phase ausgiebig mit Chloroform extrahiert, der Extrakt nach dem Trocknen über Natriumsulfat unter vermindertem Druck eingeengt und der Rückstand durch Filtration über eine Kieselgel-Säule im Fließmittel Chloroform gereinigt, wobei 150,4 g (91% der Theorie) öliges Produkt anfielen, das sich allmählich verfestigte und aus Essigsäureethylester unter Zusatz von Petrolether in der Siedehitze umkristallisieren ließ.
Ausbeute: 136,8 g (82% der Theorie); Schmelzpunkt: 98°C
C₁₇H₂₄N₄O₃ (MG = 332,41 g/mol)

| | | | | |
|---|---|---|---|---|
| Analyse | Berechnet | C 61,42% | H 7,28% | N 16,86% |
| | Gefunden | C 61,48% | H 7,37% | N 16,68% |

### D2) 1-(8-Diethylamino-5-hydroxy-5-methyl-oct-6-inyl)-3-methyl-7-propylxanthin

16,6 g (50 mmol) der Zwischenverbindung aus Stufe D1), 1,8 g (60 mmol) Paraformaldehyd, 7,3 g (0,1 mol) Diethylamin und 0,8 g Zink(II)chlorid wurden in 250 ml trockenem Dioxan 5 Stunden unter Rückfluß gerührt. Danach destillierte man das Lösungsmittel unter vermindertem Druck ab und reinigte den rötlichen, öligen Rückstand durch Filtration über eine Kieselgel-Säule im Fließmittel Chloroform/Methanol (19:1).
Ausbeute: 12,6 g (60% der Theorie); hellgelbes Öl
C₂₂H₃₅N₅O₃ (MG = 417,56 g/mol)

### F3) 1-(8-Diethylamino-5-hydroxy-5-methyl-oct-6-inyl)-3-methyl-7-propylxanthin Hydrochlorid

Zur Salzbildung löste man die 12,6 g (30 mmol) der Base aus Stufe D2) in 30 ml 1 N Salzsäure, dampfte unter vermindertem Druck bis zur Trockne ein, trocknete den festen Rückstand über Nacht im Ölpumpenvakuum, nahm mit heißem Ethanol auf, entfärbte die Lösung mit Aktivkohle, filtrierte heiß, versetzte in der Siedehitze mit Diisopropylether bis zur Trübung und ließ das Hydrochlorid unter Erkalten auskristallisieren.
Ausbeute: 11,5 g (84% der Theorie); Schmelzpunkt: 132°C
C₂₂H₃₆ClN₅O₃ (MG = 454,03 g/mol)

| | | | | | |
|---|---|---|---|---|---|
| Analyse | Berechnet | C 58,20% | H 7,99% | Cl 7,81% | N 15,43% |
| | Gefunden | C 58,12% | H 8,24% | Cl 7,84% | N 15,37% |

nach Verfahrensvarianten C und F:

### C1) N,N-Diethyl-2-propinylamin

Eine Mischung aus 100 ml (0,16 mol) einer 1,6 M Lösung von n-Butyllithium in n-Hexan und 100 ml Tetrahydrofuran wurde unter Rühren auf -78°C abgekühlt und bei dieser Temperatur tropfenweise mit 11,7 g (0,16 mol) Diethylamin versetzt; anschließend ließ man auf Raumtemperatur kommen, rührte eine Stunde nach, kühlte erneut auf -20°C ab und tropfte eine Lösung von 9,04 g (76 mmol) 2-Propinylbromid in 50 ml Tetrahydrofuran hinzu. Das Reaktionsgemisch wurde über Nacht bei Raumtemperatur stehen gelassen, dann in eine kalte wäßrige Phosphatpufferlösung eingerührt, mit Chloroform ausgiebig extrahiert, der Extrakt über Natriumcarbonat getrocknet, eingeengt und der Rückstand über eine Kolonne fraktionierend destilliert.
Ausbeute: 6,2 g (73% der Theorie); Siedepunkt: 117°C (Literatur: 119°C)
C₇H₁₃N (MG = 111, 19 g/mol)

### C2) 1-(8-Diethylamino-5-hydroxy-5-methyl-oct-6-inyl)-3-methyl-7-propylxanthin

Zu 5,8 g (52 mmol) in 40 ml trockenem Tetrahydrofuran gelöstem N,N-Diethyl-2-propinylamin aus Stufe C1) tropfte man zwischen -60°C und -65°C in 30 Minuten 32,4 ml (52 mmol) einer 1,6 M Lösung von n-Butyllithium in n-Hexan. Es wurde eine Stunde bei -70°C nachgerührt, dann auf Raumtemperatur erwärmt und während 20 Minuten tropfenweise mit einer Lösung von 12,3 g (40 mmol) 3-Methyl-1-(5-oxohexyl)-7-propylxanthin in 60 ml Tetrahydrofuran versetzt, wobei die Temperatur der Reaktionsmischung bis auf 35°C anstieg. Nach vierstündigem Rühren bei Raumtemperatur fügte man 100 ml kalte 1 N Salzsäure hinzu, schüttelte mehrmals mit Dichlormethan aus, stellte die wäßrige Phase mit Natriumcarbonat alkalisch, extrahierte das Reaktionsprodukt mit Dichlormethan, trocknete über Natriumsulfat und engte unter vermindertem Druck ein. Der ölige Rückstand wurde durch Filtration über eine Kieselgel-Säule im Fließmittel Chloroform/Methanol (19:1) gereinigt.
Ausbeute: 13,9 g (83% der Theorie); farbloses Öl
C₂₂H₃₅N₅O₃ (MG = 417,56 g/mol)

### F3) 1-(8-Diethylamino-5-hydroxy-5-methyl-oct-6-inyl)-3-methyl-7-propylxanthin Hydrochlorid

Die Umwandlung der 13,9 g (33,3 mmol) Base aus Stufe C2) in das Hydrochlorid erfolgte wie bei Verfahrensvariante D beschrieben, wobei allerdings auf die Anwendung von Aktivkohle bei der Umkristallisation aus Ethanol/ Ethanol/Diisopropylether verzichtet werden konnte.
Ausbeute: 13,8 g (91% der Theorie); Schmelzpunkt: 132°C
C₂₂H₃₆ClN₅O₃ (MG = 454,03 g/mol)

| | | | | | |
|---|---|---|---|---|---|
| Analyse | Berechnet | C 58,20% | H 7,99% | Cl 7,81% | N 15,43% |
| | Gefunden | C 58,02% | H 8,26% | Cl 7,94% | N 15,27% |

nach Verfahrensvarianten B und F:

### B1) 1-Chlor-5-hydroxy-5-methyl-6-heptin

200 g (2,17 mol) Lithiumacetylid in Form des Ethylendiamin-Komplexes wurden in 800 ml trockenem Dioxan suspendiert und unter kräftigem Rühren und Eiskühlung mit 269,2 g (2,0 mol) 1-Chlor-5-hexanon in rascher Tropfenfolge versetzt, wobei die Temperatur bis auf 48°C anstieg. Man ließ die exotherme Reaktion unter 3-stündigem Nachrühren ohne weitere Außenkühlung abklingen, fügte vorsichtig 500 ml Wasser hinzu, filtrierte, destillierte den größten Teil des Dioxans unter vermindertem Druck ab, extrahierte die wäßrige Phase ausgiebig mit Chloroform, trocknete den Extrakt über Natriumsulfat, dampfte das Lösungsmittel unter vermindertem Druck ab und unterwarf den Rückstand einer fraktionierenden Destillation.
Ausbeute: 190,2 g (59% der Theorie); Siedepunkt (8 mbar): 87 - 88°C
C₈H₁₃ClO (MG = 160,65 g/mol)

### B2) 1-(5-Hydroxy-5-methyl-hept-6-inyl)-3-methyl-7-propylxanthin

Das Gemisch aus 6,25 g (30 mmol) 3-Methyl-7-propylxanthin, 4,8 g (30 mmol) des Chloralkinols aus Stufe B1) und 4,15 g (30 mmol) Kaliumcarbonat in 150 ml Dimethylformamid wurde 3 Stunden bei 130°C gerührt, anschließend heiß filtriert und unter vermindertem Druck eingeengt. Man nahm den Rückstand in Chloroform auf, wusch zunächst mit 1 N Natronlauge und dann mit Wasser neutral, trocknete über Natriumsulfat, destillierte das Lösungsmittel unter vermindertem Druck ab und kristallisierte aus Essigsäureethylester unter Zusatz von Petrolether in der Siedehitze um.
Ausbeute: 3,6 g (36% der Theorie); Schmelzpunkt: 98°C
C₁₇H₂₄N₄O₃ (MG = 332,41 g/mol)

| | | | | |
|---|---|---|---|---|
| Analyse | Berechnet | C 61,42% | H 7,28% | N 16,86% |
| | Gefunden | C 61,63% | H 7,41% | N 16,87% |

Diese mit dem Produkt des Beispiels 1D1) identische Zwischenverbindung ließ sich durch Mannich-Reaktion mit Paraformaldehyd und Diethylamin und Salzbildung (Beispiele 1D2) und 1F3)) in das Endprodukt überführen.

### nach Verfahrensvarianten A und F:

### A1) 1-Chlor-8-diethylamino-5-hydroxy-5-methyl-6-octin

Zu einer Lösung von 2,0 g (18 mmol) N,N-Diethyl-2-propinylamin (Beispiel 1C1)) in 50 ml Tetrahydrofuran tropfte man langsam bei -78°C 12,37 ml (19,8 mmol) einer 1,6 M Lösung von n-Butyllithium in n-Hexan. Nach einer Stunde bei -78°C wurde auf Raumtemperatur erwärmt und mit 2,42 g (18 mmol) 1-Chlor-5-hexanon versetzt. Man rührte eine Stunde bei Raumtemperatur nach, stellte mit 2 H Salzsäure auf pH 7 und verteilte zwischen 5%iger Natriumhydrogencarbonat-Lösung und Dichlormethan. Die organische Phase wurde über Magnesiumsulfat getrocknet und unter vermindertem Druck vom Lösungsmittel befreit. Ausbeute: 4,38 g (99% der Theorie); öliges Produkt
C₁₃H₂₄ClNO (MG = 245,83 g/mol); ¹H-NMR (DMSO-d₆, 200 MHz): δ = 0,97 (t, 6 H, N(CH₂CH₃)₂); 1,33 (s, 3 H, CH₃); 1,40-1,85 (m, 6 H, CH₂); 2,45 (q, 4 H, N(CH₂CH₃)₂); 3,33 (s, 2 H, NCH₂C≡C); 3,63 (t, 2 H, CH₂Cl); 5,12 (s, 1 H, OH)
Die Substanz konnte ohne weitere Reinigung direkt in die Alkylierungsreaktion gemäß Stufe A2) eingesetzt werden.

### A2) 1-(8-Diethylamino-5-hydroxy-5-methyl-oct-6-inyl)-3-methyl-7-propylxanthin

Zu einer 60°C heißen Lösung von 2,0 g (9,6 mmol) 3-Methyl-7-propylxanthin in 60 ml Dimethylformamid gab man 2,12 g (15,3 mmol) Kaliumcarbonat und rührte eine Stunde bei 60°C. Dann fügte man tropfenweise 3,07 g (12,5 mmol) 1-Chlor-8-diethylamino-5-hydroxy-5-methyl-6-octin aus Stufe A1) hinzu und rührte 12,5 Stunden bei 80°C. Anschließend ließ man auf Raumtemperatur abkühlen, gab Wasser zu und extrahierte dreimal mit tert.-Butyl-methylether. Die organische Phase wurde über Magnesiumsulfat getrocknet, unter vermindertem Druck eingeengt und flash-chromatographisch gereinigt, Dichlormethan/ Methanol = 19/1.
Ausbeute: 2,29 g (57% der Theorie); gelbliches Öl
C₂₂H₃₅N₅O₃ (MG = 417,56 g/mol)
Die Substanz war mit den in Beispiel 1D2) und 1C2) hergestellten Produkten identisch und ließ sich analog Beispiel 1F3) in das Hydrochlorid umwandeln.

### Beispiel 1a: ( + )-1-(8-Diethylamino-5-hydroxy-5-methyl-oct-6-inyl)-3-methyl-7-propylxanthin Hydrochlorid

### Beispiel 1b: (-)-1-(8-Diethylamino-5-hydroxy-5-methyl-oct-6-inyl)-3-methyl-7-propylxanthin Hydrochlorid

### nach Verfahrensvarianten H und F:

Das in Beispiel 1 nach Verfahrensvarianten A, B, C oder D und F hergestellte racemische 1-(8-Diethylamino-5-hydroxy-5-methyl-oct-6-inyl)-3-methyl-7-propylxanthin Hydrochlorid wurde durch Hochdruckflüssigkeitschromatographie (HPLC) an einer Säule (250 x 4,6 mm) mit chiralem Trägermaterial (CSP Chiralpak AD) im Elutionsmittel n-Hexan/2-Propanol (85 + 15) unter Zusatz von 0,1% Diethylamin in die enantiomerenreinen Basen getrennt.
C₂₂H₃₅N₅O₃ (MG = 417,56 g/mol)
(+)-Enantiomer: Retentionszeit 11,61 Minuten; optische Reinheit 100% (-)-Enantiomer: Retentionszeit 14,46 Minuten; optische Reinheit 100%
Die Umwandlung der enantiomeren Basen in die Hydrochloride erfolgte gemäß Beispiel 1F3) bei Verfahrensvariante C.
C₂₂H₃₆ClN₅O₃ (MG = 454,03 g/mol)

| | | |
|---|---|---|
| (+)-Enantiomer 1a | Ausbeute 82%; | Schmelzpunkt 86°C |
| (-)-Enantiomer 1b | Ausbeute 70%; | Schmelzpunkt 89°C |

### Beispiel 2: N,N-Diethyl-N-[4-hydroxy-4-methyl-8-(3-methyl-7-propyl-xanthin-1-yl)-oct-2-inyl]-N-methylammoniumiodid (nach Verfahrensvariante G)

1 g (2,4 mmol) 1-(8-Diethylamino-5-hydroxy-5-methyl-oct-6-inyl)-3-methyl-7-propylxanthin - hergestellt nach Beispiel 1A2), 1C2) oder 1D2) - wurde in 30 ml Diethylether vorgelegt, mit 425 mg (3,0 mmol) Methyliodid versetzt und 20 Stunden bei Raumtemperatur gerührt. Danach gab man nochmals 212 mg (1,5 mmol) Methyliodid hinzu und rührte 2 Stunden unter Rückfluß. Das entstandene Kristallisat wurde abgesaugt, mit Diethylether gewaschen und getrocknet.
Ausbeute: 813 mg (60% der Theorie); Schmelzpunkt: 160°C
C₂₃H₃₈IN₅O₃ (MG = 559,51 g/mol); Massenspektrum: 432 (100%, M⁺)

### Beispiel 3: 1-(6-Dimethylamino-3-hydroxy-3-methyl-hex-4-inyl)-3-methyl-7-propylxanthin Fumarat

### nach Verfahrensvarianten C und F:

### C1) 1-(6-Dimethylamino-3-hydroxy-3-methyl-hex-4-inyl)-3-methyl-7-propylxanthin

4,32 g (52 mmol) N,N-Dimethyl-2-propinylamin, 32,4 ml (52 mmol) n-Butyllithium als 1,6 M Lösung in n-Hexan und 11,1 g (40 mmol) 3-Methyl-1-(3-oxobutyl)-7-propylxanthin wurden in Tetrahydrofuran analog Beispiel 1C2) umgesetzt und aufgearbeitet, wobei man allerdings Chloroform anstelle von Dichlormethan als Extraktionsmittel verwendete.
Ausbeute: 13,2 g (91% der Theorie); öliges Produkt
C₁₈H₂₇N₅O₃ (MG = 361,45 g/mol)

### F2) 1-(6-Dimethylamino-3-hydroxy-3-methyl-hex-4-inyl)-3-methyl-7-propylxanthin Fumarat

Zur Umwandlung der Base in das Fumarat wurden die 13,2 g (36,5 mmol) ölige Substanz aus Stufe C1) in 50 ml Ethanol aufgenommen und mit einer heißen Lösung von 4,24 g (36,5 mmol) Fumarsäure in 100 ml Ethanol versetzt.

Anschließend engte man bis zur beginnenden Trübung ein, kochte auf und ließ das Salz auskristallisieren.
Ausbeute: 14,1 g (81% der Theorie); Schmelzpunkt: 170°C
C₂₂H₃₁N₅O₇ (MG = 477,53 g/mol)

### nach Verfahrensvarianten D und F:

### D1) 1-(3-Hydroxy-3-methyl-pent-4-inyl)-3-methyl-7-propylxanthin

Zu einer Suspension von 36,8 g (0,4 mol) Lithiumacetylid als Ethylendiamin-Komplex und 98,6 g (0,4 mol) wasserfreiem Cer(III)chlorid in einer Mischung aus jeweils 500 ml trockenem Dioxan und Toluol tropfte man in 45 Minuten unter Rühren bei 50°C eine Lösung von 55,7 g (0,2 mol) 3-Methyl-1-(3-oxobutyl)-7-propylxanthin im Gemisch aus jeweils 200 ml Dioxan und Toluol. Dann wurde 7 Stunden bei 50°C nachgerührt, abgekühlt, mit kaltem Wasser versetzt, mit 2 N Salzsäure angesäuert, intensiv mit Chloroform extrahiert, der Extrakt mit Wasser gewaschen, über Natriumsulfat getrocknet, unter vermindertem Druck eingedampft und der Rückstand durch Filtration über eine Kieselgel-Säule im Fließmittel Chloroform/Methanol (50:1) gereinigt, wobei man 35,0 g (58% der Theorie) Feststoff erhielt, der aus Ethanol verlustreich umkristallisiert wurde.
Ausbeute: 18,0 g (30% der Theorie); Schmelzpunkt: 149°C
C₁₅H₂₀N₄O₃ (MG = 304,36 g/mol)

| | | | | |
|---|---|---|---|---|
| Analyse | Berechnet | C 59,20% | H 6,62% | N 18,41% |
| | Gefunden | C 58,72% | H 6,51% | N 18,33% |

Diese Zwischenverbindung ließ sich durch Mannich-Reaktion mit Paraformaldehyd und Dimethylamin Hydrochlorid analog Beispiel 1D2) und anschließende Salzbildung gemäß Beispiel 3F2) zum Endprodukt umsetzen.

### Beispiel 4: 1-(5-Hydroxy-5-methyl-8-pyrrolidino-oct-6-inyl)-3-methyl-7-propylxanthin Fumarat (nach Verfahrensvariante B oder D und F)

Das Gemisch aus 9,97 g (30 mmol) der Zwischenverbindung 1-(5-Hydroxy-5-methyl-hept-6-inyl)-3-methyl-7-propylxanthin aus Beispiel 1D1) oder hergestellt gemäß Beispiel 1B2), 1,02 g (34 mmol) Paraformaldehyd, 2,05 g (34 mmol) Eisessig, 2,42 g (34 mmol) Pyrrolidin und 0,6 g Kupfer(I)chlorid in 150 ml trockenem Dioxan wurde 18 Stunden bei 45°C gerührt, anschließend unter vermindertem Druck eingeengt, in Dichlormethan aufgenommen, dreimal mit je 70 ml 1 N Salzsäure extrahiert, der saure Extrakt mit Natriumcarbonat alkalisch gestellt und das Produkt mit Dichlormethan ausgeschüttelt. Nach dem Trocknen über Natriumsulfat und Eindampfen unter vermindertem Druck fiel die Mannich-Base (C₂₂H₃₃N₅O₃; MG = 415,55 g/mol) als öliges Rohprodukt in nahezu quantitativer Ausbeute an, das man mit 3,5 g (30 mmol) Fumarsäure analog Beispiel 3F2) in das Fumarat überführte.
Ausbeute: 12,4 g (78% der Theorie); Schmelzpunkt: 151°C
C₂₆H₃₇N₅O₇ (MG = 531,62 g/mol)

| | | | | |
|---|---|---|---|---|
| Analyse | Berechnet | C 58,74% | H 7,02% | N 13,17% |
| | Gefunden | C 58,18% | H 6,81% | N 12,68% |

### Beispiel 5: 1-(9-Diethylamino-6-hydroxy-6-methyl-non-7-inyl)-3-methyl-7-propylxanthin Hydrochlorid (nach Verfahrensvarianten D und F)

### D1) 1-(6-Hydroxy-6-methyl-oct-7-inyl)-3-methyl-7-propylxanthin

Unter Stickstoffatmosphäre, Feuchtigkeitsausschluß und Rühren versetzte man 2,55 g (26 mmol) Ethinyltrimethylsilan in 25 ml Tetrahydrofuran bei -60°C bis -70°C in 45 Minuten tropfenweise mit 16,2 ml (26 mmol) 1,6 M n-Butyllithium-Lösung in n-Hexan, rührte eine Stunde bei -70°C nach, ließ auf Raumtemperatur kommen und tropfte in 20 Minuten 6,4 g (20 mmol) 3-Methyl-1-(6-oxo-heptyl)-7-propylxanthin in 20 ml Tetrahydrofuran zu. Dann wurde 4 Stunden bei Raumtemperatur nachgerührt, 50 ml kalte 1 N Salzsäure hinzugefügt, mit Chloroform ausgiebig extrahiert, die organische Phase über Natriumsulfat getrocknet, unter vermindertem Druck eingedampft und der ölige Rückstand durch Filtration über eine Kieselgel-Säule im Fließmittel Chloroform/Methanol (10:1) gereinigt, wobei man 6,8 g (81% der Theorie) des ethinylständig trimethylsilylierten Alkinols C₂₁H₃₄N₄O₃Si (MG = 418,62 g/mol; Schmelzpunkt: 91°C) erhielt.

Zur Desilylierung rührte man eine Lösung von 4,19 g (10 mmol) dieses Produktes in 50 ml Methanol nach Zusatz von 58,1 mg (1 mmol) Kaliumfluorid für 2 Stunden unter Rückfluß. Danach engte man unter vermindertem Druck ein, nahm in Chloroform auf, wusch mit Wasser, trocknete über Natriumsulfat und entfernte das Lösungsmittel unter vermindertem Druck. Der ölige Rückstand kristallisierte nach längerem Stehen durch und wurde in Petrolether ausgerührt.
Ausbeute: 3,2 g (92% der Theorie); Schmelzpunkt: 79°C
C₁₈H₂₆N₄O₃ (MG = 346,44 g/mol)

| | | | | |
|---|---|---|---|---|
| Analyse | Berechnet | C 62,41% | H 7,56% | N 16,17% |
| | Gefunden | C 62,23% | H 7,41% | N 16,41% |

Diese Zwischenverbindung war auch durch Umsetzung des Oxoalkylxanthins mit Lithiumacetylid sowohl analog Beispiel 1D1) als auch in Cer(III)chloridunterstützter Reaktion gemäß Beispiel 3D1) herstellbar, allerdings lagen die Ausbeuten mit 30 bis 50% deutlich niedriger, da sich speziell in diesem Fall die Tendenz des Acetylenmoleküls, beidseitig mit dem Keton unter Bildung des Alkindiols C₃₄H₅₀N₈O₆ (MG = 666,84 g/mol; Schmelzpunkt: 129°C) als Nebenprodukt zu reagieren, besonders störend bemerkbar machte und die Reinisolierung des erwünschten monosubstituierten Produktes außerordentlich verlustreich gestaltete.

### D2) 1-(9-Diethylamino-6-hydroxy-6-methyl-non-7-inyl)-3-methyl-7-propylxanthin

10,4 g (30 mmol) der nach Stufe D1) hergestellten Zwischenverbindung wurden analog Beispiel 4 unter Einsatz von 2,49 g (34 mmol) Diethylamin anstelle von Pyrrolidin der Mannich-Reaktion unterworfen. Das gewonnene ölige Rohprodukt reinigte man durch Filtration über eine Kieselgel-Säule im Fließmittel Chloroform/Methanol (10:1).
Ausbeute: 8,3 g (64% der Theorie); öliges Produkt
C₂₃H₃₇N₅O₃ (MG = 431,59 g/mol)

### F3) 1-(9-Diethylamino-6-hydroxy-6-methyl-non-7-inyl)-3-methyl-7-propylxanthin Hydrochlorid

Die 8,3 g (19,2 mmol) Mannich-Base aus Stufe D2) wurden in Methanol gelöst und mit einer stöchiometrischen Menge methanolischer Salzsäure versetzt. Man destillierte das Lösungsmittel unter vermindertem Druck ab, trocknete den Rückstand im Hochvakuum, digerierte mit trockenem Diethylether und nutschte den Feststoff ab.
Ausbeute: 8,8 g (98% der Theorie); Schmelzpunkt: ca. 100°C
(hygroskopisch); C₂₃H₃₈ClN₅O₃ (MG = 468,05 g/mol)

### Beispiel 6: 1-(6-Dibutylamino-3-hydroxy-3-methyl-hex-4-inyl)-3-methyl-7-propylxanthin Hydrochlorid (nach Verfahrensvarianten C und F)

### C1) 1-(6-Dibutylamino-3-hydroxy-3-methyl-hex-4-inyl)-3-methyl-7-propylxanthin

Zu einer Lösung von 2,09 ml (10,77 mmol) N,N-Dibutyl-2-propinylamin in 20 ml Tetrahydrofuran tropfte man langsam bei -65°C bis -70°C 6,73 ml (10,77 mmol) einer 15%igen Butyllithium-Lösung in n-Hexan. Es wurde eine Stunde bei -60°C bis -65°C gerührt, auf Raumtemperatur erwärmt und eine Lösung von 2,0 g (7,18 mmol) 3-Methyl-1-(3-oxobutyl)-7-propylxanthin in 30 ml Tetrahydrofuran zugegeben. Nach 30 Minuten war die leicht exotherme Reaktion beendet. Es wurde mit 1 N Salzsäure auf pH 5-6 gestellt und zwischen Dichlormethan und Wasser verteilt. Die organische Phase wurde mit Wasser gewaschen, mit Magnesiumsulfat getrocknet und unter vermindertem Druck eingeengt. Das ölige Rohprodukt wurde flash-chromatographisch gereinigt, Dichlormethan/Methanol = 19/0,75.
Ausbeute: 2,37 g (74% der Theorie); Schmelzpunkt: 73°C
C₂₄H₃₉N₅O₃ (MG = 445,61 g/mol)

### F2) 1-(6-Dibutylamino-3-hydroxy-3-methyl-hex-4-inyl)-3-methyl-7-propylxanthin Hydrochlorid

597 mg (1,34 mmol) des in Stufe C1) hergestellten Xanthins wurden in 1,34 ml 1 N Salzsäure gelöst, im Hochvakuum eingeengt, 2 Tage mit Diethylether ausgerührt und filtriert.
Ausbeute: 591 mg (91% der Theorie); Schmelzpunkt: 179°C
C₂₄H₄₀ClN₅O₃ (MG = 482,07 g/mol)
Massenspektrum: 446,5 (100%, M+H); 428,5 (32%)

### Beispiel 7: 1-(6-N-Benzyl-N-methylamino-3-hydroxy-3-methyl-hex-4-inyl)-3-methyl-7-propylxanthin Fumarat (nach Verfahrensvarianten C und F)

### C1) 1-(6-N-Benzyl-N-methylamino-3-hydroxy-3-methyl-hex-4-inyl)-3-methyl-7-propylxanthin

1-(6-N-Benzyl-N-methylamino-3-hydroxy-3-methyl-hex-4-inyl)-3-methyl-7-propylxanthin wurde unter Verwendung von 3-Methyl-1-(3-oxobutyl)-7-propylxanthin und N-Benzyl-N-methyl-2-propinylamin gemäß Beispiel 6C1) als ölige Substanz in 86%iger Ausbeute hergestellt. C₂₄H₃₁N₅O₃ (MG = 437,55 g/mol)

### F2) 1-(6-N-Benzyl-N-methylamino-3-hydroxy-3-methyl-hex-4-inyl)-3-methyl-7-propylxanthin Fumarat

540 mg (1,23 mmol) des in Stufe C1) hergestellten Xanthins wurden in Ethanol gelöst, mit einer heißen Lösung von 146 mg (1,23 mmol) Fumarsäure in Ethanol versetzt und 30 Minuten bei 50°C gerührt. Es wurde im Hochvakuum eingeengt, mit Diethylether ausgerührt und filtriert.
Ausbeute: 570 mg (83% der Theorie); Schmelzpunkt: 104°C
C₂₈H₃₅N₅O₇ (MG = 553,62 g/mol)
Massenspektrum: 438,4 (100%, M + H); 420,4 (87%)

### Beispiel 8: 1-(4-Hydroxy-4-methyl-7-[4-methylpiperazino]-hept-5-inyl)-3-methyl-7-propylxanthin Fumarat

### nach Verfahrensvarianten C und F:

### C1) 4-Methyl-1-(2-propinyl)-piperazin

Zu einer Lösung von 22,2 ml (0,20 mol) N-Methylpiperazin in 100 ml Toluol gab man unter Eiskühlung 11,1 ml (0,10 mol) einer 80%igen 2-Propinylbromid-Lösung in Toluol. Nach 30 Minuten unter Rückfluß wurde das entstandene N-Methylpiperazin-Hydrobromid abgesaugt, mit Toluol gewaschen, das Filtrat je zweimal mit 15%iger Natronlauge und gesättigter Natriumchlorid-Lösung gewaschen, eingeengt und unter Vakuum destilliert.
Ausbeute: 4,19 g (30% der Theorie); Siedepunkt: 100°C/47 mbar (GC: 98,6%)
C₈H₁₄N₂ (MG = 138,21 g/mol)
Massenspektrum: 139,2 (100%, M + H); 138,2 (22%); 101,1 (24%); ¹H-NMR (DMSO-d₆, 300 MHz): δ = 2,10-2,54 (m, 8 H, CH₂); 2,13 (s, 3 H, NCH₃); 3,12 (t, 1 H, C≡CH); 3,23 (d, 2 H, NCH₂C≡C)

### C2) 1-(4-Hydroxy-4-methyl-7-[4-methylpiperazino]-hept-5-inyl)-3-methyl-7-propylxanthin Fumarat

1-(4-Hydroxy-4-methyl-7-[4-methylpiperazino]-hept-5-inyl)-3-methyl-7-propylxanthin wurde unter Verwendung von 3-Methyl-1-(4-oxopentyl)-7-propylxanthin und 4-Methyl-1-(2-propinyl)-piperazin aus Stufe C1) gemäß Beispiel 6C1) als ölige Substanz in 82%iger Ausbeute hergestellt. Die Salzbildung mit Fumarsäure gelang gemäß Beispiel 7F2) in 55%iger Ausbeute.
Schmelzpunkt: 168°C
C₂₆H₃₈N₆O₇ (MG = 546,63 g/mol), Base C₂₂H₃₄N₆O₃ (MG = 430,56 g/mol)
Massenspektrum: 431,4 (100%, M + H); 413,4 (7%)

### nach Verfahrensvariante D:

### D1) 1-(4-Hydroxy-4-methyl-hex-5-inyl)-3-methyl-7-propylxanthin

Diese Zwischenverbindung der Formel IX wurde aus 3-Methyl-1-(4-oxopentyl)-7-propylxanthin sowohl durch Cer(III)chlorid-unterstützte Ethinylierung mit Lithiumacetylid gemäß Beispiel 3D1) in 67%iger Ausbeute als auch durch Umsetzung mit lithiiertem Ethinyltrimethylsilan und anschließender Desilylierung analog Beispiel 5D1) in einer Gesamtausbeute von 69% aufgebaut.
C₁₆H₂₂N₄O₃ (MG = 318,38 g/mol); Schmelzpunkt: 108°C

| | | | | |
|---|---|---|---|---|
| Analyse | Berechnet | C 60,36% | H 6,97% | N 17,60% |
| | Gefunden | C 60,09% | H 7,10% | N 17,39% |

Die Umsetzung dieses Produktes mit N-Methylpiperazin und Paraformaldehyd nach Mannich unter den in Beispiel 4 beschriebenen Reaktionsbedingungen führte ebenfalls zur Titelverbindung vorliegenden Beispiels in Form der Base.

### Beispiel 9: 1-(5-Diethylamino-2-hydroxy-2-methyl-pent-3-inyl)-3-methyl-7-propylxanthin Fumarat (nach Verfahrensvarianten C und F)

1-(5-Diethylamino-2-hydroxy-2-methyl-pent-3-inyl)-3-methyl-7-propylxanthin wurde unter Verwendung von 3-Methyl-1-(2-oxopropyl)-7-propylxanthin und N,N-Diethyl-2-propinylamin gemäß Beispiel 6C1) als ölige Substanz in 48%iger Ausbeute hergestellt. Die Salzbildung mit Fumarsäure gelang gemäß Beispiel 7F2) in 98%iger Ausbeute. Schmelzpunkt: 109°C
C₂₃H₃₃N₅O₇ (MG = 491,56 g/mol), Base C₁₉H₂₉N₅O₃ (MG = 375,48 g/mol)
Massenspektrum: 376,2 (30%, M + H); 358,2 (66%); 285,1 (100%); 150,2 (48%)

### Beispiel 10: 1-(7-Dipropylamino-4-hydroxy-4-methyl-hept-5-inyl)-3-ethyl-7-propylxanthin Hemifumarat (nach Verfahrensvarianten C und F)

### C1) 1-(7-Dipropylamino-4-hydroxy-4-methyl-hept-5-inyl)-3-ethyl-7-propylxanthin

Zu einer Lösung von 1,36 ml (7,8 mmol) N,N-Dipropyl-2-propinylamin in 6 ml Tetrahydrofuran wurden bei -78°C 5,3 ml (8,45 mmol) einer 15%igen Butyllithium-Lösung in n-Hexan langsam zugetropft und eine Stunde bei -78°C gerührt. Nach Erwärmung auf Raumtemperatur gab man eine Lösung von 2,0 g (6,5 mmol) 3-Ethyl-1-(4-oxopentyl)-7-propylxanthin in 8 ml Tetrahydrofuran zu. Nach 7 Stunden bei Raumtemperatur wurde mit 4 N Salzsäure auf pH 6 - 7 gestellt und zwischen 5%iger Natriumhydrogencarbonat-Lösung und Dichlormethan verteilt. Die organische Phase wurde mit Magnesiumsulfat getrocknet, unter vermindertem Druck eingeengt und flash-chromatographisch gereinigt, Dichlormethan/Methanol = 19/1.
Ausbeute: 0,71 g (24% der Theorie), öliges Produkt
C₂₄H₃₉N₅O₃ (MG = 445,62 g/mol)

### F2) 1-(7-Dipropylamino-4-hydroxy-4-methyl-hept-5-inyl)-3-ethyl-7-propylxanthin Hemifumarat

Die Salzbildung zum Hemifumarat gelang mit 1 Äquivalent Fumarsäure gemäß Beispiel 7F2) in 86%iger Ausbeute. Schmelzpunkt: 117°C
C₂₆H₄₁N₅O₅ (MG = 503,65 g/mol)
Massenspektrum: 446,2 (100%, M + H); 329,2 (50%); 307,1 (56%); 100,1 (83%); ¹H-NMR (DMSO-d₆, 200 MHz): δ = 0,79 (t, 6 H, N((CH₂)₂CH₃)₂); 0,83 (t, 3 H, N⁷(CH₂)₂-CH₃); 1,23 (t, 3 H, N³CH₂CH₃); 1,33 (s, 3 H, CH₃); 1,26-1,89 (m, 10 H, CH₂); 2,32 (t, 4 H, N(CH₂CH₂CH₃)₂); 3,31 (s, 2 H, NCH₂C≡C); 3,88 (t, 2 H, N¹-CH₂); 4,03 (q, 2 H, N³-CH₂); 4,20 (t, 2 H, N⁷-CH₂); 5,12 (s, 1 H, OH); 6,61 (s, 1 H, C=CH-COOH); 8,10 (s, 1 H, N=CH)

### Beispiel 11: 1-(8-Dimethylamino-5-hydroxy-5-methyl-oct-6-inyl)-3-ethyl-7-propylxanthin Fumarat (nach Verfahrensvarianten C und F)

1-(8-Dimethylamino-5-hydroxy-5-methyl-oct-6-inyl)-3-ethyl-7-propylxanthin wurde unter Verwendung von 3-Ethyl-1-(5-oxohexyl)-7-propylxanthin und N,N-Dimethyl-2-propinylamin gemäß Beispiel 6C1) als ölige Substanz in 57%iger Ausbeute hergestellt. Die Salzbildung zum Fumarat gelang gemäß Beispiel 7F2) in 51%iger Ausbeute. Schmelzpunkt: 117°C
C₂₅H₃₇N₅O₇ (MG = 519,60 g/mol); Base C₂₁H₃₃N₅O₃ (MG = 403,54 g/mol)
Massenspektrum: 404,2 (100%, M + H); 386,2 (44%); 321,2 (49%)

### Beispiel 12: 3-Ethyl-1-(3-hydroxy-3-methyl-6-pyrrolidino-hex-4-inyl)-7-propylxanthin Fumarat (nach Verfahrensvarianten C und F)

3-Ethyl-1-(3-hydroxy-3-methyl-6-pyrrolidino-hex-4-inyl)-7-propylxanthin wurde unter Verwendung von 3-Ethyl-1-(3-oxobutyl)-7-propylxanthin und N-(2-Propinyl)-pyrrolidin gemäß Beispiel 6C1) als ölige Substanz in 50%iger Ausbeute hergestellt. Salzbildung zum Fumarat erfolgte wie in Beispiel 7F2) in 90%iger Ausbeute. Schmelzpunkt: 133°C
C₂₅H₃₅N₅O₇ (MG = 517,59 g/mol), Base C₂₁H₃₁N₅O₃ (MG = 401,52 g/mol)
Massenspektrum: 402,2 (100%, M + H); 116,9 (65%)

### Beispiel 13: 3,7-Dipropyl-1-(5-hydroxy-5-methyl-8-[4-methylpiperazino]-oct-6-inyl)-xanthin Fumarat (nach Verfahrensvarianten C und F)

3,7-Dipropyl-1-(5-hydroxy-5-methyl-8-[4-methylpiperazino]-oct-6-inyl)-xanthin wurde unter Verwendung von 3,7-Dipropyl-1-(5-oxohexyl)-xanthin und 4-Methyl-1-(2-propinyl)-piperazin aus Beispiel 8C1) gemäß Beispiel 6C1) als ölige Substanz in 71%iger Ausbeute hergestellt. Die Salzbildung zum Fumarat gelang gemäß Beispiel 7F2) in 95%iger Ausbeute. Schmelzpunkt: 98°C
C₂₉H₄₄N₆O₇ (MG = 588,71 g/mol), Base C₂₅H₄₀N₆O₃ (MG = 472,64 g/mol)
Massenspektrum: 473,2 (98%, M + H); 335,1 (95%); 138,9 (100%); 85,1 (67%)

### Beispiel 14: 3-Butyl-1-(5-hydroxy-5-methyl-8-piperidino-oct-6-inyl)-7-propylxanthin Hydrochlorid (nach Verfahrensvarianten C und F)

C1) 3-Butyl-1-(5-hydroxy-5-methyl-8-piperidino-oct-6-inyl)-7-propylxanthin Die Verbindung wurde unter Verwendung von 3-Butyl-1-(5-oxohexyl)-7-propylxanthin und N-(2-Propinyl)-piperidin gemäß Beispiel 6C1) als ölige Substanz in 58%iger Ausbeute hergestellt. C₂₆H₄₁N₅O₃ (MG = 471,65 g/mol)

### F2) 3-Butyl-1-(5-hydroxy-5-methyl-8-piperidino-oct-6-inyl)-7-propylxanthin Hydrochlorid

470 mg (1 mmol) des in Stufe C1) hergestellten Xanthins wurden in Methanol gelöst, mit 1 ml 1 N Salzsäure versetzt, im Hochvakuum eingeengt, mit Aceton ausgerührt und filtriert.
Ausbeute: 450 mg (84% der Theorie), Schmelzpunkt: 177°C
C₂₆H₄₂ClN₅O₃ (MG = 508,11 g/mol)
Massenspektrum: 472,5 (100%, M + H); 454,4 (12%)

### Beispiel 15: 3-Butyl-1-(6-dipropylamino-3-hydroxy-3-methyl-hex-4-inyl)-7-propylxanthin (nach Verfahrensvariante C)

3-Butyl-1-(6-dipropylamino-3-hydroxy-3-methyl-hex-4-inyl)-7-propylxanthin wurde unter Verwendung von 3-Butyl-1-(3-oxobutyl)-7-propylxanthin und N,N-Dipropyl-2-propinylamin gemäß Beispiel 6C1) in 28%iger Ausbeute hergestellt.
Schmelzpunkt: 101°C; C₂₅H₄₁N₅O₃ (MG = 459,64 g/mol)
Massenspektrum: 460,2 (100%; M + H); 442,2 (15%)

### Beispiel 16: 3-Butyl-1-(5-hydroxy-5-methyl-8-morpholino-oct-6-inyl)-7-propylxanthin Hydrochlorid (nach Verfahrensvarianten C und F)

3-Butyl-1-(5-hydroxy-5-methyl-8-morpholino-oct-6-inyl)-7-propylxanthin wurde unter Verwendung von 3-Butyl-1-(5-oxohexyl)-7-propylxanthin und N-(2-Propinyl)-morpholin gemäß Beispiel 6C1) als ölige Substanz in 76%iger Ausbeute hergestellt. Die Salzbildung zum Hydrochlorid gelang gemäß Beispiel 14F2) in 86%iger Ausbeute. Schmelzpunkt: 126°C
C₂₅H₄₀ClN₅O₄ (MG = 510,08 g/mol), Base C₂₅H₃₉N₅O₄ (MG = 473,63 g/mol)
Massenspektrum: 474,3 (100%, M + H); 456,4 (83%)

### Beispiel 17: 7-(8-Diethylamino-5-hydroxy-5-methyl-oct-6-inyl)-3-methyl-1-propylxanthin Hydrochlorid

### nach Verfahrensvarianten C und F:

7-(8-Diethylamino-5-hydroxy-5-methyl-oct-6-inyl)-3-methyl-1-propylxanthin wurde unter Verwendung von 3-Methyl-7-(5-oxohexyl)-1-propylxanthin und N,N-Diethyl-2-propinylamin gemäß Beispiel 6C1) als ölige Substanz in 35%iger Ausbeute hergestellt. Die Salzbildung zum Hydrochlorid gelang gemäß Beispiel 14F2) in 64%iger Ausbeute. Schmelzpunkt: 127°C
C₂₂H₃₆ClN₅O₃ (MG = 454,01 g/mol), Base C₂₂H₃₅N₅O₃ (MG = 417,55 g/mol)
Massenspektrum: 418,3 (100%, M + H); 400,3 (35%)

### nach Verfahrensvarianten B und F:

### B1) 7-(5-Hydroxy-5-methyl-hept-6-inyl)-3-methyl-xanthin

33,2 g (0,2 mol) 3-Methylxanthin in 350 ml Dimethylformamid wurden mit 32,1 g (0,2 mol) 1-Chlor-5-hydroxy-5-methyl-6-heptin aus Beispiel 1B1) in Gegenwart von 13,8 g (0,1 mol) Kaliumcarbonat 6 Stunden bei 120°C gerührt. Danach filtrierte man die heiße Mischung, dampfte unter vermindertem Druck bis zur Trockene ein, nahm in Ethanol auf, versetzte in der Siedehitze mit Diisopropylether bis zur Trübung und ließ unter Erkalten auskristallisieren.
Ausbeute: 38,8 g (67% der Theorie); Schmelzpunkt: 173°C
C₁₄H₁₈N₄O₃ (MG = 290,33 g/mol)

| | | | | |
|---|---|---|---|---|
| Analyse | Berechnet | C 57,92% | H 6,25% | N 19,30% |
| | Gefunden | C 57,62% | H 6,27% | N 19,20% |

### B2) 7-(5-Hydroxy-5-methyl-hept-6-inyl)-3-methyl-1-propylxanthin

19,5 g (67 mmol) der Zwischenverbindung aus Stufe B1), 9,3 g (67 mmol) Kaliumcarbonat und 8,24 g (67 mmol) Propylbromid wurden in 200 ml Dimethylformamid - wie in Stufe B1) beschrieben - umgesetzt. Die Reinigung des öligen Rohproduktes erfolgte durch Filtration über eine Kieselgel-Säule mit Essigsäureethylester als Fließmittel und anschließende Umkristallisation des Feststoffes aus Diisopropylether unter Zugabe von Essigsäureethylester in der Siedehitze bis zur klaren Lösung.
Ausbeute: 15,1 g (68% der Theorie); Schmelzpunkt: 97°C
C₁₇H₂₄N₄O₃ (MG = 332,41 g/mol)

| | | | | |
|---|---|---|---|---|
| Analyse | Berechnet | C 61,42% | H 7,28% | N 16,86% |
| | Gefunden | C 61,20% | H 7,39% | N 16,74% |

Die Produkte der Stufen B1) und B2) sind als Verbindungen der Formel IX der Mannich-Reaktion zugänglich. So lieferte die Umsetzung des Alkinols aus Stufe B2) mit Diethylamin und Paraformaldehyd analog Beispiel 4 und Salzbildung gemäß Beispiel 1F3) ebenfalls die Titelverbindung vorliegenden Beispiels.

### Beispiel 18: 1,7-Bis-(8-diethylamino-5-hydroxy-5-methyl-oct-6-inyl)-3-methylxanthin (nach Verfahrensvariante C)

Die Verbindung wurde unter Verwendung von 1,7-Bis-(5-oxohexyl)-3-methylxanthin und N,N-Diethyl-2-propinylamin gemäß Beispiel 6C1) als ölige Substanz in 30%iger Ausbeute hergestellt. C₃₂H₅₂N₆O₄ (MG = 584,82 g/mol) ¹H-NMR (DMSO-d₆, 300 MHz): δ = 0,93 und 0,94 (2 t, 12 H, N(CH₂CH₃)₂); 1,20-1,62 und 1,71-1,85 (m, 12 H, CH₂); 1,31 (s, 6 H, CH₃); 2,32-2,48 (m, 8 H, N(CH₂CH₃)₂); 3,35 (2 s, 4 H, NCH₂C≡C); 3,42 (s, 3 H, N³CH₃); 3,80-3,90 (m, 2 H, N⁷CH₂); 4,24 (t, 2 H, N¹CH₂); 5,10 und 5,11 (2 s, 2 H, OH); 8,08 (s, 1 H, N = CH)

### Beispiel 19: 1-(7-Dipropylamino-4-hydroxy-4-methyl-hept-5-inyl)-3-methylxanthin

### nach Verfahrensvariante C:

Die Verbindung wurde gemäß Beispiel 6C1) aus 3-Methyl-1-(4-oxopentyl)-xanthin und N,N-Dipropyl-2-propinylamin als ölige Substanz mit 51% Ausbeute hergestellt.
C₂₀H₃₁N₅O₃ (MG = 389,51 g/mol)
Massenspektrum: 390,2 (100%, M + H); 372,2 (47%)
¹H-NMR (DMSO-d₆, 300 MHz): δ = 0,80 (t, 6 H, N(CH₂)₂-CH₃); 1,32-1,88 (m, 8 H, CH₂); 1,32 (s, 3 H, C(OH)CH₃); 2,32 (m br., 4 H, NCH₂-C₂H₅); 3,33 (s, 2 H, NCH₂-C≡C); 3,45 (s, 3 H, N³-CH₃); 3,90 (t, 2 H, N¹-CH₂); 5,12 (s, 1 H, OH); 8,04 (s, 1 H, N=CH); 13,53 (s br., 1 H, N⁷-H)

### nach Verfahrensvariante B:

### B1) 7-Ethoxymethyl-1-(5-hydroxy-5-methyl-hept-6-inyl)-3-methylxanthin

44,84 g (0,2 mol) 7-Ethoxymethyl-3-methylxanthin wurden analog Beispiel 1B2) mit 32,13 g (0,2 mol) 1-Chlor-5-hydroxy-5-methyl-6-heptin aus Beispiel 1B1) umgesetzt und aufgearbeitet.
Ausbeute: 52,3 g (75% der Theorie); Schmelzpunkt: 106°C
C₁₇H₂₄N₄O₄ (MG = 348,41 g/mol)

| | | | | |
|---|---|---|---|---|
| Analyse | Berechnet | C 58,61% | H 6,94% | N 16,08% |
| | Gefunden | C 58,41% | H 7,08% | N 15,97% |

### B2) 1-(5-Hydroxy-5-methyl-hept-6-inyl)-3-methyl-xanthin

41,8 g (0,12 mol) des Alkinols aus Stufe B1) wurden in jeweils 600 ml 1 N Salzsäure und Eisessig 4 Stunden bei 60°C gerührt. Danach wurde unter vermindertem Druck eingeengt, mit 1 N Natronlauge neutralisiert, das Produkt mit Chloroform extrahiert, der Extrakt über Natriumsulfat getrocknet, unter vermindertem Druck eingedampft und der Rückstand nach flash-chromatographischer Reinigung mit Chloroform als mobiler Phase aus Ethanol/Petrolether umkristallisiert.
Ausbeute: 23,6 g (68% der Theorie); Schmelzpunkt: 172°C
C₁₄H₁₈N₄O₃ (MG = 290,33 g/mol)

| | | | | |
|---|---|---|---|---|
| Analyse | Berechnet | C 57,92% | H 6,25% | N 19,30% |
| | Gefunden | C 57,65% | H 6,25% | N 19,33% |

Die Mannich-Reaktion dieser Zwischenverbindung mit Dipropylamin und Paraformaldehyd gemäß Beispiel 4 führte ebenfalls zur Titelverbindung vorliegenden Beispiels.

### Beispiel 20: 3-Cyclopropyl-1-(8-diethylamino-5-hydroxy-5-methyl-oct-6-inyl)-7-propylxanthin Hydrochlorid (nach Verfahrensvarianten C und F)

3-Cyclopropyl-1-(8-diethylamino-5-hydroxy-5-methyl-oct-6-inyl)-7-propylxanthin wurde gemäß Beispiel 6C1) aus 3-Cyclopropyl-1-(5-oxohexyl)-7-propylxanthin und N,N-Diethyl-2-propinylamin als ölige Substanz in 89%iger Ausbeute hergestellt. Die Salzbildung zum Hydrochlorid gelang gemäß Beispiel 6F2) in 93%iger Ausbeute. Schmelzpunkt: 146°C
C₂₄H₃₈ClN₅O₃ (MG = 480,06g/mol), Base: C₂₄H₃₇N₅O₃ (MG = 443,60 g/mol)
Massenspektrum: 444,3 (100%; M + H); 426,3 (41%); 253,1 (21%)

### Beispiel 21: 1-(8-Diethylamino-5-hydroxy-oct-6-inyl)-3-methyl-7-propylxanthin Hydrochlorid (nach Verfahrensvarianten C und F)

### C1) 1-(8-Diethylamino-5-hydroxy-oct-6-inyl)-3-methyl-7-propylxanthin

Unter Argon und Rühren tropfte man bei -78°C zu einer Lösung aus 676 µl (4,9 mmol) N,N-Diethyl-2-propinylamin in 4 ml Tetrahydrofuran langsam 2,93 ml (4,68 mmol) einer 15%igen Butyllithium-Lösung in n-Hexan. Es wurde eine Stunde bei dieser Temperatur gerührt, auf Raumtemperatur erwärmt und eine Lösung aus 1,05 g (3,6 mmol) 3-Methyl-1-(5-oxopentyl)-7-propylxanthin in 5 ml Tetrahydrofuran langsam zugegeben. Nach 1,5 Stunden war die Reaktion beendet. Es wurde mit 4 N Salzsäure neutralisiert, das Tetrahydrofuran im Vakuum abgezogen, der Rückstand mit Dichlormethan aufgenommen, mit gesättigter Natriumhydrogencarbonat-Lösung gewaschen, über Magnesiumsulfat getrocknet, das Trockenmittel abfiltriert und unter vermindertem Druck das Lösungsmittel entfernt. Das Rohprodukt wurde mit dem Laufmittelgemisch Dichlormethan/Methanol/gesättigte Ammoniak-Lösung = 19/1/0,05 flashchromatographisch gereinigt.
Ausbeute: 1,1 g (76% der Theorie); gelbliches Öl
C₂₁H₃₃N₅O₃ (MG = 403,53 g/mol)

### F2) 1-(8-Diethylamino-5-hydroxy-oct-6-inyl)-3-methyl-7-propylxanthin Hydrochlorid

430 mg (1,07 mmol) des in Stufe C1) hergestellten Xanthins wurden in 1,07 ml 1 N Salzsäure gelöst, im Hochvakuum eingeengt, mit Pentan versetzt und 3 Tage gerührt. Nach dem Abziehen des Pentans wurde mit Diethylether versetzt und weitere vier Wochen bis zur vollständigen Kristallisation gerührt. Der Ether wurde abgezogen, und der Rückstand erneut 10 Minuten mit Pentan gerührt. Nach Abziehen des Pentans wurde der Rückstand nochmals mit Pentan behandelt, das anschließend am Rotationsverdampfer und dann im Hochvakuum vollständig entfernt wurde. Das Produkt blieb als weißer Feststoff zurück. Ausbeute: 460 mg (98% der Theorie); Schmelzpunkt: 65°C
C₂₁H₃₄ClN₅O₃ (MG = 439,99 g/mol)
Massenspektrum: 404,3 (100%, M + H)

### Beispiel 22: 1-(8-Amino-5-hydroxy-5-methyl-oct-6-inyl)-3-methyl-7-propylxanthin Hydrochlorid (nach Verfahrensvarianten C und F)

### C1) N,N'-Bis-(trimethylsilyl)-2-propinylamin

Unter Argon und Rühren tropfte man bei -78°C zu einer Lösung aus 95 ml Ether und 95 ml (152 mmol) einer 15%igen Butyllithium-Lösung in n-Hexan langsam 32 ml (154 mmol) Hexamethyldisilazan. Man ließ die Mischung auf Raumtemperatur kommen, rührte eine Stunde, kühlte auf -20°C ab und tropfte langsam 8,24 ml (73 mmol) einer 80%igen Lösung aus 2-Propinylbromid in Toluol zu. Nach der Zugabe wurde das Kältebad entfernt und noch 5 Stunden bei Raumtemperatur gerührt. Anschließend wurde das Reaktionsgemisch in 200 ml Phosphat-Puffer gegeben, der sich aus 7,36 g Kaliumdihydrogenphosphat, 5,81 g Dinatriumhydrogenphosphat und 200 ml Wasser zusammensetzte. Der Niederschlag wurde abgesaugt, die Phasen getrennt, die organische Phase mit Wasser gewaschen und über Natriumcarbonat getrocknet, das Trockenmittel abfiltriert, das Lösungsmittel am Rotationsverdampfer entfernt und der Rückstand durch zweimalige Vakuumdestillation fraktioniert.
Ausbeute: 9,26 g (63% der Theorie); Siedepunkt: 50°C/4 mbar
C₉H₂₁NSi₂ (MG = 199,45 g/mol)
¹H-NMR (DMSO-d₆, 250 MHz): δ = 0,11 (s, 18 H, C[Si(CH₃)₃]₂); 3,00 (t, 1 H, C≡CH); 3,50 (d, 2 H, NCH₂C≡C)

### C2) 1-(8-Amino-5-hydroxy-5-methyl-oct-6-inyl)-3-methyl-7-propylxanthin

Unter Argon und Rühren tropfte man bei -40°C zu einer Lösung aus 9,26 g (46,5 mmol) N,N-Bis-(trimethylsilyl)-2-propinylamin aus Stufe C1 in 50 ml Tetrahydrofuran langsam 29 ml (46,5 mmol) einer 15%igen Butyllithium-Lösung in n-Hexan. Anschließend wurde auf Raumtemperatur erwärmt, erneut auf -40°C abgekühlt und eine Lösung aus 14,25 g (46,5 mmol) 3-Methyl-1-(5-oxohexyl)-7-propylxanthin in 40 ml Tetrahydrofuran langsam zugegeben. Nach Entfernen des Kältebads wurde 6 Stunden bei Raumtemperatur gerührt. Dann wurde das Reaktionsgemisch in eine 0°C kalte, gesättigte Ammoniumchlorid-Lösung gegeben, die wäßrige Phase mit Ether extrahiert, die organische Phase über Natriumsulfat getrocknet, das Trockenmittel abfiltriert und unter vermindertem Druck das Lösungsmittel entfernt. Das Rohprodukt wurde zunächst mit dem Laufmittelgemisch Dichlormethan/Methanol/gesättigte Ammoniak-Lösung = 19/1,5/2,5 und dann mit 9/1/2,5 flash-chromatographiert.
Ausbeute: 9,79 g (58% der Theorie); gelbliches Öl
C₁₈H₂₇N₅O₃ (MG = 361,45 g/mol)
Die Verbindung ist ebenfalls durch direkte Umsetzung mit 2-Propinylamin zugänglich:
Unter Argon und Rühren tropfte man bei -78°C zu einer Lösung aus 630 µl (9,1 mmol) 2-Propinylamin in 20 ml Tetrahydrofuran langsam 5,3 ml (8,49 mmol) einer 15%igen Butyllithium-Lösung in n-Hexan. Es wurde eine Stunde bei dieser Temperatur gerührt, auf Raumtemperatur erwärmt und eine Lösung aus 2,0 g (6,53 mmol) 3-Methyl-1-(5-oxohexyl)-7-propylxanthin in 5 ml Tetrahydrofuran langsam zur inzwischen gelben Suspension gegeben. Nach 3 Stunden war die Reaktion zum Stillstand gekommen. Es wurde mit 2 N Salzsäure und 5%iger Natriumhydrogencarbonat-Lösung neutralisiert, mit Dichlormethan extrahiert, über Magnesiumsulfat getrocknet, das Trockenmittel abfiltriert und unter vermindertem Druck das Lösungsmittel entfernt. Das Rohprodukt wurde mit dem Laufmittelgemisch Dichlormethan/Methanol/gesättigte Ammoniak-Lösung = 19/1/0,02 flash-chromatographisch gereinigt. Da die erhaltenen 1,4 g Produkt immer noch leicht verunreinigt waren, wurden diese erneut flash-chromatographiert (Laufmittelgemisch Dichlormethan/Methanol/ gesättigte Ammoniak-Lösung = 19/1,5/0,02).
Ausbeute: 1,01g (43%); gelbliches Öl

### F3) 1-(8-Amino-5-hydroxy-5-methyl-oct-6-inyl)-3-methyl-7-propylxanthin Hydrochlorid

940 mg (2,6 mmol) des in Stufe C2) hergestellten Xanthins wurden in 2,6 ml 1 N Salzsäure gelöst, im Hochvakuum eingeengt, mit Pentan versetzt und 3 Wochen gerührt. Der entstandene Feststoff wurde abgesaugt. Der dabei bereits wieder zerfließende Anteil (hygroskopisch) wurde zurück in den Kolben gegeben, in wenig Wasser gelöst, im Vakuum getrocknet, erneut mit Pentan versetzt und gerührt. Nach zwei Tagen wurde das Pentan abgezogen, und der zurückbleibende pulvrige Feststoff vom nicht kristallisierten Produkt getrennt. Ausbeute: 587 mg (57% der Theorie) weißer Feststoff; Schmelzpunkt: 80°C 345 mg (33% der Theorie) nicht kristallisiertes Produkt
C₁₈H₂₈ClN₅O₃ (MG = 397,91 g/mol), Base: C₁₈H₂₇N₅O₃ (MG = 361,45 g/mol)
Massenspektrum: 362,3 (7% M + H); 344,2 (59%); 209,0 (100%)

### Beispiel 23: 1-(8-Ethylamino-5-hydroxy-5-methyl-oct-6-inyl)-3-methyl-7-propylxanthin (nach Verfahrensvariante C)

### C1) N-Ethyl-2-propinylamin

Bei -78°C wurden 33 ml (0,5 mol) Ethylamin in einen ausgeheizten und mit Argon gespülten Kolben einkondensiert. Nach Erwärmen auf 0°C wurden 5,57 ml (50 mmol) einer 80%igen Lösung von 2-Propinylbromid in Toluol über 45 Minuten langsam zugetropft. Im Gaschromatogramm zeigte sich nach einer Stunde vollständige Umsetzung. Das überschüssige Ethylamin wurde nach Entfernen des Eisbads mit Stickstoff ausgetrieben, der Rückstand mit einem Gemisch aus Ether und Wasser aufgenommen, die wäßrige Phase mehrfach mit Ether extrahiert, die vereinigten Etherphasen mit Kaliumcarbonat getrocknet, das Trockenmittel abfiltriert und das Filtrat am Rotationsverdampfer eingeengt. Die anschließende fraktionierende Destillation lieferte 937 mg (18% der Theorie) eines Gemisches aus 83% N-Ethyl-2-propinylamin und 17% Toluol, das direkt weiter umgesetzt wurde. C₅H₉N (MG = 83,15 g/mol)
¹H-NMR(CDCl₃, 250 MHz): δ = 1,12 (t, 3 H, CH₂CH₃); 1,30 (s br, NH); 2,20 (t, 1 H, C≡CH); 2,74 (q, 2 H, CH₂CH₃); 3,42 (d, 2 H, NCH₂C≡C)

### C2) 1-(8-Ethylamino-5-hydroxy-5-methyl-oct-6-inyl)-3-methyl-7-propylxanthin

Unter Argon und Rühren tropfte man bei -78°C zu einer Lösung aus 937 mg (9,35 mmol) N-Ethyl-2-propinylamin (83%ig in Toluol aus Stufe C1) in 30 ml Tetrahydrofuran langsam 5,43 ml (8,69 mmol) einer 15%igen Butyllithium-Lösung in n-Hexan. Es wurde eine Stunde bei dieser Temperatur gerührt, auf Raumtemperatur erwärmt und eine Lösung aus 2,05 g (6,68 mmol) 3-Methyl-1-(5-oxohexyl)-7-propylxanthin in 12 ml Tetrahydrofuran langsam zur inzwischen weißen Suspension gegeben. Nach einer Stunde wurde mit 2 N Salzsäure und 5%iger Natriumhydrogencarbonat-Lösung neutralisiert, mit Dichlormethan extrahiert, über Magnesiumsulfat getrocknet, das Trockenmittel abfiltriert und unter vermindertem Druck das Lösungsmittel entfernt. Das Rohprodukt wurde mit dem Laufmittelgemisch Dichlormethan/Methanol/gesättigte Ammoniak-Lösung = 19/1,5/0,02 flash-chromatographisch gereinigt.
Ausbeute: 2,35 g (90%); Öl
1,3 g des noch mit Dichlormethan verunreinigten Produkts wurden in einem Gemisch aus Aceton und Wasser gelöst und anschließend am Rotationsverdampfer und dann im Hochvakuum wieder von den Solvenzien befreit. Zurück blieben 1,3 g eines zähen, lösungsmittelfreien Öls.
C₂₀H₃₁N₅O₃ (MG = 389,56 g/mol)
Massenspektrum: 390,2 (100%, M + H); 372,2 (28%); 209,1 (47%);
¹H-NMR (DMSO-d₆, 200 MHz): δ = 0,85 (t, 3 H, N⁷(CH₂)₂CH₃); 0,98 (t, 3 H, NCH₂CH₃); 1,30-1,65 (m, 6 H, CH₂); 1,30 (s, 3 H, C(OH)CH₃); 1,79 (sex, 2 H, N⁷CH₂CH₂CH₃); 2,56 (q, 2 H, NCH₂CH₃); 3,30 (s, 2 H, NCH₂C≡C); 3,44 (s, 3 H, N³CH₃); 3,77-3,95 (m, 2 H, N¹CH₂); 4,21 (t, 2 H, N⁷CH₂); 5,07 (s, 1 H, OH); 8,10 (s, 1 H, N=CH)

### Beispiel 24: 1-(8-Ethylpropylamino-5-hydroxy-5-methyl-oct-6-inyl)-3-methyl-7-propylxanthin (nach Verfahrensvariante E)

650 mg (1,67 mmol) 1-(8-Ethylamino-5-hydroxy-5-methyl-oct-6-inyl)-3-methyl-7-propylxanthin aus Beispiel 23 wurden in 30 ml Ethanol gelöst. Nach Kühlen auf -78°C wurden 602 µl (8,34 mmol) Propionaldehyd zugegeben, anschließend auf 0°C erwärmt und mit 105 mg (1,67 mmol) Natriumcyanoborhydrid versetzt. Um die Umsetzung zu vervollständigen, wurden nach 2 Stunden eine Spatelspitze Natriumcyanoborhydrid und nach 3 Stunden erneut 602 µl (8,34 mmol) Propionaldehyd und 105 mg (1,67 mmol) Natriumcyanoborhydrid zugegeben. Nach vollständiger Umsetzung wurde das Reaktionsgemisch am Rotationsverdampfer unter vermindertem Druck eingeengt, der Rückstand mit gesättigter Natriumhydrogencarbonat-Lösung versetzt, die wäßrige Phase mit Dichlormethan extrahiert, die vereinigten Extrakte mit Magnesiumsulfat getrocknet, das Trockenmittel abfiltriert und das Filtrat unter vermindertem Druck am Rotationsverdampfer eingeengt. Das Rohprodukt wurde mit dem Laufmittelgemisch Dichlormethan/Methanol/gesättigte Ammoniak-Lösung = 19/1/0,02 flash-chromatographisch gereinigt.
Ausbeute: 503 mg (70%); Öl
C₂₃H₃₇N₅O₃ (MG = 431,65 g/mol); Massenspektrum: 432,4 (100%, M + H); 414,3 (44%); ¹H-NMR (DMSO-d₆, 200 MHz): δ = 0,75-0,88 (2 t, 6 H, N(CH₂)₂CH₃, N⁷(CH₂)₂CH₃); 0,94 (t, 3 H, NCH₂CH₃); 1,30-1,68 (m, 8 H, CH₂); 1,30 (s, 3 H, C(OH)CH₃); 1,79 (sex, 2 H, N⁷CH₂-CH₂CH₃); 2,24-2,45 (m, 4 H, NCH₂CH₂CH₃, NCH₂CH₃); 3,32 (s, 2 H, NCH₂C≡C); 3,43 (s, 3 H, N³CH₃); 3,76-3,95 (m, 2 H, N¹CH₂); 4,21 (t, 2 H, N⁷CH₂); 5,06 (s, 1 H, OH); 8,10 (s, 1 H, N=CH)

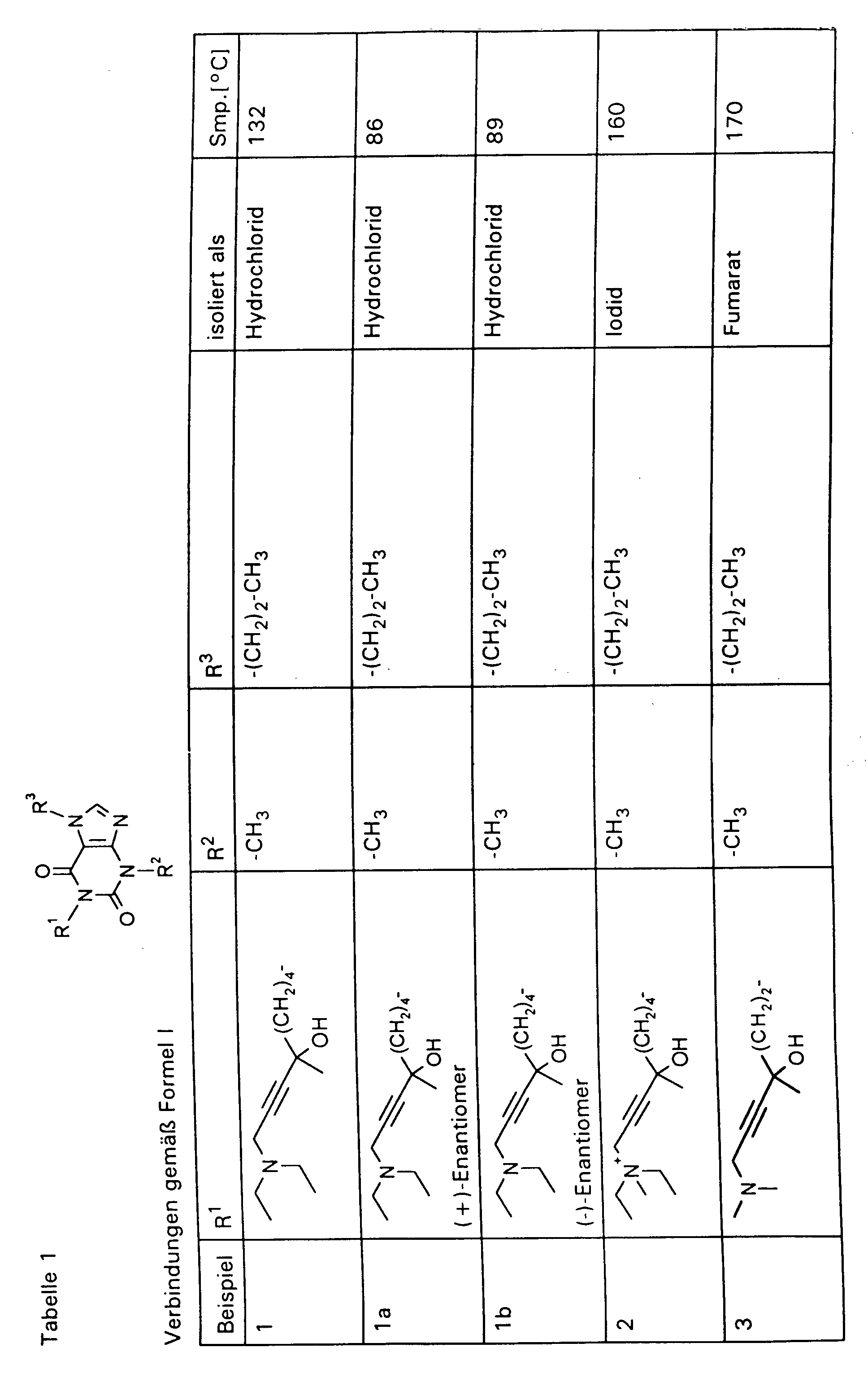

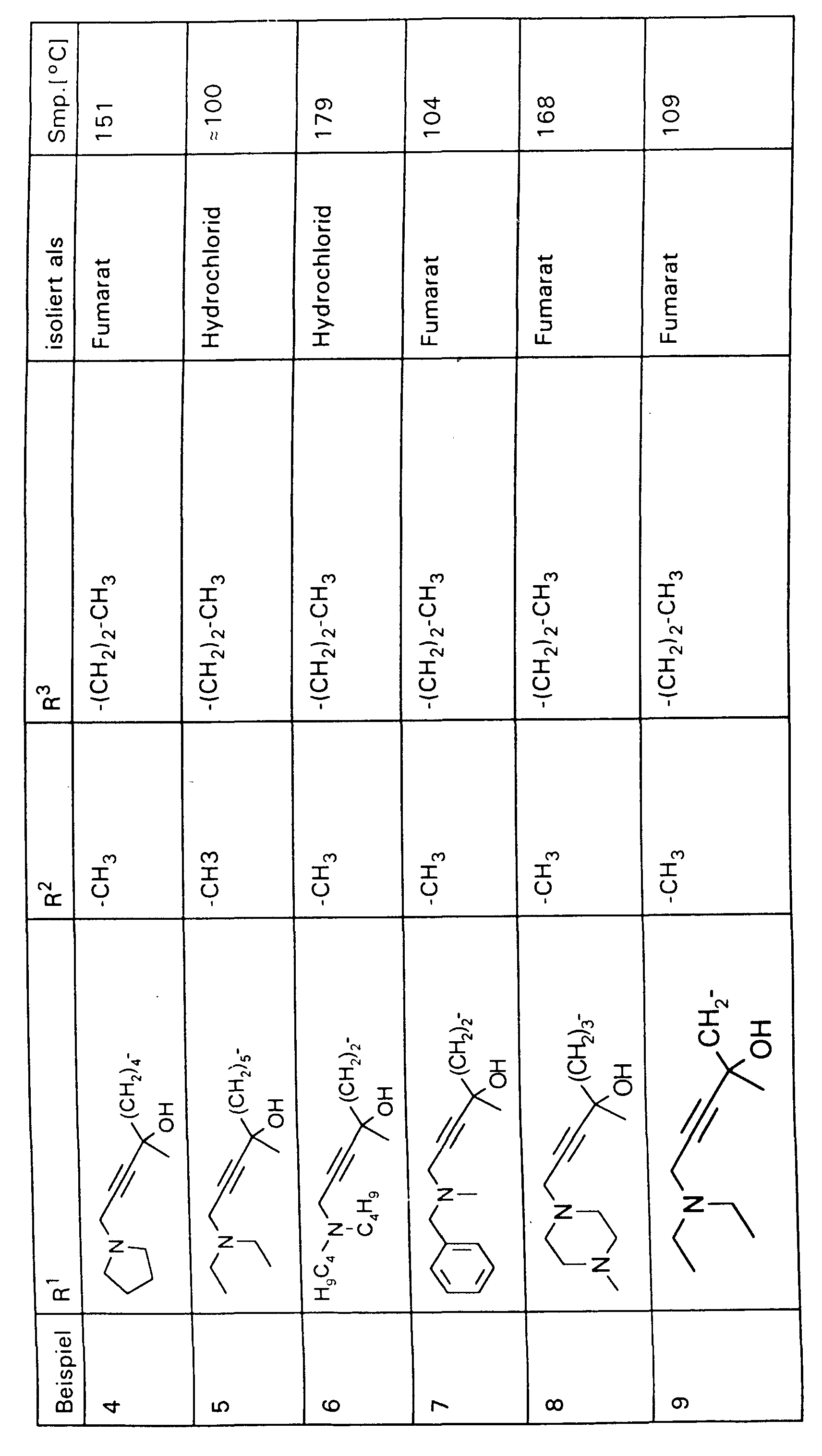

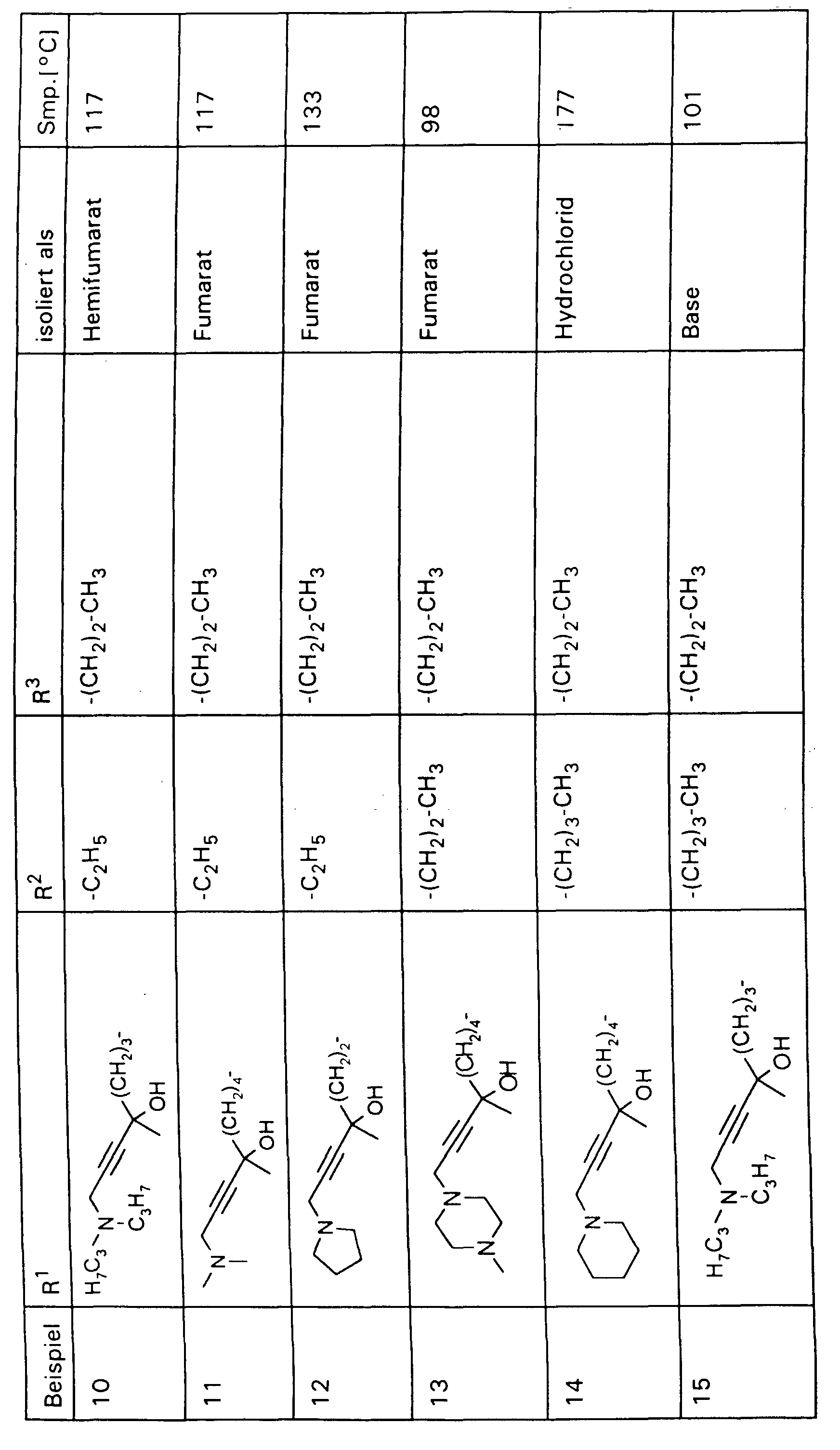

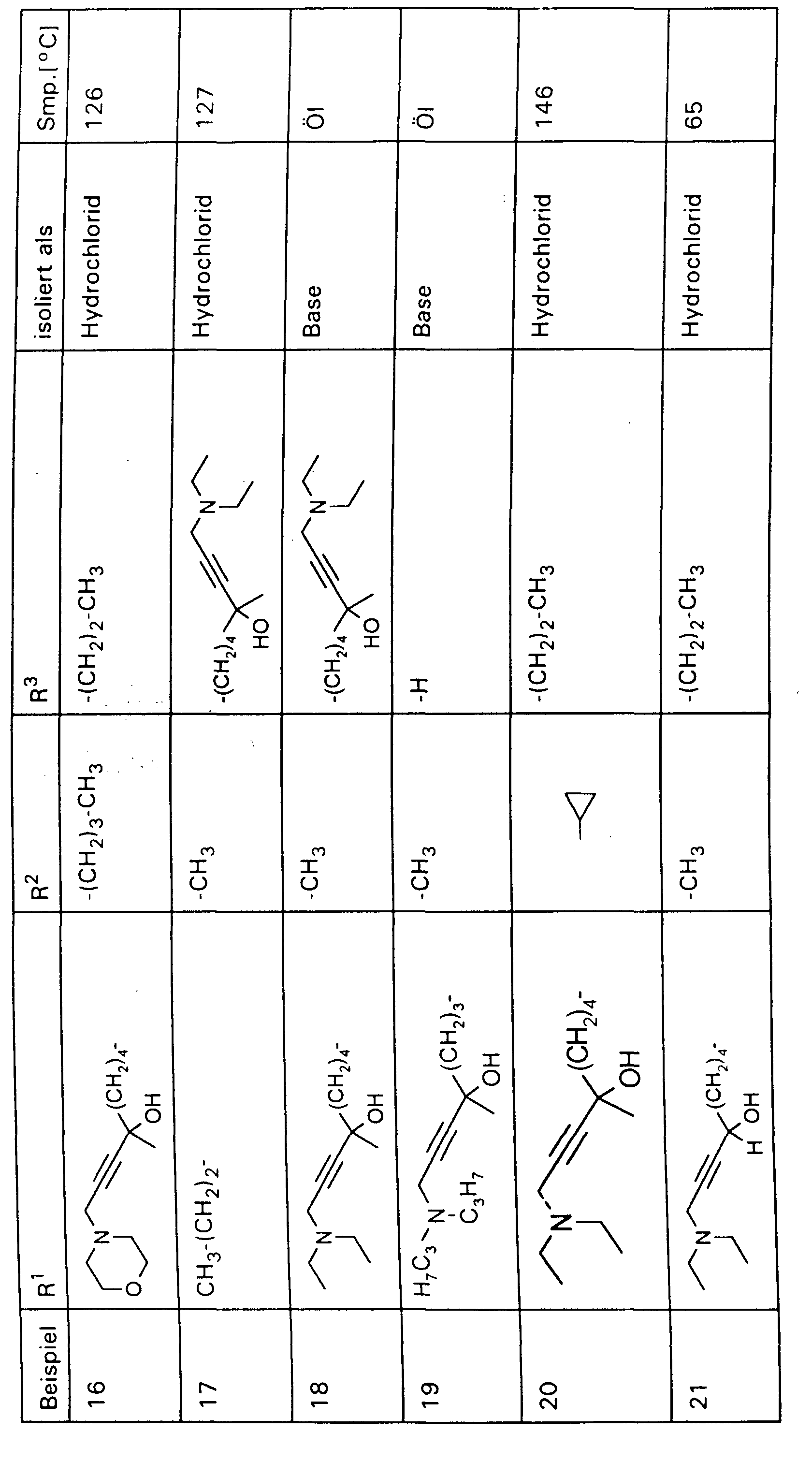

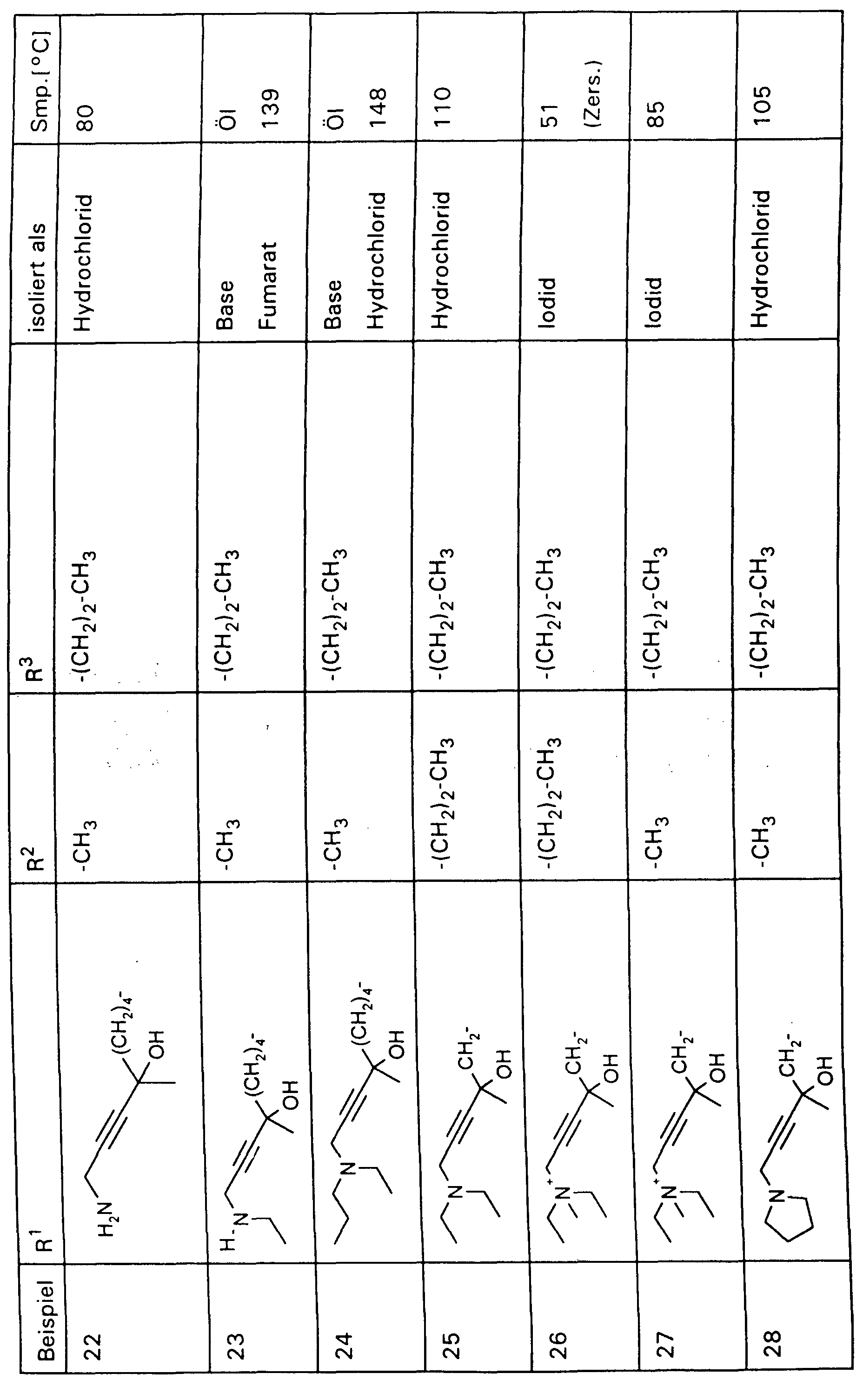

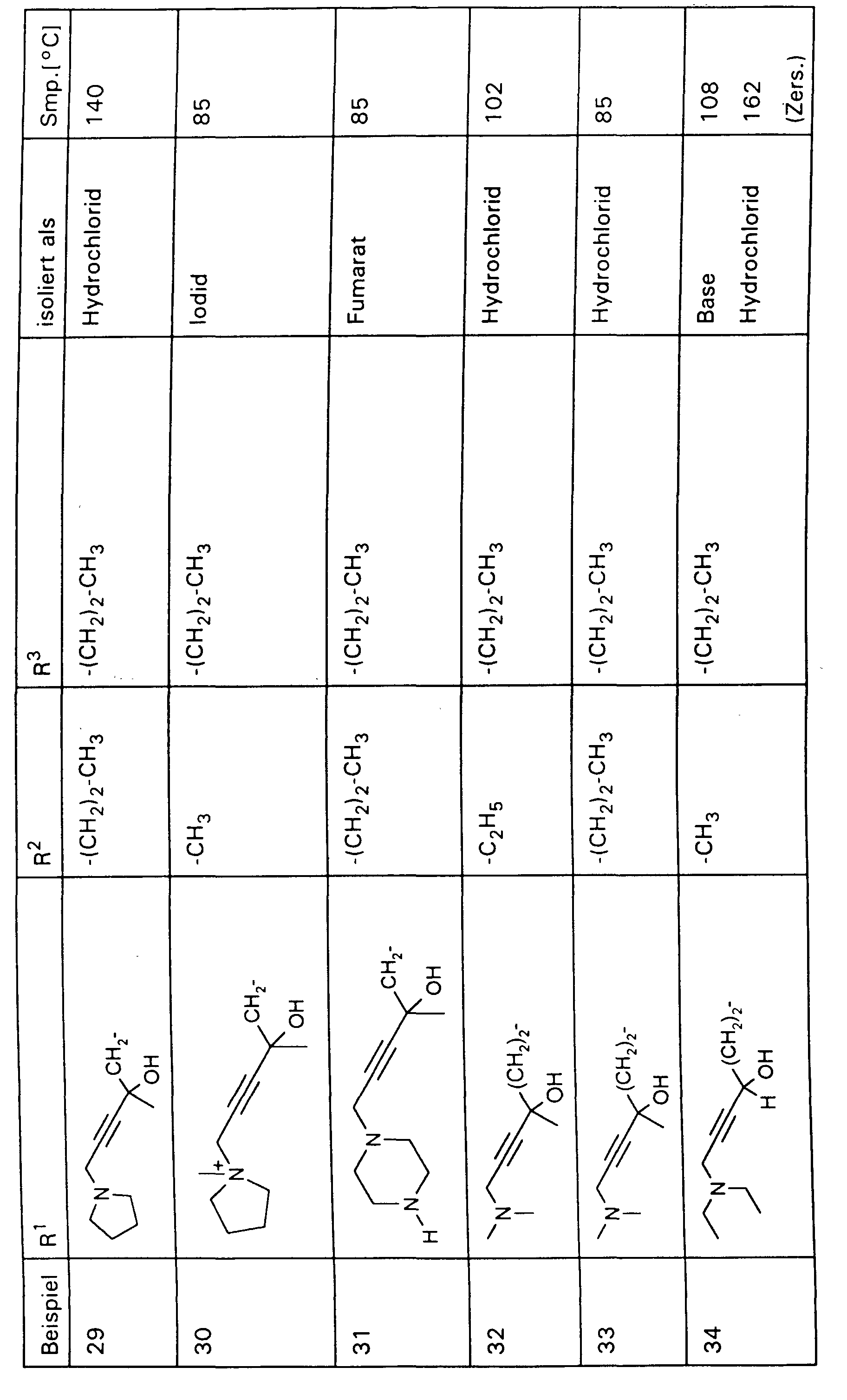

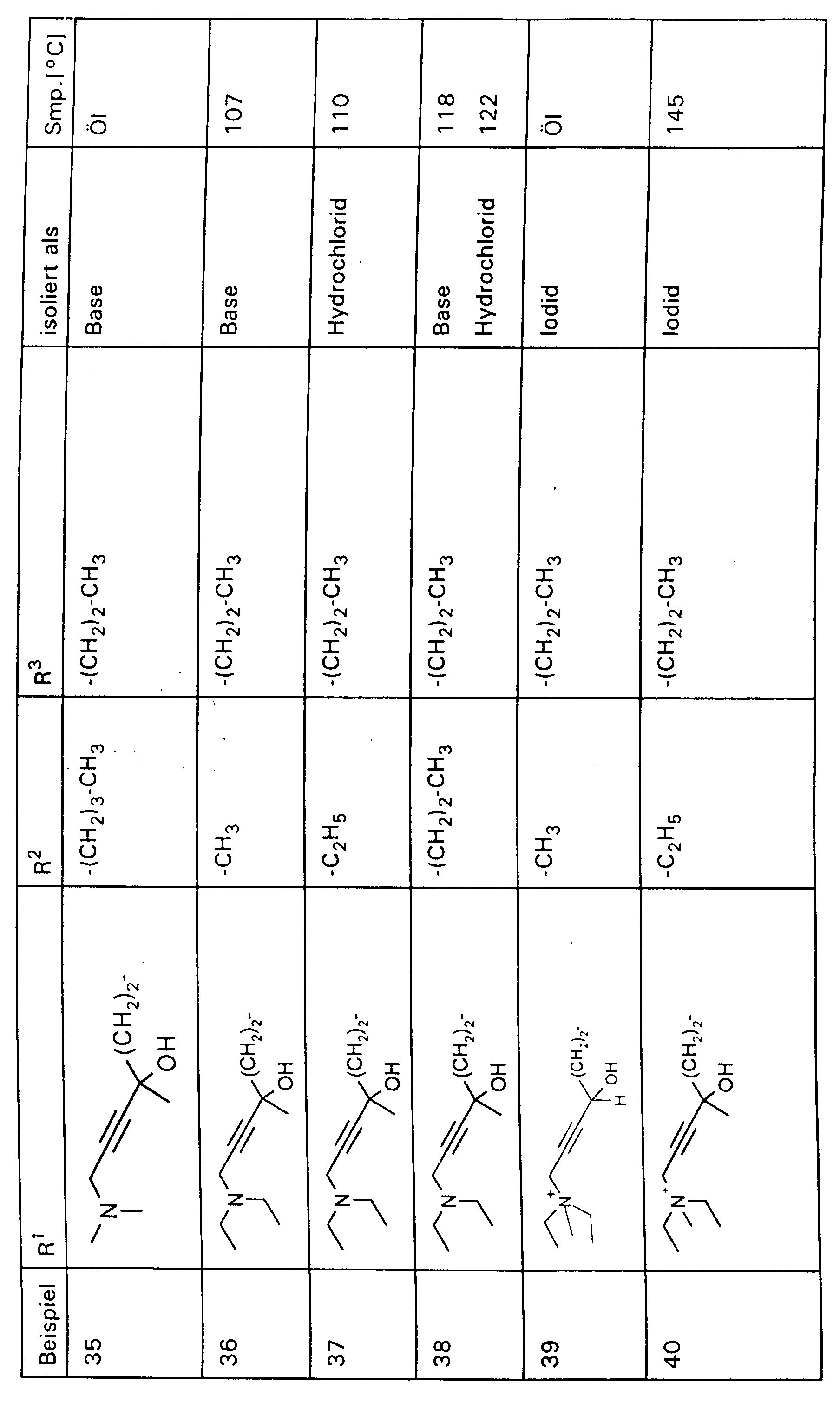

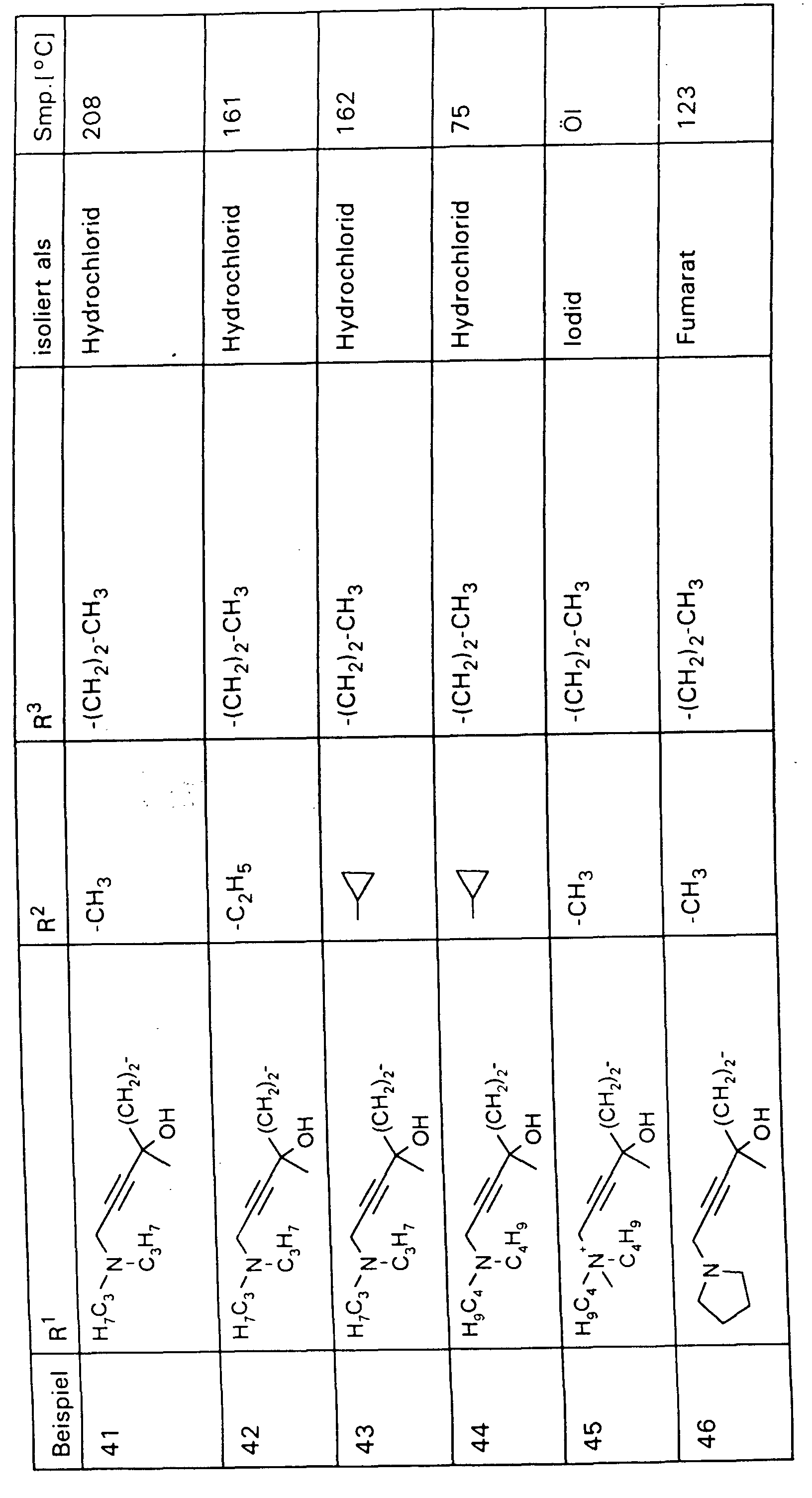

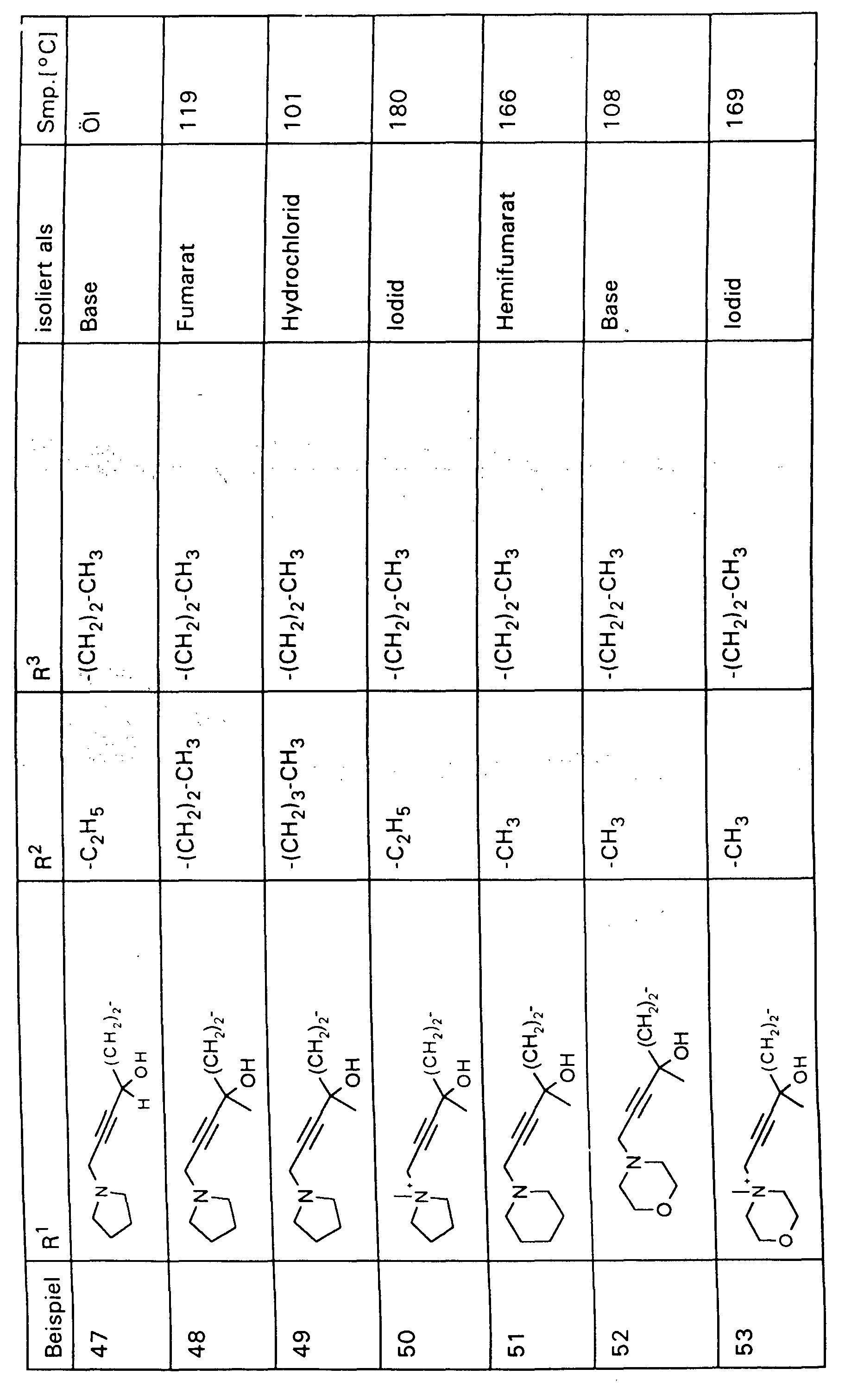

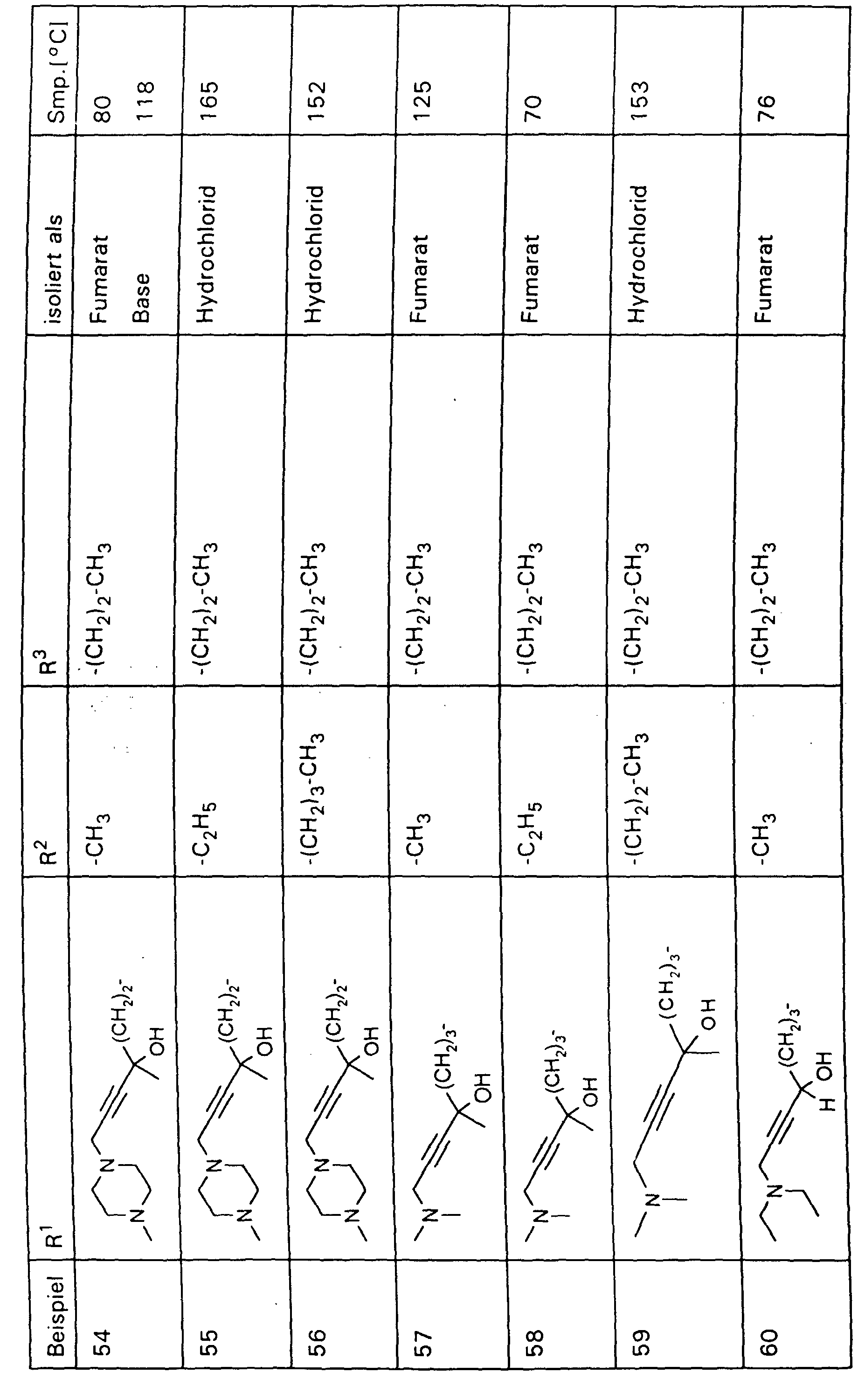

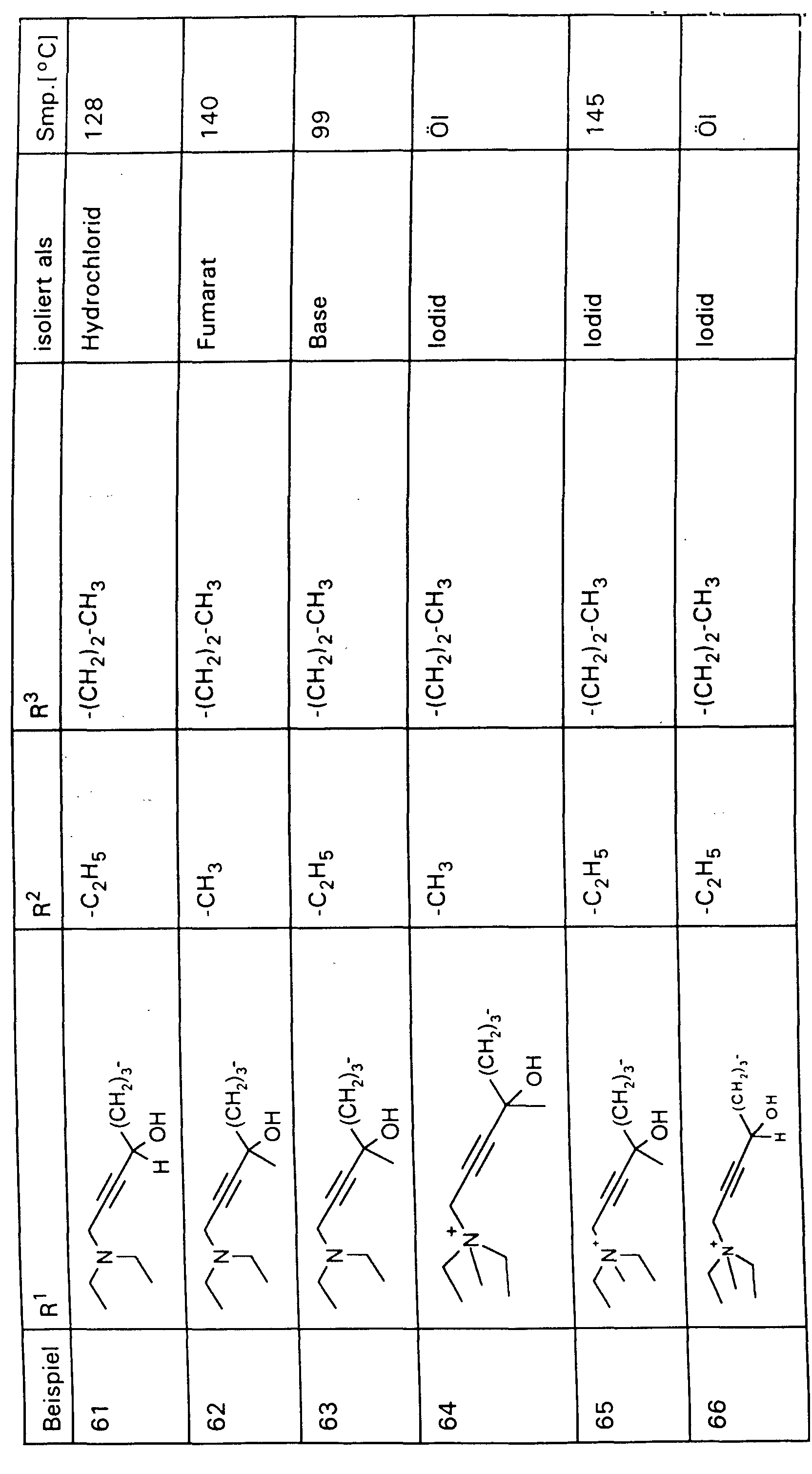

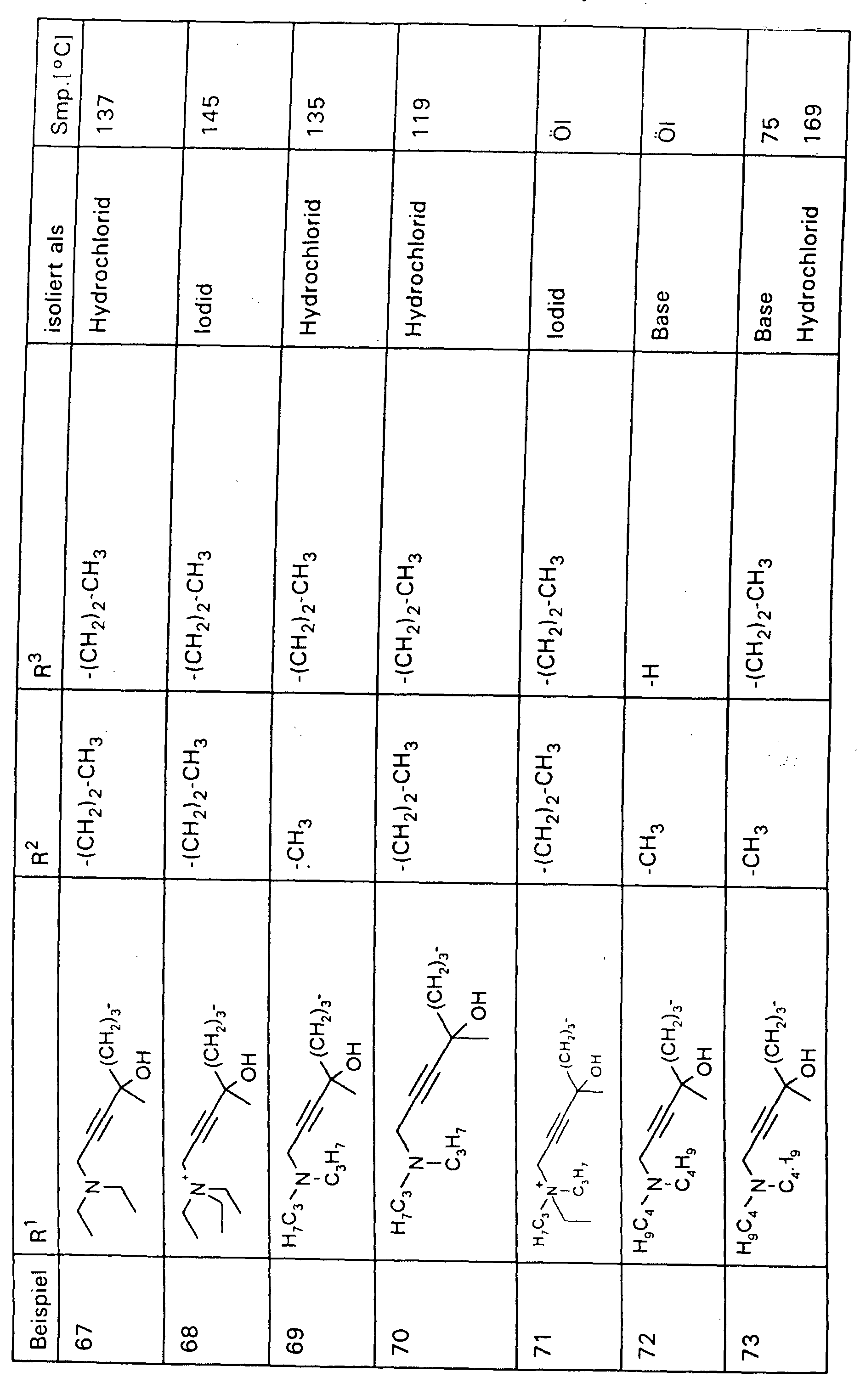

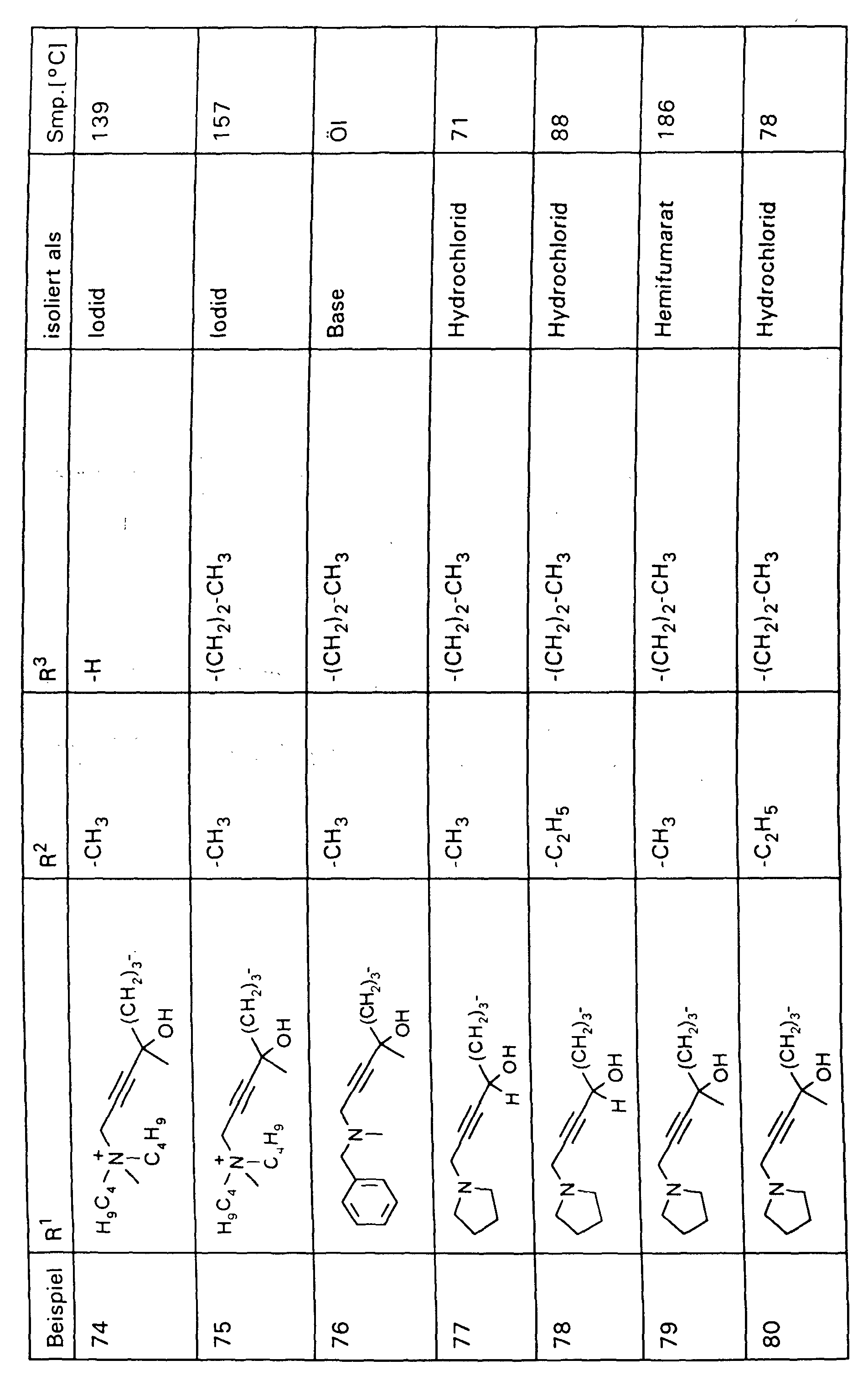

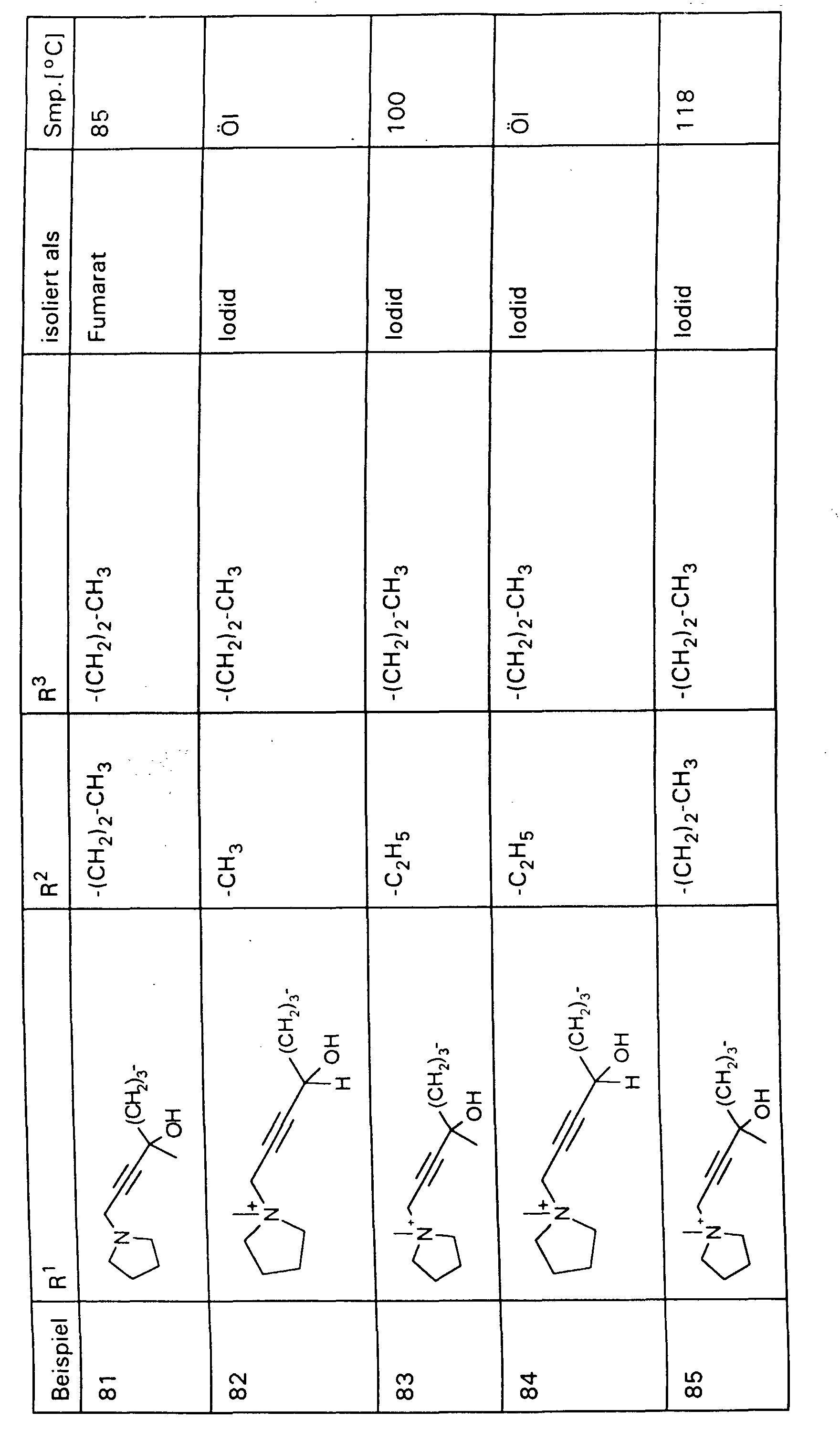

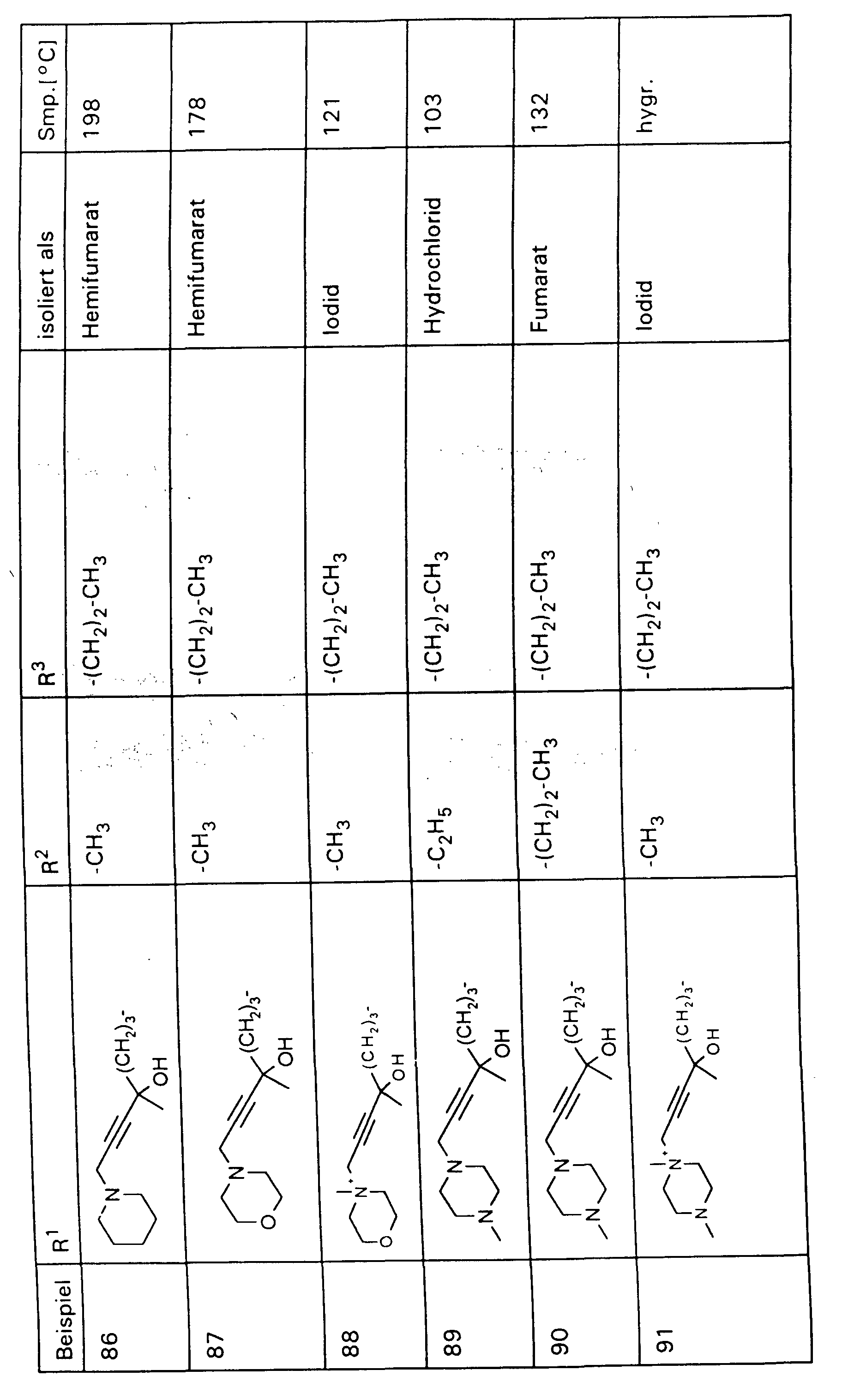

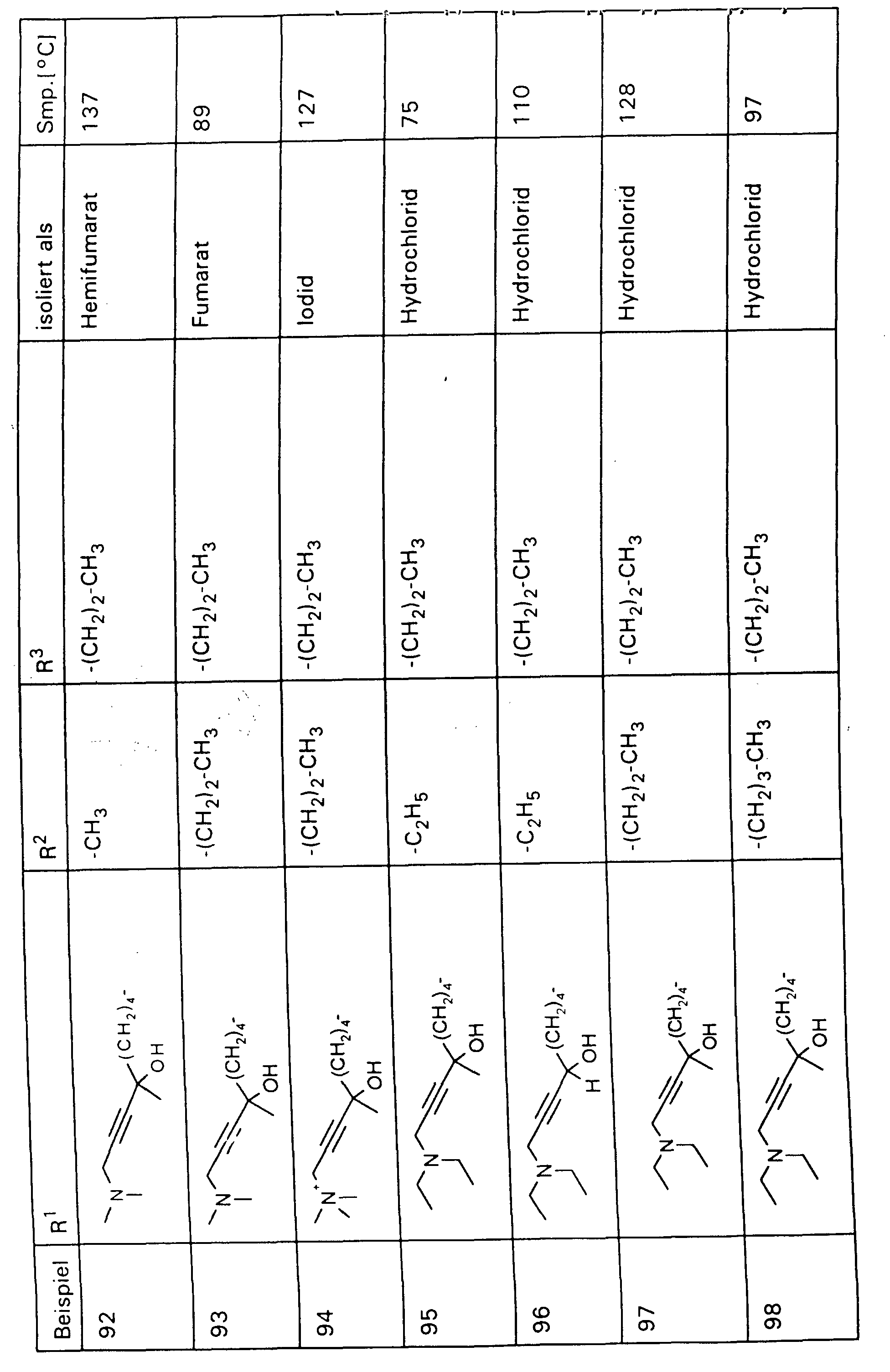

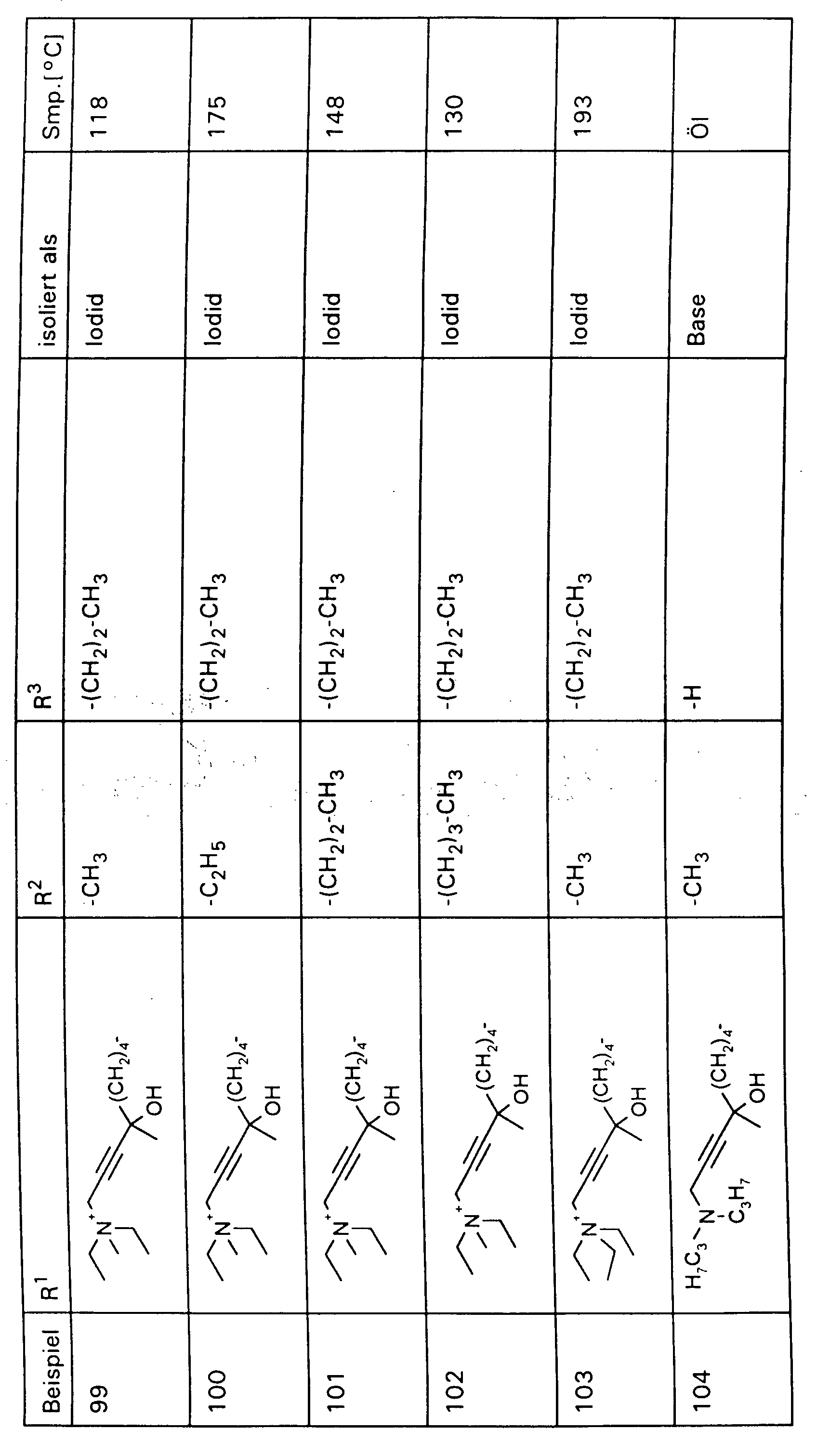

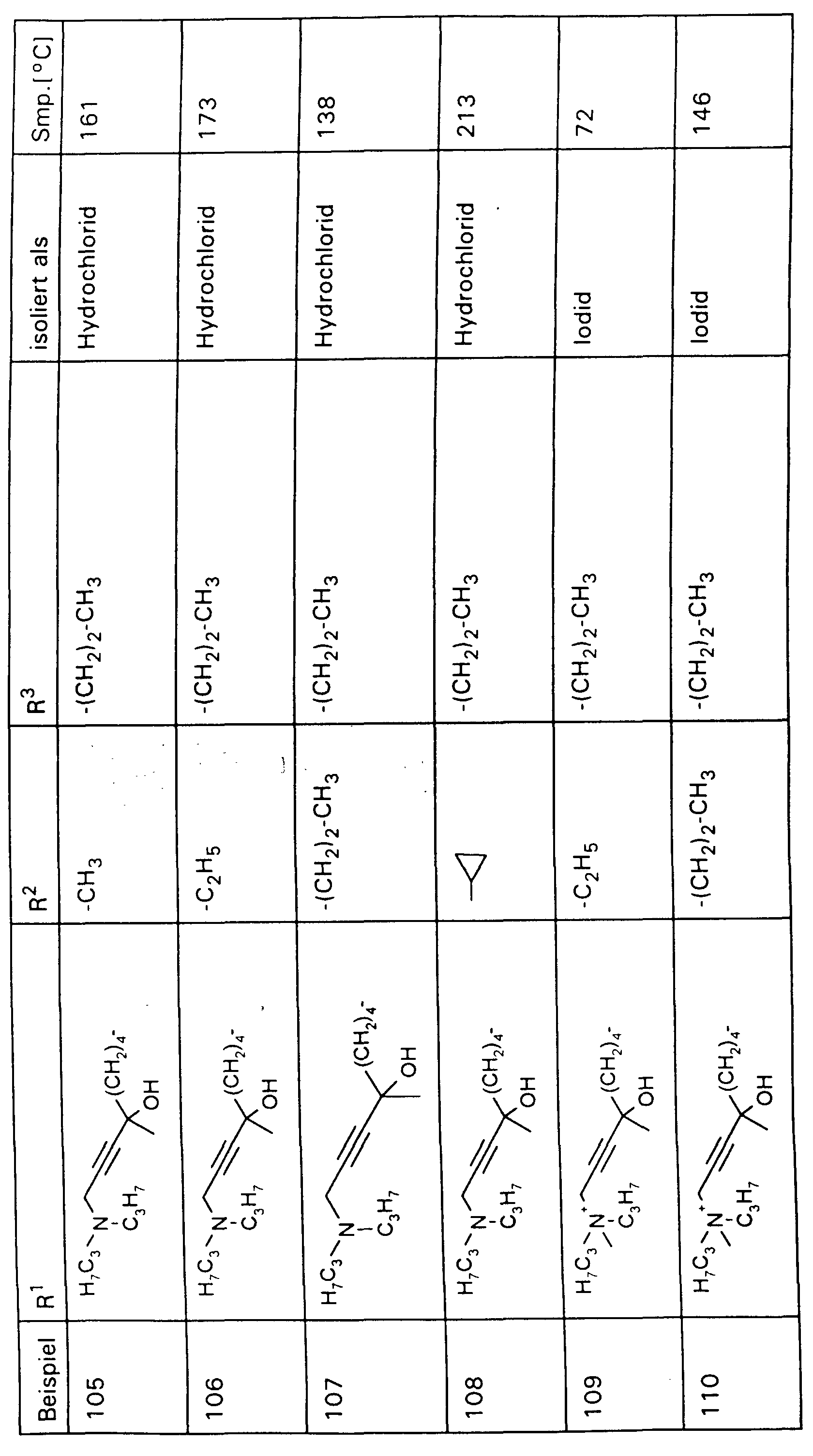

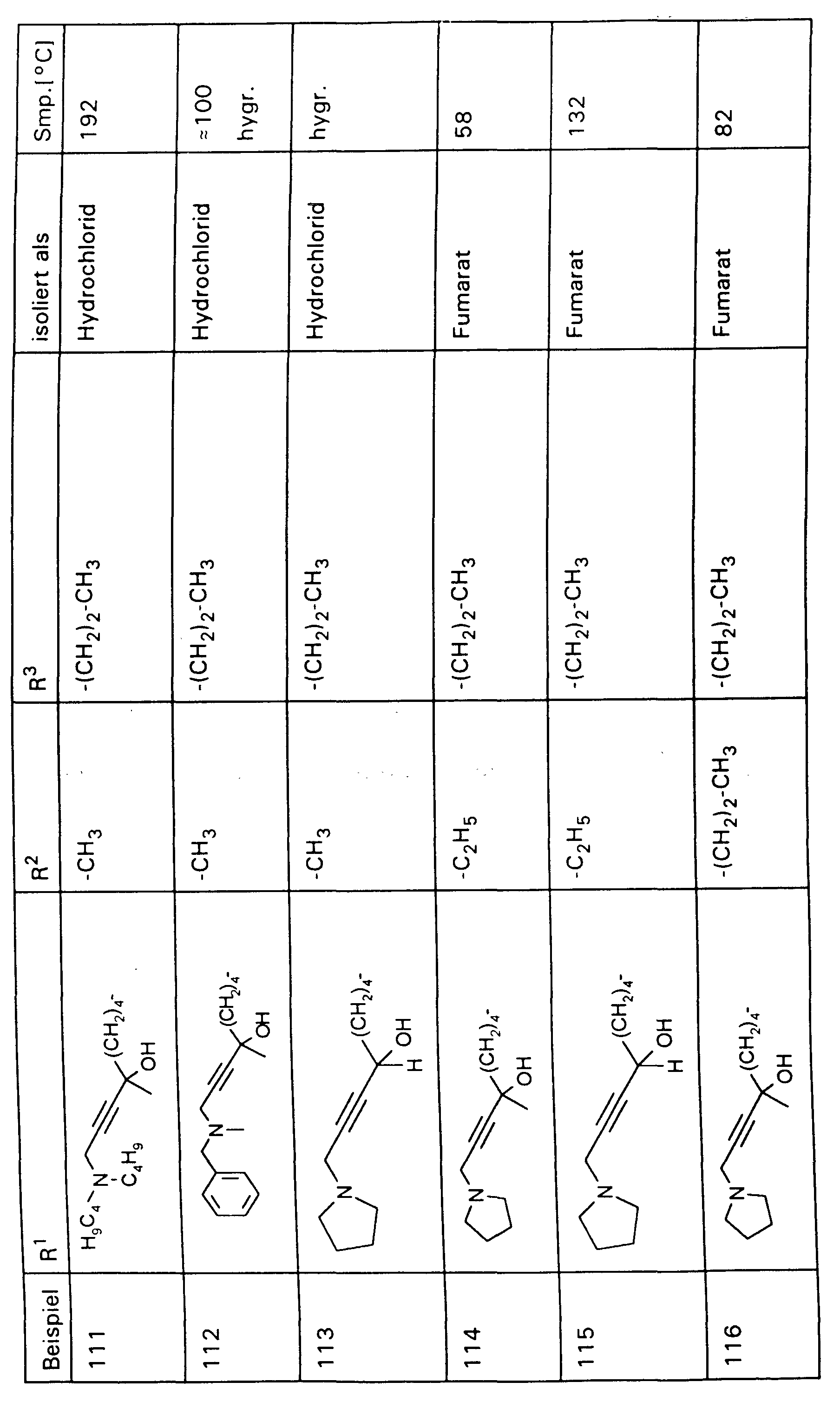

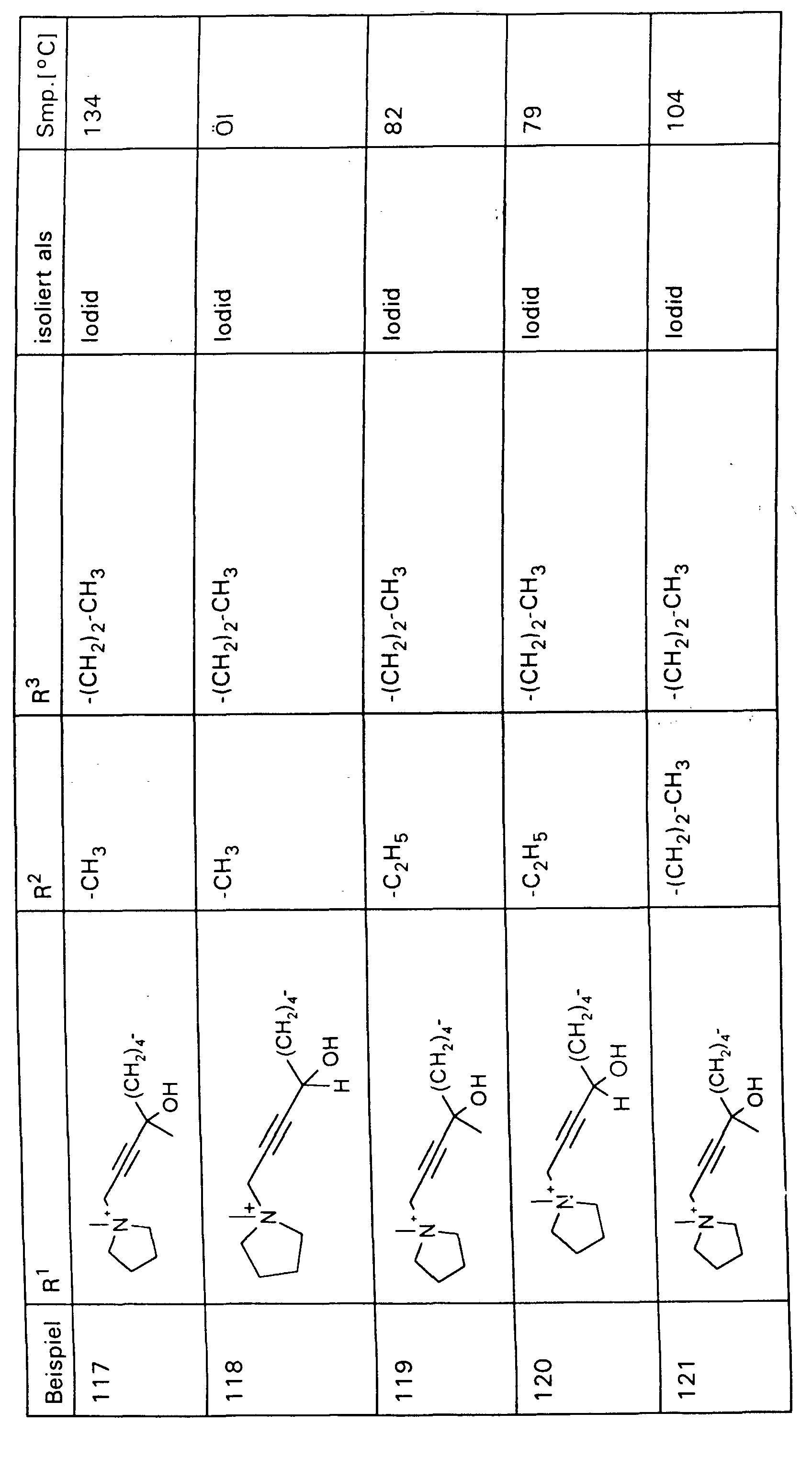

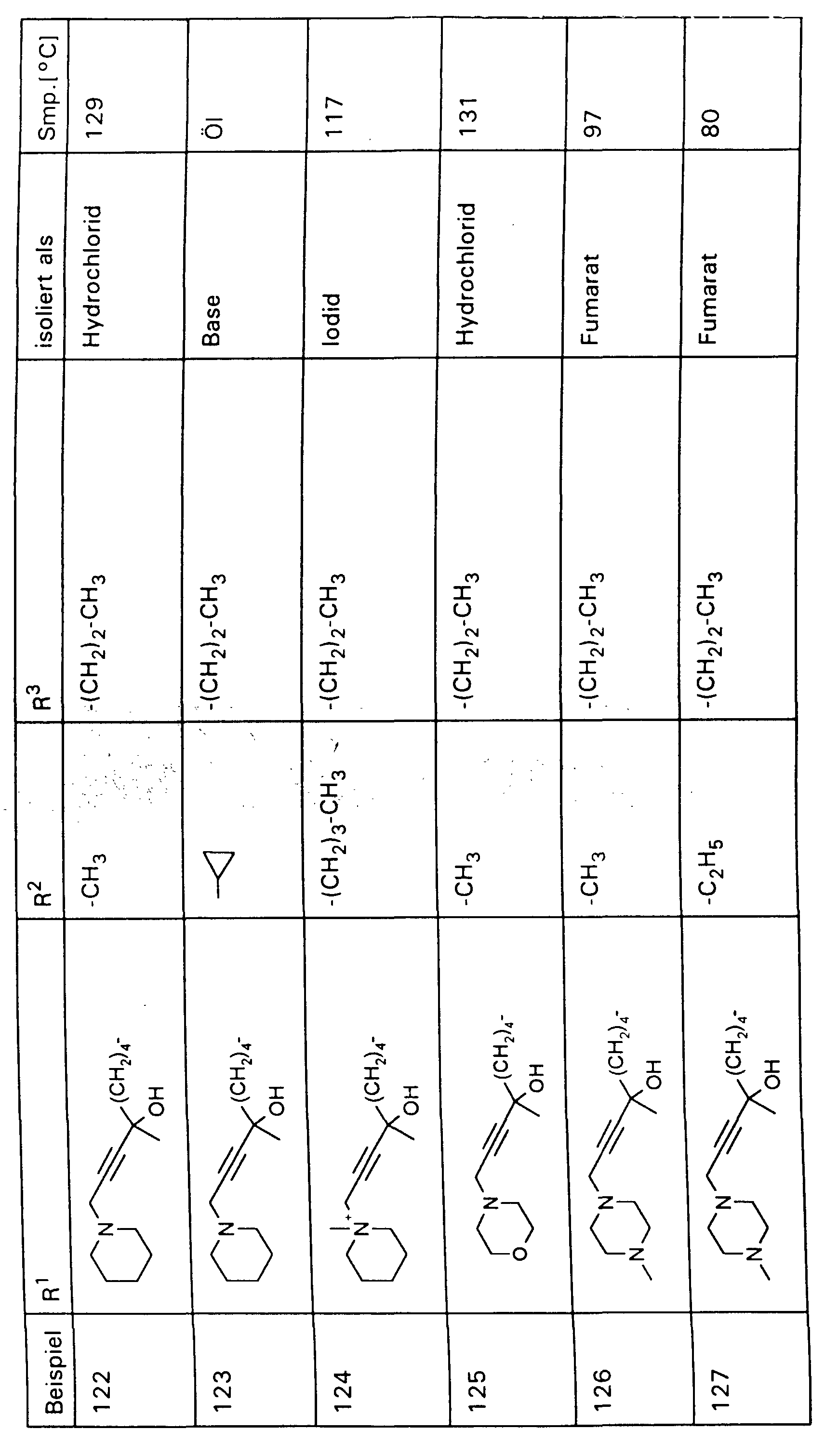

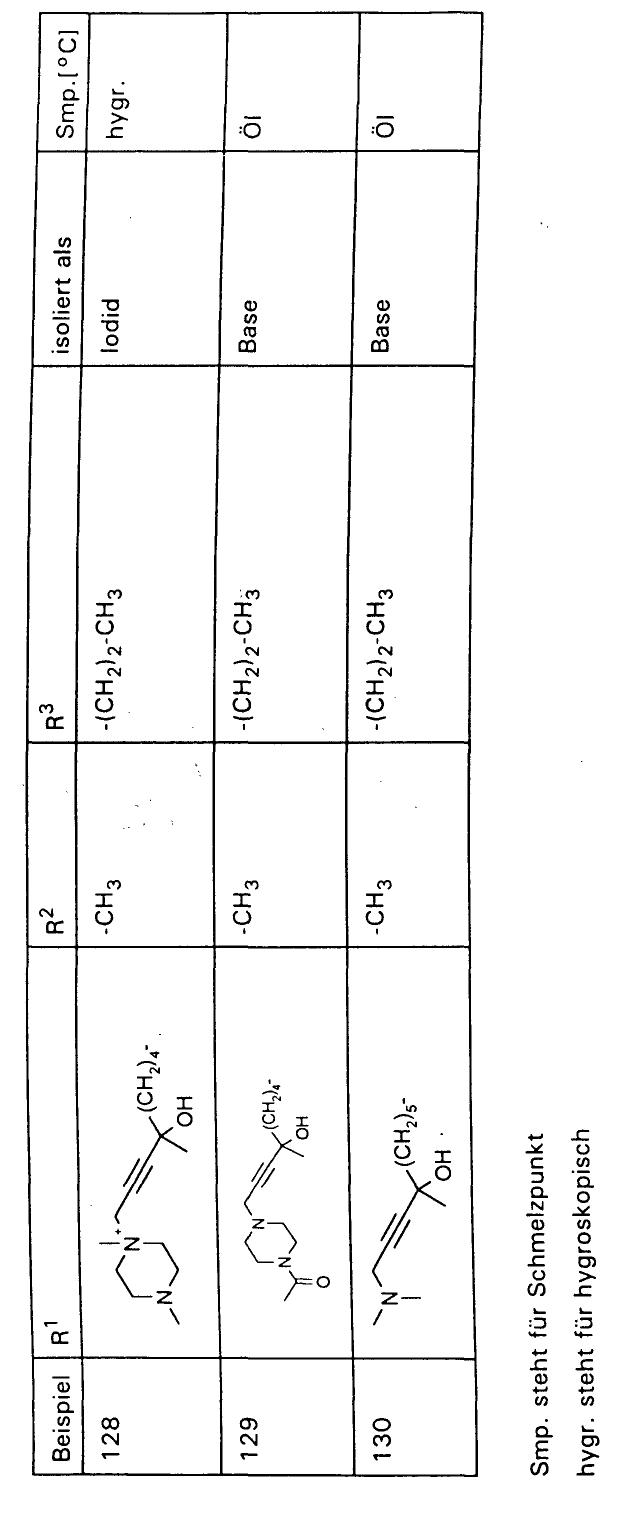

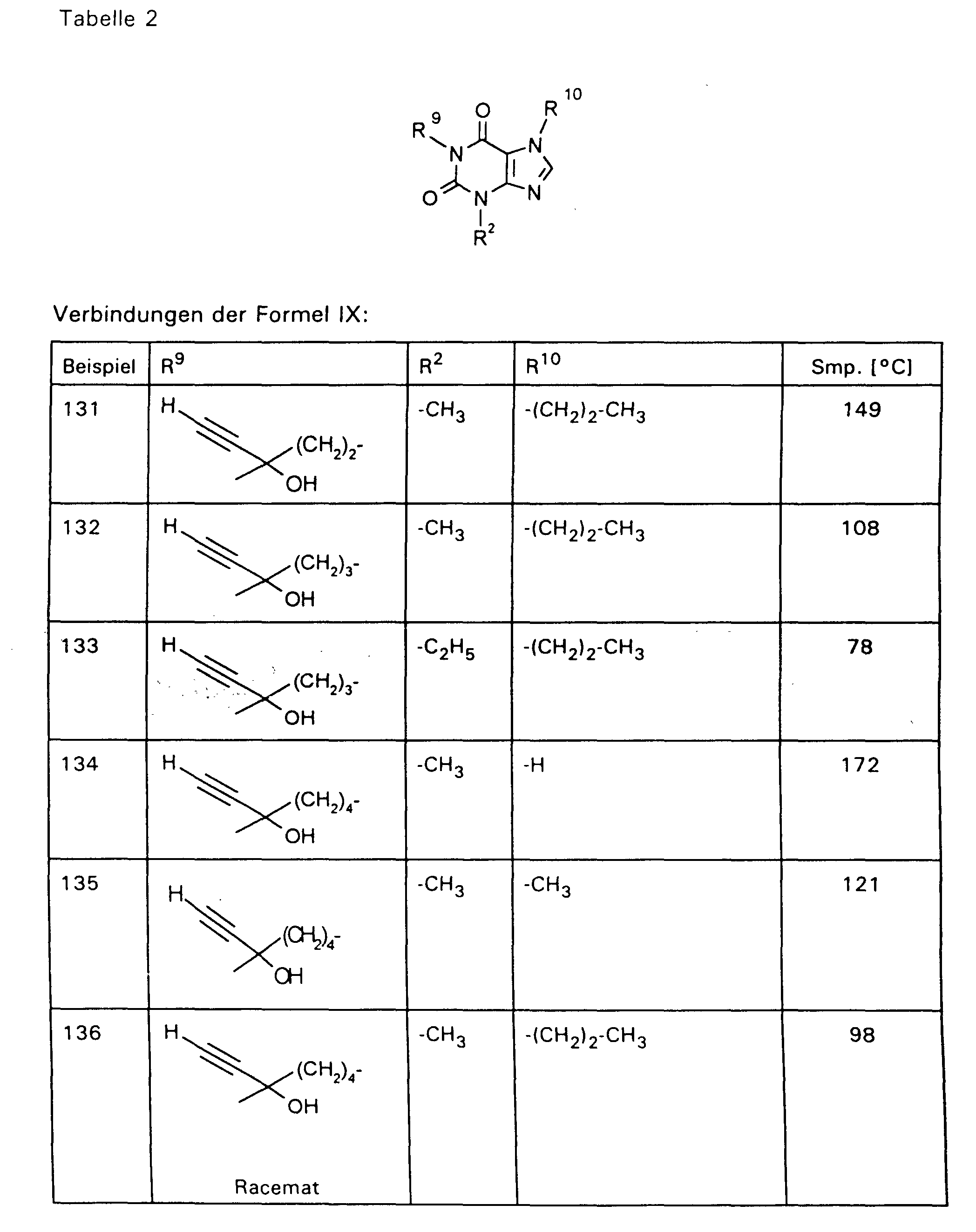

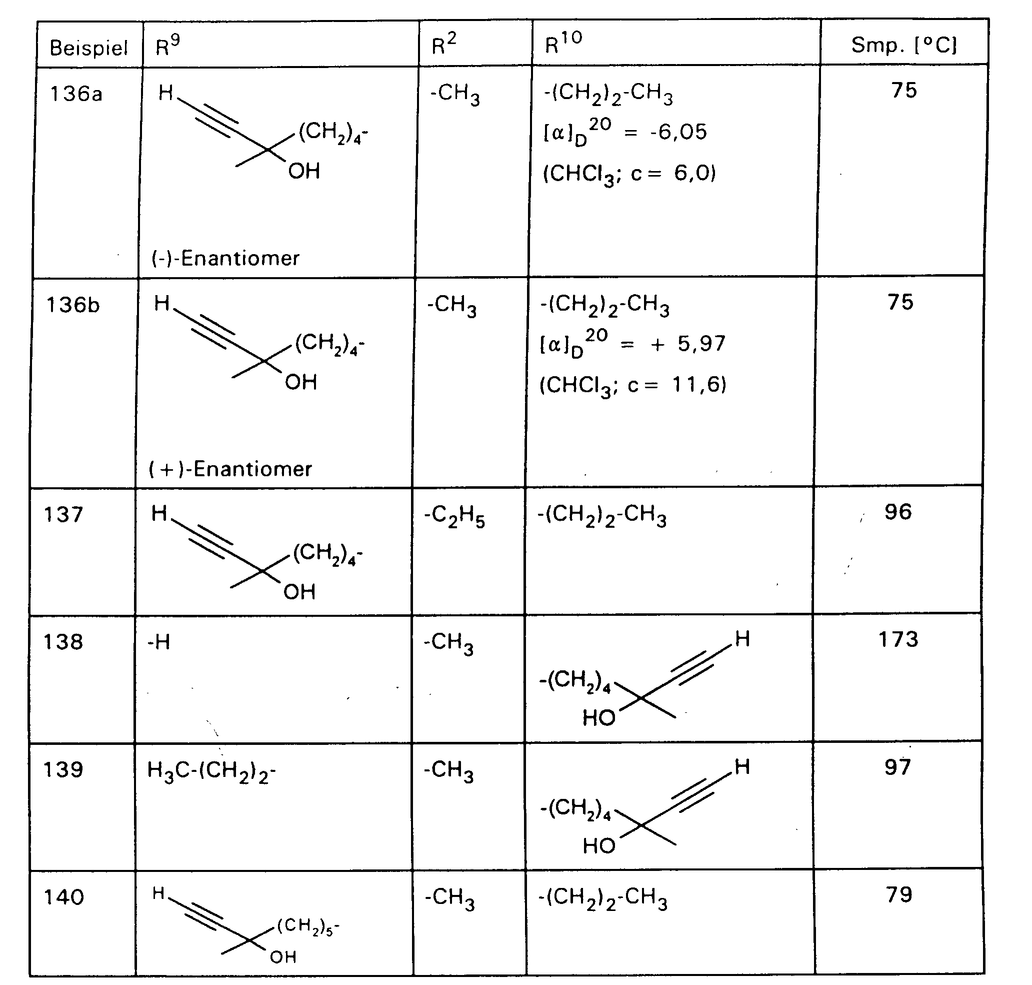

### Pharmakologische Prüfung und Ergebnisse

Die ausgeprägte neuronale Schutzwirkung der Verbindungen gemäß Formel I ließ sich in tierexperimentellen, für die Klinik relevanten Eignungsmodellen demonstrieren, wobei das Xanthinderivat Propentofyllin (3-Methyl-1-(5-oxohexyl)-7-propylxanthin) als Vergleichspräparat in die Untersuchungen miteinbezogen wurde.
Die Testergebnisse belegen, daß die erfindungsgemäßen Verbindungen dem Vergleichspräparat deutlich überlegen sind und demzufolge ein größeres therapeutisches Potential für die kurative und prophylaktische Behandlung zerebrovaskulärer Erkrankungen besitzen.

### 1. Neuroprotektive Wirkung im Modell der transienten globalen Ischämie am Gerbil

Zur Versuchsdurchführung, die nach den Richtlinien des Deutschen Tierschutzgesetzes erfolgte, wurden 30 männliche Mongolische Gerbils mit einem Körpergewicht zwischen 60 und 70 g randomisiert auf zwei Kollektive mit jeweils 15 Tieren verteilt. Die Tiere des ersten Kollektives erhielten 30 Minuten nach der Ischämieperiode die jeweilige Testsubstanz durch intraperitoneale Injektion, während die Tiere des zweiten Kollektives, das als unbehandelte Kontrollgruppe diente, lediglich das gleiche Volumen des betreffenden Vehikels erhielten.

Zur Erzeugung der temporären Vorderhirnischämie wurden die Tiere unter Halothan-Narkose auf einem beheizten Operationstisch in Rückenlage fixiert, beide Arteria carotides communes vorsichtig freigelegt und mittels Mikroaneurysma-Clips für drei Minuten verschlossen (J. Cereb. Blood Flow Metab. 1987, 7/1: 74 - 81). 7 Tage nach der 3-minütigen Ischämieperiode wurden die Tiere in Halothan-Narkose dekapitiert, die Gehirne rasch und schonend entnommen, zunächst in Carnoy'scher Lösung (Ethanol/Chloroform/Essigsäure 6 : 3 : 1) immersionsfixiert und dann in Paraffin eingebettet, anschließend 4 bis 6 µm dicke Coronarschnitte durch den Hippokampus etwa in Höhe des Bregma hergestellt und diese mit Hämatoxylin und Eosin gefärbt. Danach bestimmte man im Rahmen eines Blindversuches lichtmikroskopisch das Ausmaß der eosinophilen Nekrosen der Pyramidenzellen in der CA1-Region des Hippokampus anhand eines semiquantitativen histopathologischen Scores (0 = keine; 1 = leichte; 2 = mittelschwere; 3 = schwere und 4 = komplette Nekrosen). Als Bewertungsgröße für die neuroprotektive Wirkung diente die prozentuale Änderung der mittleren histopathologischen Scores der Präparatgruppe gegenüber dem der unbehandelten Kontrollgruppe. Die Versuchsergebnisse sind in Tabelle 3 zusammengestellt.

**Tabelle 3**

| Hemmung des ischämischen Nervenzellschadens am Mongolischen Gerbil | | |
|---|---|---|
| Verbindung aus Beispiel | Dosis in mg/kg | Hemmung des neuronalen CA1-Hippokampusschadens in % |
| 1 | 10 | 31 |
| 1 | 5 | 19 |
| 2 | 10 | 49 |
| 3 | 10 | 30 |
| 4 | 10 | 45 |
| 4 | 5 | 39 |
| 6 | 10 | 30 |
| 13 | 10 | 36 |
| 14 | 10 | 48 |
| 18 | 10 | 38 |
| 21 | 10 | 38 |
| 25 | 10 | 54 |
| 32 | 10 | 48 |
| 41 | 10 | 42 |
| 44 | 10 | 39 |
| 52 | 10 | 31 |
| 62 | 10 | 22 |
| 63 | 10 | 50 |
| 77 | 10 | 39 |
| 87 | 10 | 39 |
| 94 | 10 | 25 |
| 97 | 10 | 34 |
| 107 | 10 | 30 |
| 110 | 10 | 32 |
| 111 | 10 | 34 |
| 112 | 10 | 21 |
| 124 | 10 | 31 |
| 126 | 10 | 36 |
| 129 | 10 | 45 |
| Propentofyllin (Vergleich) | 10 | 19 |

Die überraschend gute neuroprotektive Aktivität der erfindungsgemäßen Verbindungen der Formel I konnte auch in den nachfolgend beschriebenen, verfahrenstechnisch noch aufwendigeren Versuchsanordnungen überzeugend nachgewiesen werden.

### 2. Hemmwirkung auf die neurologische Symptomatik im Modell der permanenten fokalen zerebralen Ischämie an der Ratte

Als Versuchstiere dienten erwachsene, 300 bis 400 g schwere, männliche Sprague-Dawley-Ratten mit experimentell durch permanenten Verschluß der mittleren zerebralen Arterie (MCA) erzeugtem fokalem Hirninfarkt (J. Cereb. Blood Flow Metab. 1981, 1: 53 - 60).

Die etwa 20 bis 30 Minuten währende chirurgische Präparation erfolgte unter Anästhesie mit 1 bis 1,2 % Halothan-haltigem Lachgas, das der Atemluft bei spontaner Atmung über eine Gasmaske zugemischt wurde. Nach Katheterisierung der rechten Femoralarterie und -vene für Blutdruckmessung, Blutentnahmen und spätere Testsubstanz-Verabreichung wurde der Verschluß der linken MCA unter starker Vergrößerung eines Operationsmikroskopes via subtemporalen Zugang ohne Entfernung von Jochbogen und Schläfenmuskulatur durch Elektrokoagulation und anschließende Gefäßdurchtrennung herbeigeführt, wobei man den Operationsverlauf durch kontinuierliche Registrierung des mittleren arteriellen Blutdruckes mit Hilfe eines elektromechanischen Druckaufnehmers (Modell 7E Polygraph; Grass, USA) überwachte. Nach dem operativen Eingriff ließ man die Tiere aus der Narkose erwachen und hielt ihre Körpertemperatur mit einer homöothermen Heizdecke (Homeothermic Blanket System; Harvard Apparatus, UK) im Normbereich um 37°C.

15 Minuten nach dem Gefäßverschluß verabreichte man den Tieren der Präparatgruppe (n = 8) die Prüfsubstanz in Form einer intraperitonealen Bolusinjektion von 10 mg/kg als Startdosis und setzte die Behandlung durch 24-stündige Dauerinfusion von 0,1 mg/kg/min über den venösen Katheter mit Hilfe eines speziellen, frei drehbaren Spannschloßsystems (Harvard Apparatus, UK) fort, während die Tiere der unbehandelten Kontrollgruppe (n = 7) auf entsprechendem Wege lediglich das Vehikel (physiologische Kochsalzlösung) erhielten. 15 Minuten vor und unmittelbar nach der Gefäßokklusion sowie kurz nach Beginn der Dauerinfusion des Prüfpräparates bzw. des Vehikels wurden zur Erfassung von physiologischen Unregelmäßigkeiten die arteriellen Blutgase und der pH-Wert (178 pH/Blutgas-Analysator; Corning, USA) sowie der Hämatokrit und Blutglukosespiegel kontrolliert; danach konnte der arterielle Katheter entfernt werden. Darüber hinaus wurden vom Operationsbeginn bis 10 Minuten nach dem Start der Dauerinfusion und einige Minuten vor Versuchsende die Temperatur der Schläfenmuskulatur bilateral (Therm 2250-1; Ahlborn Meß- und Regeltechnik, BRD) und die Körpertemperatur rektal erfaßt. 24 Stunden nach dem Gefäßverschluß beendete man die Dauerinfusion und bestimmte das Ausmaß des ischämisch bedingten neurologischen Defizits anhand der vierstufigen Symptomenskala nach Bederson et al. (Stroke 1986, 17: 422 - 476) mit folgenden Bewertungskriterien:
- 0 =: keine neurologischen Ausfallerscheinungen;
- 1 =: Beugehaltung der Vorderextremitäten;
- 2 =: verminderte Widerstandskraft gegen seitliches Anstoßen ohne Kreisbewegungen;
- 3 =: gleiche Symptomatik wie bei Grad 2, jedoch mit Kreisbewegungen.

Zur biostatistischen Analyse der Versuchsdaten wurde die Häufigkeitsverteilung der neurologischen Scores von Präparat- und Kontrollgruppe mit dem Student's t-Test verglichen (Signifikanzniveau p < 0,05). Hierbei bewirkte beispielsweise die Verbindung gemäß Beispiel 1 eine signifikante Verringerung (p < 0,01) des neurologischen Defizits (1,1 ± 0,4; Mittelwert ± SD) gegenüber dem der unbehandelten Kontrolltiere (2,3 ± 0,5; Mittelwert ± SD), entsprechend einer Verbesserung des neurologischen Status um 52 %, ohne daß sich irgendein negativer Einfluß auf die untersuchten physiologischen Parameter erkennen ließ.

### 3. Neuroprotektive Wirkung im Modell der permanenten fokalen zerebralen Ischämie an der Ratte

Die Versuchsanlage entsprach weitestgehend der im Experiment 2 beschriebenen Methode. Präparat- und Kontrollgruppe umfaßten jeweils n = 6 Tiere. Die Prüfsubstanzen wurden jedoch unter Verzicht auf die am wachen Tier recht komplizierte intravenöse Dauerinfusion ausschließlich intraperitoneal verabreicht, und zwar durch dreimalige Gabe von jeweils 10 mg/kg im zeitlichen Abstand von 15 Minuten, 3 und 6 Stunden nach dem chirurgischen MCA-Verschluß. Nach 24-stündiger Versuchsdauer wurden die Tiere unter Narkose dekapitiert, die Gehirne rasch und schonend entnommen, für 10 Minuten bei -10°C eingefroren und anschließend die Vorderhirne in definierten Schnittebenen in 8 coronare Schnitte zerlegt, die man mittels Cresyl-Färbetechnik anfärbte. Nach Art eines Blindversuches wurden dann die infarzierten, nicht färbbaren Bezirke der Coronarschnitte nach grafischer Übertragung in ein Diagramm planimetrisch vermessen und durch Integration über alle Flächenwerte (Neurosci. Lett. 1992, 147: 41 - 44) das Infarktvolumen in der von der Ischämie betroffenen linken zerebralen Hemisphäre ermittelt. Die Signifikanz (Niveau < 0,05) der Unterschiede zwischen dem unbehandelten Kontrollkollektiv und den Präparatgruppen wurde mit dem Student's t-Test beurteilt.

Bei der Testung der erfindungsgemäßen Verbindungen im direkten Vergleich mit Propentofyllin führte beispielsweise das Präparat aus dem Herstellungsbeispiel 1 nach intraperitonealer Verabreichung von 3 x 10 mg/kg (entspricht 3 x 22 µmol/kg) zu einer statistisch signifikanten (p < 0,05) 56%igen Reduktion des Infarktvolumens (99 ± 17 µl; Mittelwert ± SD) gegenüber dem der unbehandelten Kontrollgruppe (222 ± 43 µl; Mittelwert ± SD), während das Vergleichspräparat Propentofyllin ebenfalls in der Dosis von 3 x 10 mg/kg (entspricht 3 x 33 µmol/kg) eine 43%ige Senkung (127 ± 28 µl; Mittelwert ± SD) bewirkte.

### 4. Neuroprotektive Wirkung im Modell der permanenten fokalen zerebralen Ischämie an der Maus

In dieser Versuchsanordnung wurde der Effekt der Verbindungen der Formel I im Vergleich mit Propentofyllin als Referenzsubstanz auf den nekrotischen Schaden an der Oberfläche der Großhirnrinde (Cortex) nach permanentem Verschluß der rechten MCA untersucht, der ein verläßliches Maß für das Infarktvolumen darstellt (J. Pharmacol. Methods 1992, 27: 27 - 32).

Als Versuchstiere dienten männliche Swiss CD1-Mäuse mit einem Körpergewicht zwischen 33 und 40 g, deren rechte MCA durch chirurgischen Eingriff analog Experiment 2 unter Chloralhydrat-Narkose (400 mg/kg i.p.) verschlossen wurde. Vier Mäuse unterzog man einer Scheinoperation, bei der zwar die MCA in gleicher Weise freigelegt, aber nicht okkludiert wurde; diese Tiere bildeten die Kontrollgruppe, mit der ein womöglicher Einfluß des operativen Eingriffes auf den Nervenzellschaden quantifiziert werden sollte. Da sowohl Anästhesie als auch Ischämie gewöhnlich eine Hypothermie induzieren, die zu einer Verringerung der Infarktgröße führen kann (Brain Res. 1992, 587: 66 - 72), wurden auch in diesem Versuchsmodell die Schläfenmuskeltemperatur während der operativen Manipulation mit einer Halogenwärmelampe und die Körpertemperatur über die gesamte Versuchsdauer durch adäquate Adjustierung der Umgebungstemperatur im Normbereich um 37°C gehalten. 5 Minuten sowie 3 und 6 Stunden nach der MCA-Okklusion verabreichte man den Tieren der Präparatgruppe (n = 12) die jeweilige Testsubstanz - gelöst in destilliertem Wasser - durch intraperitoneale (i.p.) Injektion von jeweils 10 mg/kg, während die Tiere der Placebogruppe (n = 12) nur das Vehikel und die Mäuse der Kontrollgruppe (n = 4) weder Präparat noch Vehikel erhielten. 24 Stunden nach dem Gefäßverschluß dekapitierte man die Tiere in Isofluran-Narkose, entnahm die Gehirne und färbte sie innerhalb von 30 bis 40 Minuten in 37°C warmer, 2%iger wäßriger 2,3,5-Triphenyltetrazoliumchlorid(TTC)-Lösung an. Danach wurde die Großhirnrinde der rechten Hemisphäre isoliert und die mit TTC nicht färbbare Infarktfläche durch Bildanalyse (BIOCOM) vermessen. Die statistische Auswertung der Versuchsergebnisse erfolgte mit den nichtparametrischen Tests nach Kruskal-Wallis und Mann-Whitney. Hierbei zeigte sich, daß bei den scheinoperierten Mäusen der Kontrollgruppe so gut wie keine Nekrosen im Cortex auftraten, während die Vehikel-behandelten Tiere der Placebogruppe infolge der fokalen Ischämie signifikante neuronale Schäden mit einer Infarktfläche von 31,3 ± 1,7 mm² (Mittelwert ± SD; p = 0,0002) aufwiesen.

Dieser Schaden ließ sich beispielsweise durch die Verbindung des Beispiels 1 signifikant um 38% auf 19,3 ± 1,5 mm² (Mittelwert ± SD; p = 0,0001) reduzieren, während mit Propentofyllin als Vergleichspräparat eine Schadensbegrenzung um nur 20 % auf 25,2 ± 2,1 mm² (Mittelwert ± SD; p = 0,0153) erzielt wurde. Da auch der Unterschied zwischen den beiden Präparatgruppen mit p < 0,05 statistische Signifikanz besaß, erwies sich die erfindungsgemäße Verbindung gegenüber dem Vergleichspräparat als signifikant stärker neuroprotektiv wirksam.

## Patentansprüche

1. Verbindung der Formel I 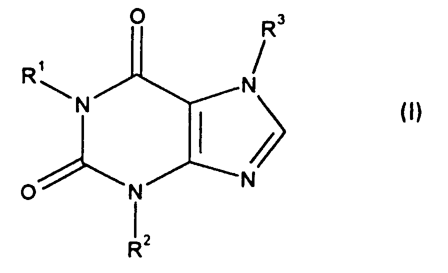 in stereoisomerenreiner Form oder als Stereoisomerengemische, wobei
1)
R¹ und R³ für einen Alkinolrest der Formel la oder Ib stehen, 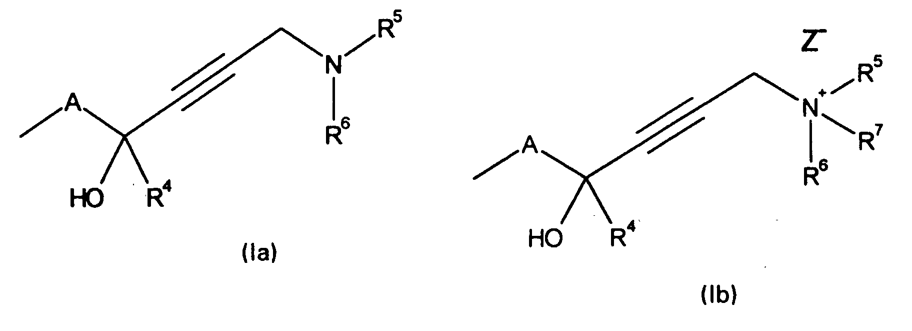
R² für
a) geradkettiges oder verzweigtes (C₁-C₅)-Alkyl,
b) (C₃-C₆)-Cycloalkyl oder
c) (C₄-C₈)-Cycloalkyl-alkyl steht,
R⁴ für ein Wasserstoffatom oder (C₁-C₃)-Alkyl steht,
R⁵, R⁶ und R⁷ unabhängig voneinander für
a) ein Wasserstoffatom,
b) (C₁-C₆)-Alkyl,
c) (C₃-C₆)-Cycloalkyl,
d) (C₄-C₈)-Cycloalkyl-alkyl,
e) Ar-(C₁-C₂)-alkyl oder
f) Tri-(C₁-C₄)-alkylsilyl stehen, oder
R⁵ und R⁶ zusammen mit dem Stickstoffatom, an das sie gebunden sind, einen 4- bis 7-gliedrigen gesättigten Ring bilden, der unsubstituiert oder ein- bis vierfach mit (C₁-C₄)-Alkyl substituiert ist, oder
R⁵ und R⁶ zusammen mit dem Stickstoffatom, an das sie gebunden sind, einen 4- bis 7-gliedrigen gesättigten Ring bilden, worin eine -CH₂-Gruppe des Ringes durch einen Rest aus der Gruppe O, S, SO, SO₂ und NR¹³ ersetzt ist,
R¹³ für ein Wasserstoffatom, (C₁-C₃)-Alkylcarbonyl oder (C₁-C₄)-Alkyl steht,
und der Ring unsubstituiert oder ein- bis vierfach mit (C₁-C₄)-Alkyl substituiert ist,
A für unverzweigtes oder verzweigtes (C₁-C₆)-Alkylen steht, und
Z⁻ für das Anion einer physiologisch verträglichen anorganischen oder organischen Säure steht, oder
2) R¹ oder R³ für einen Alkinolrest der Formel la oder Ib steht und der andere Rest R³ oder R¹ für
a) ein Wasserstoffatom oder
b) R⁸ steht,
worin R⁸ geradkettiges oder verzweigtes (C₁-C₆)-Alkyl, (C₃-C₆)-Cycloalkyl oder (C₄-C₈)-Cycloalkyl-alkyl bedeutet, und
R², R⁴, R⁵, R⁶, R⁷, A und Z⁻ wie unter 1) definiert sind.

2. Verbindung der Formel I gemäß Anspruch 1, dadurch gekennzeichnet, daß nur einer der beiden Reste R¹ oder R³ einen Alkinolrest der Formel la oder Ib darstellt und der andere Rest ein Wasserstoffatom oder R⁸ bedeutet.

3. Verbindung der Formel I gemäß Anspruch 1 oder 2, dadurch gekennzeichnet, daß R¹ für einen Alkinolrest der Formel la oder Ib und R³ für ein Wasserstoffatom oder R⁸ stehen.

4. Verbindung der Formel I gemäß einem oder mehreren der Ansprüche 1 bis 3, wobei
R¹ für einen Alkinolrest der Formel la oder Ib steht,
R² für geradkettiges (C₁-C₄)-Alkyl, Cyclopropyl oder Cyclopropylmethyl steht,
R³ für a) ein Wasserstoffatom oder b) R⁸ steht,
worin R⁸ geradkettiges oder verzweigtes (C₁-C₆)-Alkyl, Cyclopropyl oder Cyclopropylmethyl bedeutet,
R⁴ für ein Wasserstoffatom, Methyl oder Ethyl steht,
R⁵, R⁶ und R⁷ unabhängig voneinander für ein Wasserstoffatom, (C₁-C₄)-Alkyl, Cyclopropyl, Cyclopropylmethyl oder Benzyl stehen, oder
R⁵ und R⁶ zusammen mit dem Stickstoffatom, an das sie gebunden sind, einen 5- bis 6-gliedrigen gesättigten Ring aus der Gruppe Morpholin, 4-(C₁-C₃)-Alkylcarbonylpiperazin, 4-(C₁-C₂)-Alkylpiperazin, Piperazin, Piperidin, Pyrrolidin und Thiomorpholin bilden,
A für unverzweigtes (C₁-C₅)-Alkylen steht und
Z- für das Anion einer physiologisch verträglichen anorganischen oder organischen Säure steht.

5. Verbindung der Formel I gemäß Anspruch 4, wobei
R¹ für einen Alkinolrest der Formel la oder Ib steht,
R² für (C₁-C₄)-Alkyl steht,
R³ für geradkettiges (C₂-C₄)-Alkyl oder Cyclopropyl steht,
R⁴ für ein Wasserstoffatom oder Methyl steht,
R⁵, R⁶ und R⁷ unabhängig voneinander für ein Wasserstoffatom, (C₁-C₄)-Alkyl oder Benzyl stehen, oder
R⁵ und R⁶ zusammen mit dem Stickstoffatom, an das sie gebunden sind, den Morpholin-, Pyrrolidin-, Piperidin-, 4-Methylpiperazin- oder 4-Acetylpiperazinring bilden,
A für unverzweigtes (C₂-C₄)-Alkylen steht und
Z- für das Anion einer physiologisch verträglichen anorganischen oder organischen Säure steht.

6. Verbindung der Formel I gemäß Anspruch 5, dadurch gekennzeichnet, daß sie 1-(8-Diethylamino-5-hydroxy-5-methyl-oct-6-inyl)-3-methyl-, 1-(5-Hydroxy-5-methyl-8-pyrrolidino-oct-6-inyl)-3-methyl-, 3-Butyl-1-(5-hydroxy-5-methyl-8-piperidino-oct-6-inyl)-, 1-(5-Diethylamino-2-hydroxy-2-methyl-pent-3-inyl)-3-propyl-,1-(6-Dimethylamino-3-hydroxy-3-methyl-hex-4-inyl)-3-ethyl-, 1-(7-Diethylamino-4-hydroxy-4-methyl-hept-5-inyl)-3-ethyl-, 1-[8-(4-Acetyl-piperazino)-5-hydroxy-5-methyl-oct-6-inyl]-3-methyl-7-propylxanthin sowie deren physiologisch verträgliche Säureadditionssalze oder N,N-Diethyl-N-[4-hydroxy-4-methyl-8-(3-methyl-7-propyl-xanthin-1-yl)-oct-2-inyl]-N-methylammoniumiodid darstellt.

7. Verfahren zur Herstellung der Verbindung der Formel I gemäß einem oder mehreren der Ansprüche 1 bis 6, dadurch gekennzeichnet, daß nach Verfahrensvariante A ein 3-Alkylxanthin der Formel II, 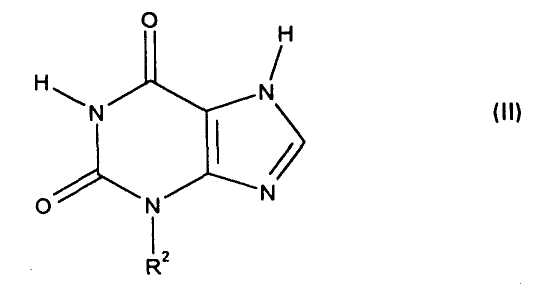 worin R² wie in Formel I definiert ist, ohne Kondensationsmittel oder in Gegenwart eines basischen Kondensationsmittels oder aber ein Salz der Verbindung der Formel II mit einer Verbindung der Formel III, 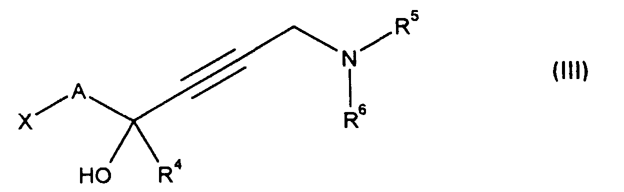 worin X für Chlor, Brom, lod oder einen Sulfonsäureester- oder Phosphorsäureester-Rest steht und A, R⁴, R⁵ und R⁶ wie in Formel I definiert sind, zu einer Verbindung der Formel Ic 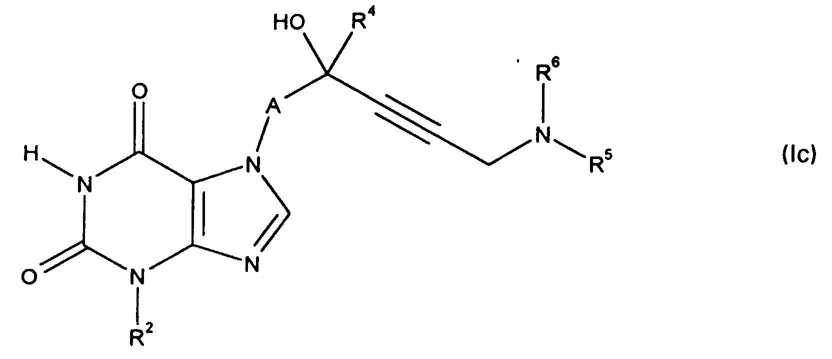 mit einem Alkinolrest der Formel Ia für R³ und einem Wasserstoffatom für R¹ gemäß Formel I umgesetzt wird und anschließend die Verbindung der Formel Ic ohne Kondensationsmittel oder in Gegenwart eines basischen Kondensationsmittels oder aber ein Salz der Verbindung der Formel Ic entweder wiederum mit einer Verbindung der Formel III zu einer Verbindung der Formel Id 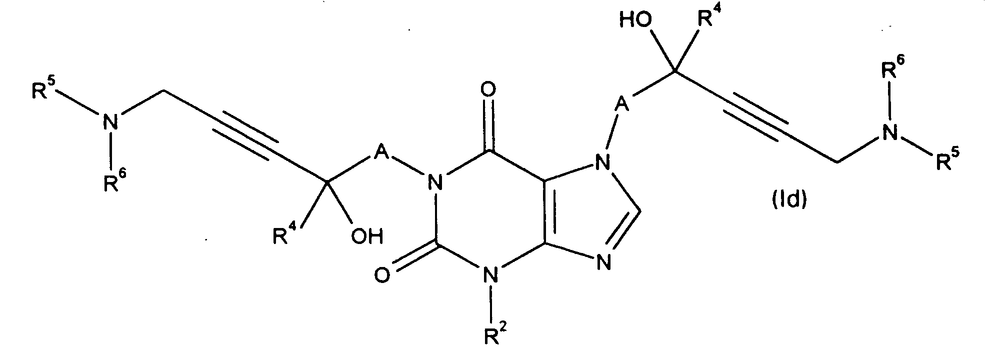 mit zwei gleichen oder verschiedenen Alkinolresten der Formel Ia für R¹ und R³ gemäß Formel I oder
mit einer Verbindung der Formel IV,
R⁸-X (IV)
worin R⁸ wie in Formel I und X wie in Formel III definiert sind,
zu einer Verbindung der Formel Ie 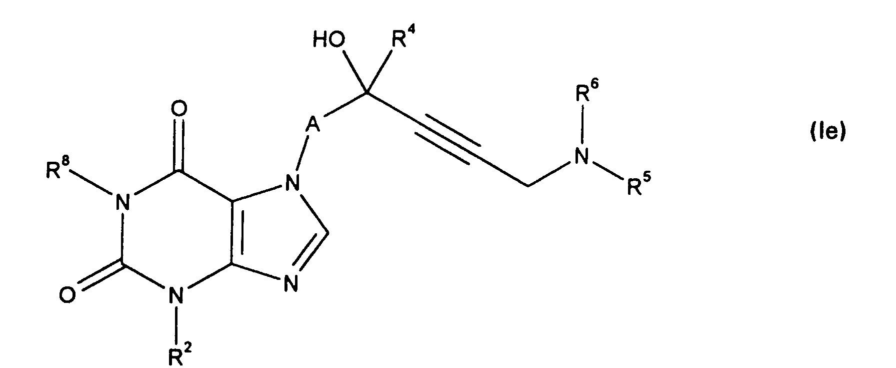 mit dem Rest R⁸ für R¹ und dem Alkinolrest der Formel Ia für R³ gemäß Formel I alkyliert wird, oder
ein 1,3-Dialkylxanthin der Formel V, 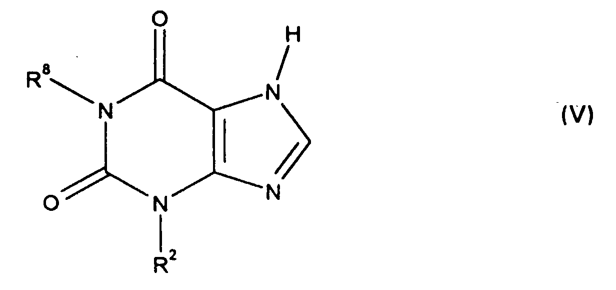 worin R² und R⁸ wie in Formel I definiert sind, ohne Kondensationsmittel oder in Gegenwart eines basischen Kondensationsmittels oder aber ein Salz der Verbindung der Formel V mit einer Verbindung der Formel III zu einer Verbindung der Formel Ie umgesetzt wird, oder
ein 3,7-Dialkylxanthin der Formel VI, 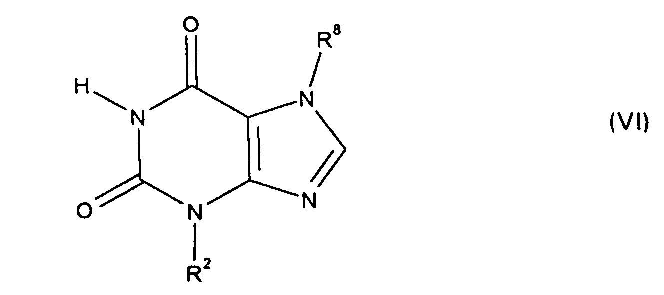 worin R² und R⁸ wie in Formel I definiert sind, ohne Kondensationsmittel oder in Gegenwart eines basischen Kondensationsmittels oder aber ein Salz der Verbindung der Formel VI mit einer Verbindung der Formel III zu einer Verbindung der Formel If 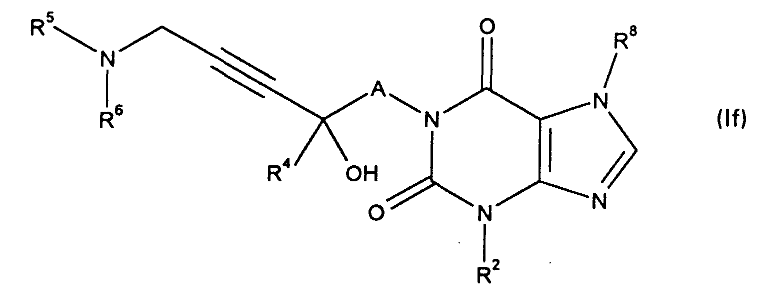 mit einem Alkinolrest der Formel Ia für R¹ und dem Rest R⁸ für R³ gemäß Formel I umgesetzt wird; oder
nach Verfahrensvariante B eine Verbindung der Formel II, V oder VI analog Verfahrensvariante A mit einer Verbindung der Formel VIII, 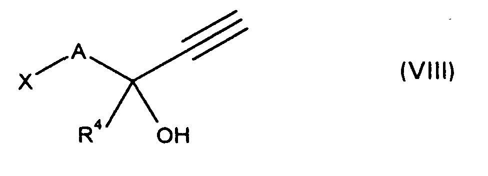 worin A und R⁴ wie in Formel I und X wie in Formel III definiert sind,
zu einer Verbindung der Formel IX 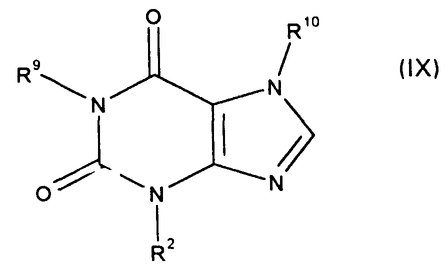 alkyliert wird, worin entweder R⁹ und R¹⁰ zwei gleiche oder verschiedene Reste der Formel IXa 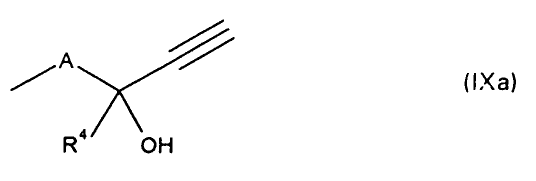 darstellen oder aber nur R⁹ oder R¹⁰ einen Rest der Formel IXa darstellt und der andere Rest R¹⁰ oder R⁹ ein Wasserstoffatom oder R⁸ darstellt, wobei R², A, R⁴ und R⁸ wie in Formel I definiert sind, und
anschließend die Verbindung der Formel IX unter den Bedingungen der Mannich-Reaktion mit Formaldehyd und einem Amin der Formel X, 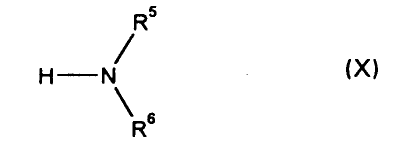 worin R⁵ und R⁶ wie in Formel I definiert sind, an der (oder den) terminalen Ethinylgruppe(n) zu einer Verbindung der Formel lc, ld, le oder If aminomethyliert wird; oder
nach Verfahrensvariante C ein 1,3- oder 3,7-di- oder 1,3,7-trisubstituiertes Xanthin der Formel XI, 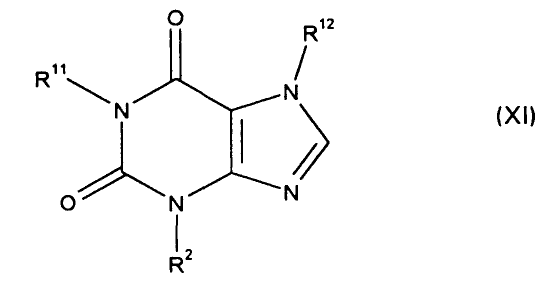 worin entweder R¹¹ und R¹² zwei gleiche oder verschiedene Reste der Formel XIa 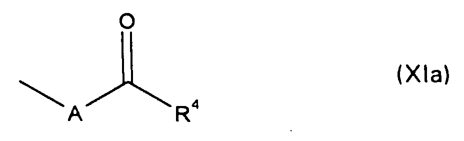 darstellen oder aber nur R¹¹ oder R¹² einen Rest der Formel XIa darstellt und der andere Rest R¹² oder R¹¹ ein Wasserstoffatom oder R⁸ darstellt, wobei R², A, R⁴ und R⁸ wie in Formel I definiert sind,
mit einer Organometall-Verbindung der Formel XII, 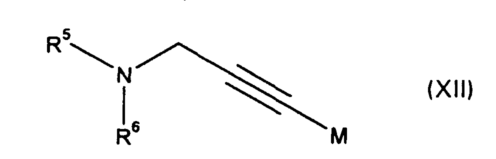 worin R⁵ und R⁶ wie in Formel I definiert sind und M ein Alkali-, Erdalkali- oder Schwermetall bedeutet,
unter reduktiver Alkinylierung der Carbonylgruppe(n) zu einer Verbindung der Formel Ic, Id, Ie, If oder Ig 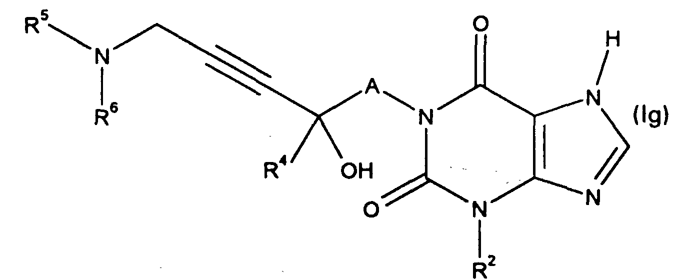 mit einem Alkinolrest der Formel Ia für R¹ und einem Wasserstoffatom für R³ gemäß Formel I umgesetzt wird; oder
nach Verfahrensvariante D ein Xanthin der Formel XI, worin R¹¹ und/oder R¹² für den Rest der Formel XIa stehen, nach Art der Nef-Reaktion entweder mit einem Acetylid der Formel XIII,
HC≡C-M (XIII)
worin M wie in Formel XII definiert ist, oder aber mit einem endständig geschützten Acetylid der Formel XIV,
R^{a}-C≡C-M (XIV)
worin M wie in Formel XII definiert ist und R^{a} eine nachträglich leicht eliminierbare Abgangsgruppe darstellt, unter Ethinylierung der Carbonylgruppe(n) zu einer Verbindung der Formel IX umgesetzt wird, worin R⁹ und/oder R¹⁰ für den Rest der Formel IXa stehen, und anschließend die erhaltene Verbindung IX durch Mannich-Reaktion mit Formaldehyd und einem Amin der Formel X analog Verfahrensvariante B zu einer Verbindung der Formel Ic, Id, le, If oder Ig aminomethyliert wird; oder
nach Verfahrensvariante E eine nach den Verfahrensvarianten A bis D hergestellte Verbindung der Formel Ic, Id, le oder If oder eine nach den Verfahrensvarianten C oder D hergestellte Verbindung der Formel Ig, in der R⁵ und/oder R⁶ jeweils ein Wasserstoffatom darstellen, ein- oder zweifach mit einem Oxoderivat (Aldehyd oder Keton) von (C₁-C₆)-Alkanen, (C₃-C₆)-Cycloalkanen, (C₄-C₈)-Cycloalkyl-alkanen oder Ar-(C₁-C₂)-alkanen reduktiv alkyliert wird; oder
nach Verfahrensvariante F eine nach den Verfahrensvarianten A bis E hergestellte Verbindung mit einer physiologisch verträglichen anorganischen oder organischen Säure HZ in ein Säureadditionssalz der Formel I umgesetzt wird, wobei R¹ und/oder R³ für den Alkinolrest der Formel Ib stehen, worin R⁷ für ein Wasserstoffatom steht, und R² wie in Formel I definiert ist; oder
nach Verfahrensvariante G eine nach den Verfahrensvarianten A bis E hergestellte Verbindung mit einem Alkylierungsmittel der Formel VII,
R⁷-Z (VII)
worin R⁷ wie in Formel I mit Ausnahme von Wasserstoff und Z wie in Formel III für X definiert sind, in ein quartäres Ammoniumsalz der Formel I umgewandelt wird, wobei R¹ und/oder R³ für den Alkinolrest der Formel Ib stehen und R² wie in Formel I definiert ist; oder
nach Verfahrensvariante H eine nach den Verfahrensvarianten A bis G hergestellte Verbindung chromatographisch oder durch fraktionierende Kristallisation in die reinen Stereoisomeren aufgetrennt wird.

8. Arzneimittel, gekennzeichnet durch den Gehalt einer therapeutisch effektiven Menge von mindestens einer Verbindung der Formel I in stereoisomerenreiner Form oder als Stereoisomerengemische gemäß einem oder mehreren der Ansprüche 1 bis 6 oder hergestellt nach Anspruch 7.

9. Arzneimittel nach Anspruch 8, dadurch gekennzeichnet, daß es zusätzlich eine effektive Menge mindestens eines Wirkstoffes aus der Gruppe Fibrinolytika, Calcium-Antagonisten, Excitatory Amino Acid-Antagonisten, Ganglioside, Phospholipase-, Cyclooxygenase- oder Lipoxygenase-Inhibitoren, PAF(plättchenaktivierender Faktor)-, Thromboxan- oder Leukotrien-Antagonisten, Sauerstoffradikalfänger, Schwermetall-Chelatoren, antiödematöse Wirkstoffe, Antikoagulantien, Thrombozytenaggregationshemmer, Serotonin-1A-Agonisten, Adenosin-Modulatoren und neurotrophe Wachstumsfaktoren oder deren Freisetzungsaktivatoren enthält.

10. Verwendung der Verbindung der Formel I gemäß einem oder mehreren der Ansprüche 1 bis 6 oder hergestellt nach Anspruch 7 für die Herstellung eines Arzneimittels zur kurativen oder prophylaktischen Behandlung von zerebrovaskulären Erkrankungen.

11. Verwendung nach Anspruch 10 für die kurative und prophylaktische Behandlung einer Erkrankung aus der Gruppe Schlaganfall, transitorische ischämische Attacken, Multiinfarktdemenz, Demenz des gemischten Typs mit vaskulärer und degenerativer (Alzheimer) Komponente, Rückenmarkschädigungen und Hirntrauma infolge von Kopfverletzungen.

12. Verwendung nach Anspruch 10 für die kurative und prophylaktische Behandlung von neuronalen Schäden nach Herzstillstand, Asphyxie, neonataler Asphyxie, Reanimation oder gefäßchirurgischen Eingriffen im Bereich der das Gehirn versorgenden Hauptarterien.

13. Verwendung gemäß einem oder mehreren der Ansprüche 10 bis 12 zur Primärprävention, Akutbehandlung und Sekundärprophylaxe zerebrovaskulärer Erkrankungen.

14. Verwendung nach einem oder mehreren der Ansprüche 10 bis 13 zur parenteralen, oralen, rektalen oder transdermalen Applikation.

15. Verfahren zur Herstellung eines Arzneimittels gemäß Anspruch 8 oder 9, dadurch gekennzeichnet, daß man mindestens eine Verbindung der Formel I gemäß einem oder mehreren der Ansprüche 1 bis 6 mit pharmazeutisch akzeptalen und physiologisch verträglichen Träger- und Zusatzstoffen, Verdünnungsmitteln und/oder anderen Wirk- und Hilfsstoffen in eine geeignete Darreichungsform bringt.

## Claims

1. A compound of the formula I, 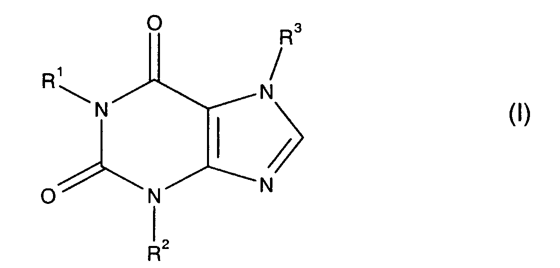 in stereoisomerically pure form or as a mixture of stereoisomers, where
1)
R¹ and R³ are an alkynol residue of the formula la or Ib, 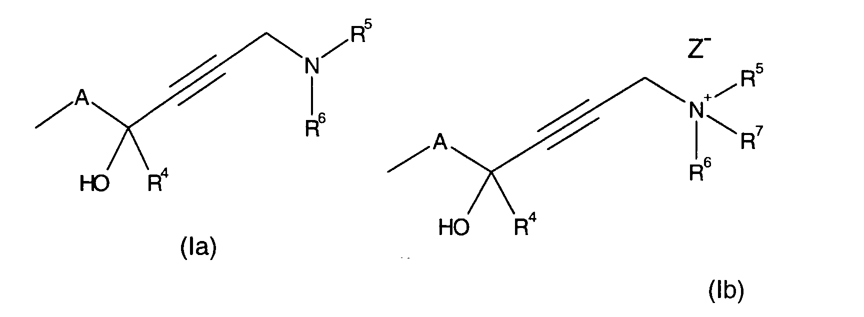
R² is
a) straight-chain or branched (C₁-C₅)-alkyl,
b) (C₃-C₆)-cycloalkyl or
c) (C₄-C₈)-cycloalkylalkyl,
R⁴ is a hydrogen atom or (C₁-C₃)-alkyl,
R⁵, R⁶ and R⁷ are, independently of one another,
a) a hydrogen atom,
b) (C₁-C₆)-alkyl,
c) (C₃-C₆)-cycloalkyl,
d) (C₄-C₈)-cycloalkylalkyl,
e) Ar-(C₁-C₂)-alkyl or
f) tri-(C₁-C₄)-alkylsilyl, or
R⁵ and R⁶ form, together with the nitrogen atom to which they are bonded, a 4- to 7-membered saturated ring which is unsubstituted or substituted once to four times by (C₁-C₄)-alkyl, or
R⁵ and R⁶ form, together with the nitrogen atom to which they are bonded, a 4- to 7-membered saturated ring in which one ring -CH₂-group is replaced by a radical from the group of O, S, SO, SO₂ and NR¹³,
R¹³ is a hydrogen atom, (C₁-C₃)-alkylcarbonyl or (C₁-C₄)-alkyl,
and the ring is unsubstituted or substituted once to four times by (C₁-C₄)-alkyl,
A is unbranched or branched (C₁-C₆)-alkylene, and
Z⁻ is the anion of a physiologically tolerated inorganic or organic acid, or
2) R¹ or R³ is an alkynol residue of the formula la or lb, and the other radical R³ or R¹ is
a) a hydrogen atom or
b) R⁸,
in which R⁸ is straight-chain or branched (C₁-C₆)-alkyl, (C₃-C₆)-cycloalkyl or (C₄-C₈)-cycloalkylalkyl, and
R², R⁴, R⁵, R⁶, R⁷, A and Z⁻ are as defined under 1).

2. A compound of the formula I as claimed in claim 1, wherein only one of the two radicals R¹ or R³ is an alkynol residue of the formula la or Ib, and the other radical is a hydrogen atom or R⁸.

3. A compound of the formula I as claimed in claim 1 or 2, wherein R¹ is an alkynol residue of the formula la or Ib and R³ is a hydrogen atom or R⁸.

4. A compound of the formula I as claimed in one or more of claims 1 to 3, where
R¹ is an alkynol residue of the formula la or lb,
R² is straight-chain (C₁-C₄)-alkyl, cyclopropyl or cyclopropylmethyl,
R³ is a) a hydrogen atom or b) R⁸,
in which R⁸ is straight-chain or branched (C₁-C₆)-alkyl, cyclopropyl or cyclopropylmethyl,
R⁴ is a hydrogen atom, methyl or ethyl,
R⁵, R⁶ and R⁷ are, independently of one another, a hydrogen atom, (C₁-C₄)-alkyl, cyclopropyl, cyclopropylmethyl or benzyl, or
R⁵ and R⁶ form, together with the nitrogen atom to which they are bonded, a 5- to 6-membered saturated ring from the group of morpholine, 4-(C₁-C₃)-alkylcarbonylpiperazine, 4-(C₁-C₂)-alkylpiperazine, piperazine, piperidine, pyrrolidine and thiomorpholine,
A is unbranched (C₁-C₅)-alkylene, and
Z⁻ is the anion of a physiologically tolerated inorganic or organic acid.

5. A compound of the formula I as claimed in claim 4, where
R¹ is an alkynol residue of the formula la or lb,
R² is (C₁-C₄)-alkyl,
R³ is straight-chain (C₂-C₄)-alkyl or cyclopropyl,
R⁴ is a hydrogen atom or methyl,
R⁵, R⁶ and R⁷ are, independently of one another, a hydrogen atom, (C₁-C₄)-alkyl or benzyl, or
R⁵ and R⁶ form, together with the nitrogen atom to which they are bonded, the morpholine, pyrrolidine, piperidine, 4-methylpiperazine or 4-acetylpiperazine ring,
A is unbranched (C₂-C₄)-alkylene, and
Z⁻ is the anion of a physiologically tolerated inorganic or organic acid.

6. A compound of the formula I as claimed in claim 5, which is 1-(8-diethylamino-5-hydroxy-5-methyl-6-octynyl)-3-methyl-, 1-(5-hydroxy-5-methyl-8-pyrrolidino-6-octynyl)-3-methyl-, 3-butyl-1-(5-hydroxy-5-methyl-8-piperidino-6-octynyl)-,1-(5-diethylamino-2-hydroxy-2-methyl-3-pentynyl)-3-propyl-, 1-(6-dimethylamino-3-hydroxy-3-methyl-4-hexynyl)-3-ethyl-, 1-(7-diethylamino-4-hydroxy-4-methyl-5-heptynyl)-3-ethyl-, 1-[8-(4-acetylpiperazino)-5-hydroxy-5-methyl-6-octynyl]-3-methyl-7-propylxanthine and their physiologically tolerated acid addition salts or N,N-diethyl-N-[4-hydroxy-4-methyl-8-(3-methyl-7-propyl-1-xanthinyl)-2-octynyl]-N-methylammonium iodide.

7. A process for preparing the compound of the formula I as claimed in one or more of claims 1 to 6, which comprises in process variant A reacting a 3-alkylxanthine of the formula II, 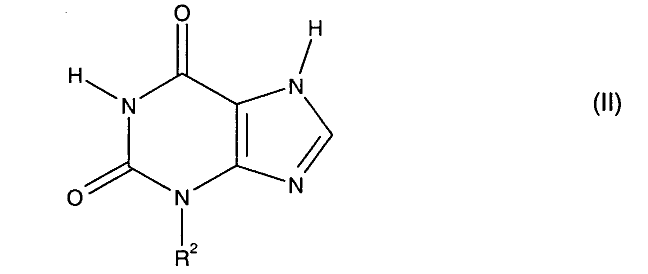 in which R² is as defined in formula I, is reacted, without condensing agent or in the presence of a basic condensing agent or of a salt of the compound of the formula II, with a compound of the formula III, 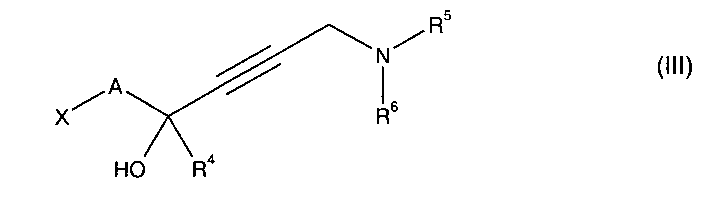 in which X is chlorine, bromine, iodine or a sulfonic ester or phosphoric ester residue, and A, R⁴, R⁵ and R⁶ are as defined in formula I, to give a compound of the formula Ic 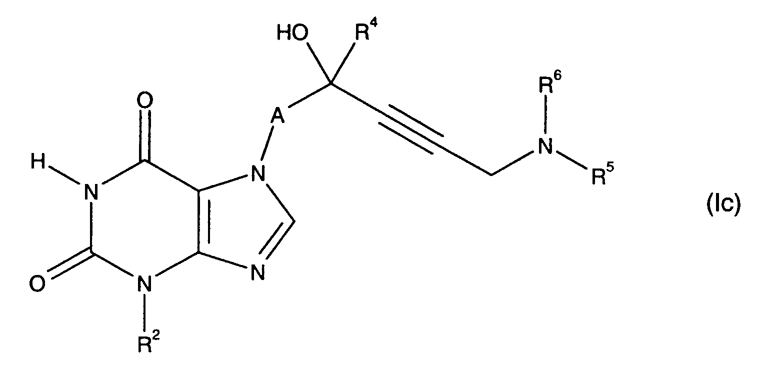 with an alkynol residue of the formula la for R³ and a hydrogen atom for R¹ according to formula I, and subsequently alkylating the compound of the formula Ic without a condensing agent or in the presence of a basic condensing agent or else a salt of the compound of the formula Ic either once again with a compound of the formula III to give a compound of the formula Id 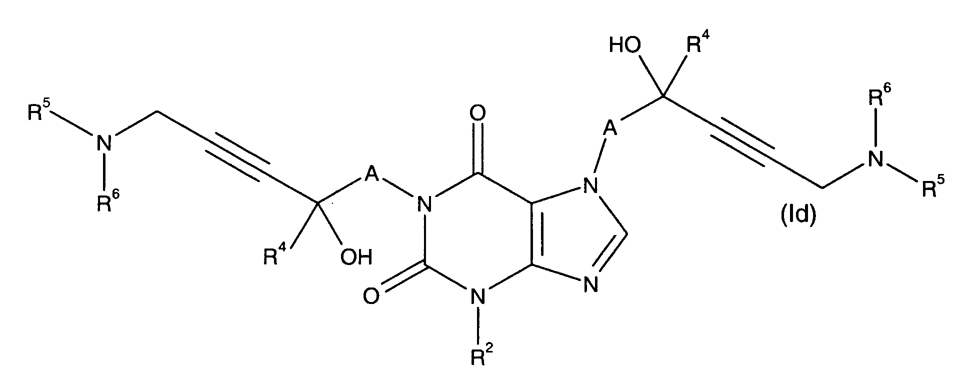 with two identical or different alkynol residues of the formula la for R¹ and R³ according to formula I or
with a compound of the formula IV,
R⁸-X (IV)
in which R⁸ is as defined in formula I and X is as defined in formula III, to give a compound of the formula le 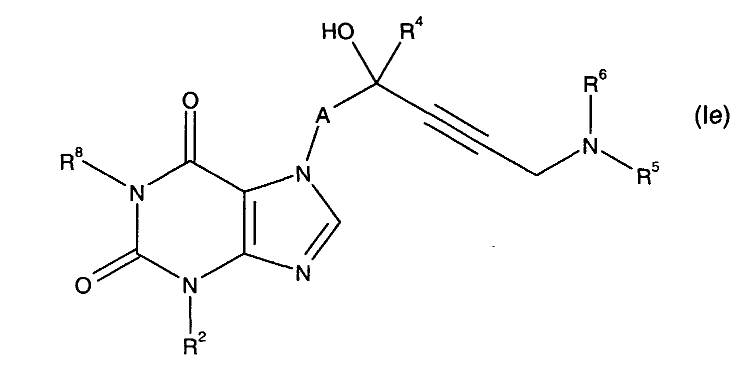 with the radical R⁸ for R¹ and the alkynol residue of the formula la for R³ according to formula I, or
a 1,3-dialkylxanthine of the formula V, 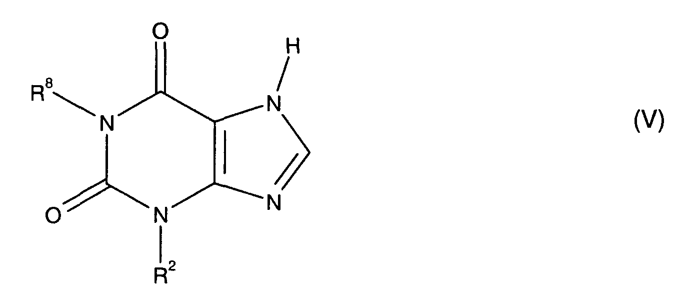 in which R² and R⁸ are as defined in formula I, is reacted without condensing agent or in the presence of a basic condensing agent or else a salt of the compound of the formula V with a compound of the formula III to give a compound of the formula le, or
a 3,7-dialkylxanthine of the formula VI, 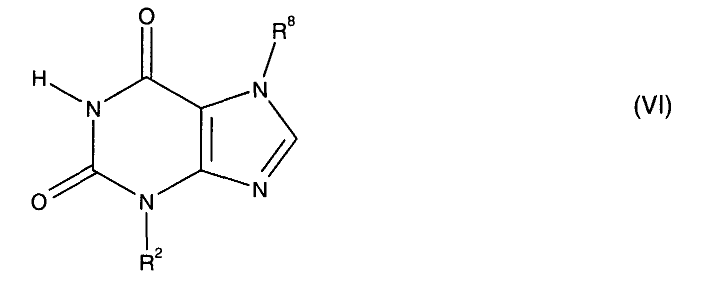 in which R² and R⁸ are as defined in formula I, is reacted without condensing agent or in the presence of a basic condensing agent or else a salt of the compound of the formula VI with a compound of the formula III to give a compound of the formula If 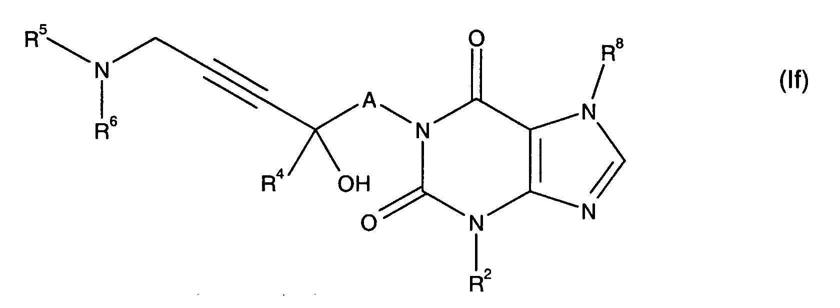 with an alkynol residue of the formula la for R¹ and the radical R⁸ for R³ according to formula I; or
in process variant B, a compound of the formula II, V or VI is alkylated in analogy to process variant A with a compound of the formula VIII, 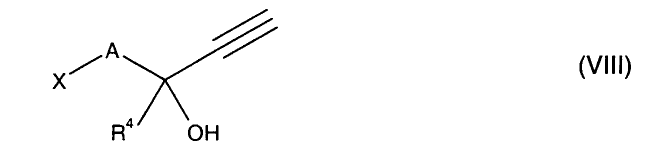 in which A and R⁴ are as defined in formula I and X is as defined in formula III, to give a compound of the formula IX 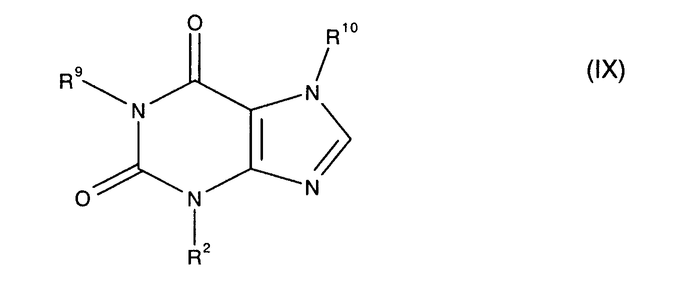 in which either R⁹ and R¹⁰ are two identical or different radicals of the formula IXa 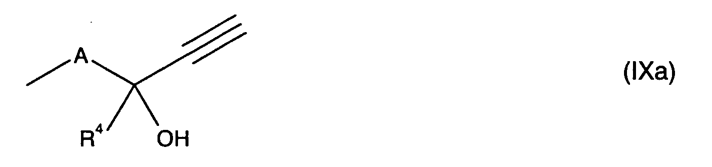 or else only R⁹ or R¹⁰ is a radical of the formula IXa, and the other radical R¹⁰ or R⁹ is a hydrogen atom or R⁸, where R², A, R⁴ and R⁸ are as defined in formula I, and
subsequently the compound of the formula IX is aminomethylated under the conditions of the Mannich reaction with formaldehyde and an amine of the formula X, 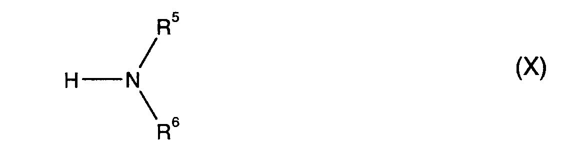 in which R⁵ and R⁶ are as defined in formula I, on the terminal ethynyl group(s) to give a compound of the formula Ic, Id, le or If; or
in process variant C, a 1,3- or 3,7-di- or 1,3,7-trisubstituted xanthine of the formula XI, 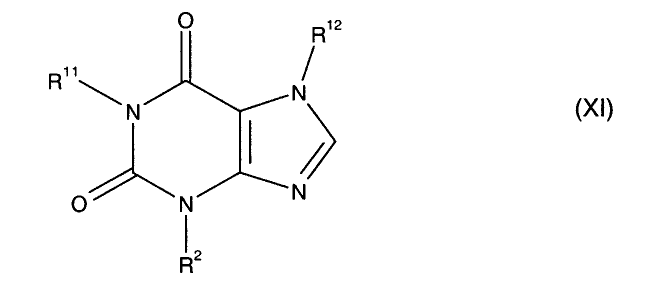 in which either R¹¹ and R¹² are two identical or different radicals of the formula Xla 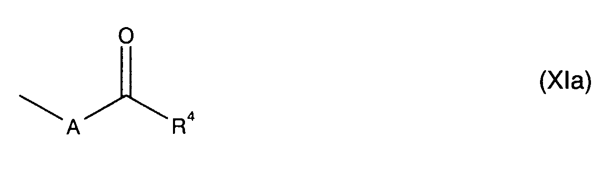 or else only R¹¹ or R¹² is a radical of the formula Xla, and the other radical R¹² or R¹¹ is a hydrogen atom or R⁸, where R², A, R⁴ and R⁸ are as defined in formula I,
is reacted with an organometallic compound of the formula XII, 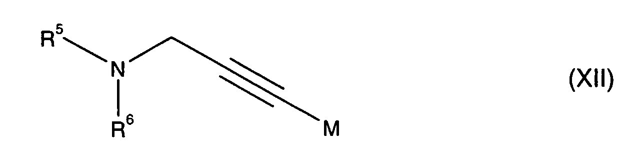 in which R⁵ and R⁶ are as defined in formula I and M is an alkali metal, alkaline earth metal or heavy metal,
with reductive alkynylation of the carbonyl group(s) to give a compound of the formula Ic, Id, le, If or Ig 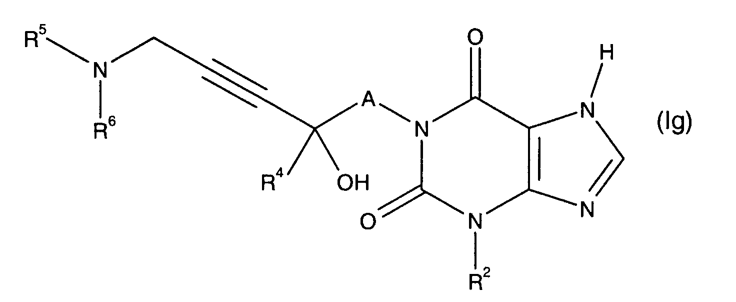 with an alkynol residue of the formula la for R¹ and a hydrogen atom for R³ according to formula I; or
in process variant D, a xanthine of the formula XI, in which R¹¹ and/or R¹² are the radical of the formula XIa is reacted in a reaction of the Nef type either with an acetylide of the formula XIII,
HC≡C-M (XIII)
in which M is as defined in formula XII, or else with a terminally protected acetylide of the formula XIV,
R^{a}-C≡C-M (XIV)
in which M is as defined in formula XII and R^{a} is a leaving group which can subsequently be easily eliminated, with ethynylation of the carbonyl group(s) to give a compound of the formula IX in which R⁹ and/or R¹⁰ are the radical of the formula IXa, and subsequently the resulting compound IX is aminomethylated by a Mannich reaction with formaldehyde and an amine of the formula X in analogy to process variant B to give a compound of the formula Ic, Id, le, If or Ig; or
in process variant E, a compound of the formula Ic, Id, le or If prepared in process variants A to D, or a compound of the formula Ig prepared in process variants C or D, in which R⁵ and/or R⁶ are each a hydrogen atom, is reductively alkylated once or twice with an oxo derivative (aldehyde or ketone) of (C₁-C₆)alkanes, (C₃-C₆)cycloalkanes, (C₄-C₈)cycloalkylalkanes or Ar-(C₁-C₂)alkanes; or
in process variant F, a compound prepared in process variants A to E is converted with a physiologically tolerated inorganic or organic acid HZ into an acid addition salt of the formula I, where R¹ and/or R³ are the alkynol residue of the formula Ib, in which R⁷ is a hydrogen atom and R² is as defined in formula I; or
in process variant G, a compound prepared in process variants A to E is converted with an alkylating agent of the formula VII,
R⁷-Z (VII)
where R⁷ is as defined in formula I with the exception of hydrogen, and Z is as defined in formula III for X, into a quaternary ammonium salt of the formula I, where R¹ and/or R³ are the alkynol residue of the formula lb and R² is as defined in formula I; or
in process variant H, a compound prepared in process variants A to G is fractionated into the pure stereoisomers by chromatography or by fractional crystallization.

8. A pharmaceutical which comprises a therapeutically effective amount of at least one compound of the formula I in stereoisomerically pure form or as a mixture of stereoisomers as claimed in one or more of claims 1 to 6 or prepared as claimed in claim 7.

9. A pharmaceutical as claimed in claim 8, which additionally comprises an effective amount of at least one active substance from the group of fibrinolytics, calcium antagonists, excitatory amino acid antagonists, gangliosides, phospholipase, cyclooxygenase or lipoxygenase inhibitors, PAF (platelet activating factor), thromboxane or leukotriene antagonists, oxygen free radical scavengers, heavy metal chelators, antiedematous active substances, anticoagulants, platelet aggregation inhibitors, serotonin 1A agonists, adenosine modulators and neurotrophic growth factors or their release activators.

10. The use of the compound of the formula I as claimed in one or more of claims 1 to 6 or prepared as claimed in claim 7 for producing a pharmaceutical for the curative or prophylactic treatment of cerebrovascular disorders.

11. The use as claimed in claim 10 for the curative and prophylatic treatment of a disorder from the group of stroke, transient ischemic attacks, multiinfarct dementia; dementia of the mixed type with vascular and degenerative (Alzheimer) components, spinal cord damage and brain trauma as a result of head injuries.

12. The use as claimed in claim 10 for the curative and prophylactic treatment of neuronal damage after cardiac arrest, asphyxia, neonatal asphyxia, resuscitation or vascular surgical operations in the region of the main arteries supplying the brain.

13. The use as claimed in one or more of claims 10 to 12 for the primary prevention, acute treatment and secondary prophylaxis of cerebrovascular disorders.

14. The use as claimed in one or more of claims 10 to 13 for parenteral, oral, rectal or transdermal administration.

15. The process for producing a pharmaceutical as claimed in claim 8 or 9, which comprises converting at least one compound of the formula I as claimed in one or more of claims 1 to 6 with pharmaceutically acceptable and physiologically tolerated excipients and additives, diluents and/or other active substances and auxiliaries into a suitable dosage form.

## Revendications

1. Composé de formule I 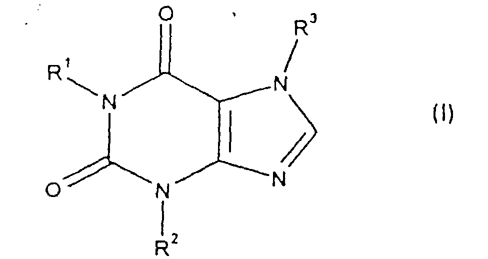 sous forme de stéréoisomère pur ou de mélanges de stéréoisomères, formule dans laquelle
1) R¹ et R³ représentent un radical alcynol de formule Ia ou Ib 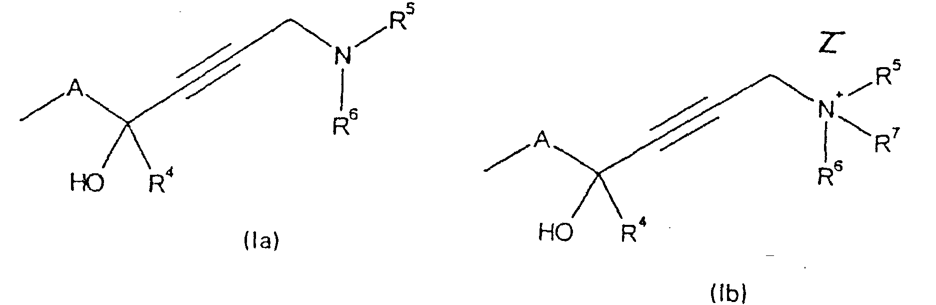
R² représente
a) un groupe alkyle en C₁-C₅ à chaîne droite ou ramifié,
b) un groupe cycloalkyle en C₃-C₆ ou
c) un groupe cycloalkyl-alkyle en C₄-C₈,
R⁴ représente un atome d'hydrogène ou un groupe alkyle en C₁-C₃,
R⁵, R⁶ et R⁷ représentent, indépendamment les uns des autres,
a) un atome d'hydrogène,
b) un groupe alkyle en C₁-C₆,
c) un groupe cycloalkyle en C₃-C₆,
d) un groupe cycloalkyl-alkyle en C₄-C₈,
e) un groupe Ar-alkyle(C₁-C₂) ou
f) un groupe trialkyl(C₁-C₄)silyle, ou
R⁵ et R⁶ forment ensemble, avec l'atome d'azote auquel ils sont fixés, un cycle saturé à 4-7 chaînons, qui est non substitué ou substitué 1 à 4 fois par un ou des groupes alkyle en C₁-C₄, ou
R⁵ et R⁶ forment ensemble, avec l'atome d'azote auquel ils sont fixés, un cycle saturé à 4-7 chaînons, dans lequel un groupe CH₂ du cycle est remplacé par un radical choisi parmi O, S, SO, SO₂ et NR¹³,
R¹³ représente un atome d'hydrogène ou un groupe alkyl(C₁-C₃)-carbonyle ou alkyle en C₁-C₄,
et le cycle est non substitué ou 1 à 4 fois substitué par un ou des groupes alkyle en C₁-C₄,
A représente un groupe alkylène en C₁-C₆ ramifié ou non ramifié, et
Z⁻ représente l'anion d'un acide organique ou minéral physiologiquement acceptable, ou
2) R¹ ou R³ représente un radical alcynol de formule Ia ou Ib et l'autre radical R³ ou R¹ représente
a) un atome d'hydrogène ou
b) R⁸,
R⁸ représentant un groupe alkyle en C₁-C₆ à chaîne droite ou ramifié, cycloalkyle en C₃-C₆ ou cycloalkyl-alkyle en C₄-C₈,
et R², R⁴, R⁵, R⁶, R⁷, A et Z- sont tels que définis en 1).

2. Composé de formule I selon la revendication 1, caractérisé en ce qu'un seul des deux radicaux R¹ et R³ représente un radical alcynol de formule la ou Ib, et l'autre radical représente un atome d'hydrogène ou R⁸.

3. Composé de formule I selon la revendication 1 ou 2, caractérisé en ce que R¹ représente un radical alcynol de formule la ou Ib et R³ représente un atome d'hydrogène ou R⁸.

4. Composé de formule I selon une ou plusieurs des revendications 1 à 3, dans lequel
R¹ représente un radical alcynol de formule Ia ou Ib,
R² représente un groupe alkyle en C₁-C₄ à chaîne droite, cyclopropyle ou cyclopropylméthyle,
R³ représente a) un atome d'hydrogène ou b) R⁸,
R⁸ représentant un groupe alkyle en C₁-C₆ à chaîne droite ou ramifié, cyclopropyle ou cyclopropylméthyle,
R⁴ représente un atome d'hydrogène ou le groupe méthyle ou éthyle,
R⁵, R⁶ et R⁷ représentent, indépendamment les uns des autres, un atome d'hydrogène ou un groupe alkyle en C₁-C₄, cyclopropyle, cyclopropylméthyle ou benzyle, ou
R⁵ et R⁶ forment ensemble, avec l'atome d'azote auquel ils sont fixés, un cycle saturé à 5-6 chaînons choisi parmi les cycles morpholine, 4-alkyl(C₁-C₃)carbonylpipérazine, 4-alkyl(C₁-C₂)pipérazine, pipérazine, pipéridine, pyrrolidine et thiomorpholine,
A représente un groupe alkylène en C₁-C₅ non ramifié et
Z⁻ représente l'anion d'un acide organique ou minéral physiologiquement acceptable.

5. Composé de formule I selon la revendication 4, dans lequel
R¹ représente un radical alcynol de formule la ou Ib,
R² représente un groupe alkyle en C₁-C₄,
R³ représente un groupe alkyle en C₂-C₄ à chaîne droite ou cyclopropyle,
R⁴ représente un atome d'hydrogène ou le groupe méthyle,
R⁵, R⁶ et R⁷ représentent, indépendamment les uns des autres, un atome d'hydrogène ou un groupe alkyle en C₁-C₄ ou benzyle, ou
R⁵ et R⁶ forment ensemble, avec l'atome d'azote auquel ils sont fixés, le cycle morpholine, pyrrolidine, pipéridine, 4-méthylpipérazine ou 4-acétylpipérazine,
A représente un groupe alkylène en C₂-C₄ non ramifié et
Z⁻ représente l'anion d'un acide organique ou minéral physiologiquement acceptable.

6. Composé de formule I selon la revendication 5, caractérisé en ce qu'il représente la 1-(8-diéthylamino-5-hydroxy-5-méthyl-oct-6-ynyl)-3-méthyl-, 1-(5-hydroxy-5-méthyl-8-pyrrolidino-oct-6-ynyl)-3-méthyl-, 3-butyl-1-(5-hydroxy-5-méthyl-8-pipéridino-oct-6-ynyl)-, 1-(5-diéthylamino-2-hydroxy-2-méthyl-pent-3-ynyl)-3-propyl-, 1-(6-diméthylamino-3-hydroxy-3-méthyl-hex-4-ynyl)-3-éthyl-, 1-(7-diéthylamino-4-hydroxy-4-méthyl-hept-5-ynyl)-3-éthyl-, 1-[8-(4-acétylpipérazino)-5-hydroxy-5-méthyl-oct-6-ynyl]-3-méthyl-7-propylxanthine ainsi que les sels d'addition avec des acides physiologiquement acceptables de tels composés ou l'iodure de N,N-diéthyl-N-[4-hydroxy-4-méthyl-8-(3-méthyl-7-propylxanthin-1-yl)oct-2-ynyl]-N-méthylammonium.

7. Procédé pour la préparation du composé de formule I selon une ou plusieurs des revendications 1 à 6, caractérisé en ce que selon la variante A du procédé, on fait réagir une 3-alkylxanthine de formule II 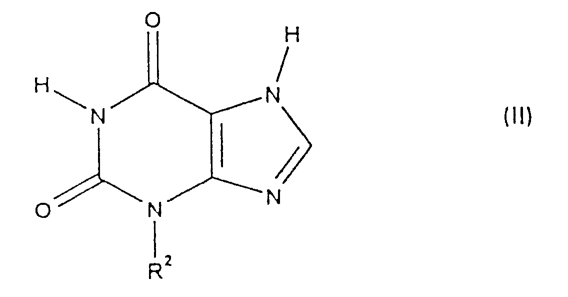 dans laquelle R² est tel que défini dans la formule I, sans agent de condensation ou en présence d'un agent de condensation basique, ou bien un sel du composé de formule II, avec un composé de formule III 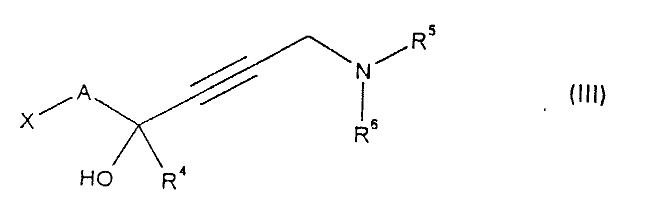 dans laquelle X représente un atome de chlore, brome ou iode, ou un reste d'ester d'acide sulfonique ou d'ester d'acide phosphorique, et A, R⁴, R⁵ et R⁶ sont tels que définis dans la formule I, pour aboutir à un composé de formule Ic 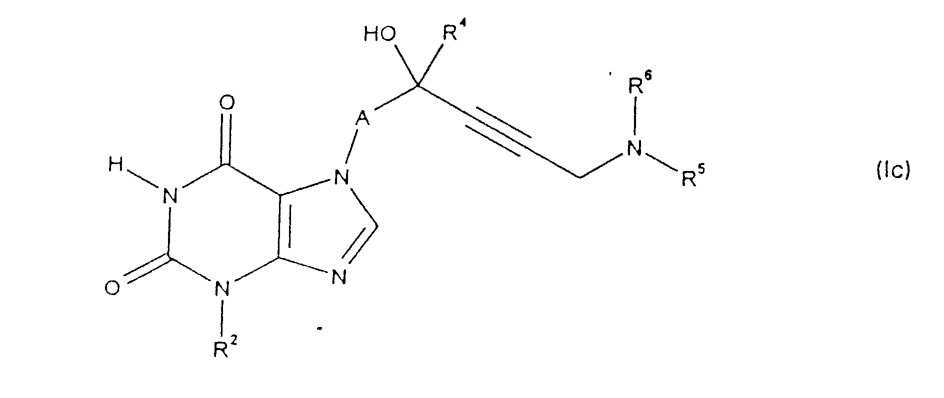 comportant un radical alcynol de formule la pour R³ et un atome d'hydrogène pour R¹, selon la formule I, et on soumet ensuite le composé de formule Ic, sans agent de condensation ou en présence d'un agent de condensation basique, ou bien un sel du composé de formule Ic, à une alkylation soit de nouveau avec un composé de formule III, pour aboutir à un composé de formule Id 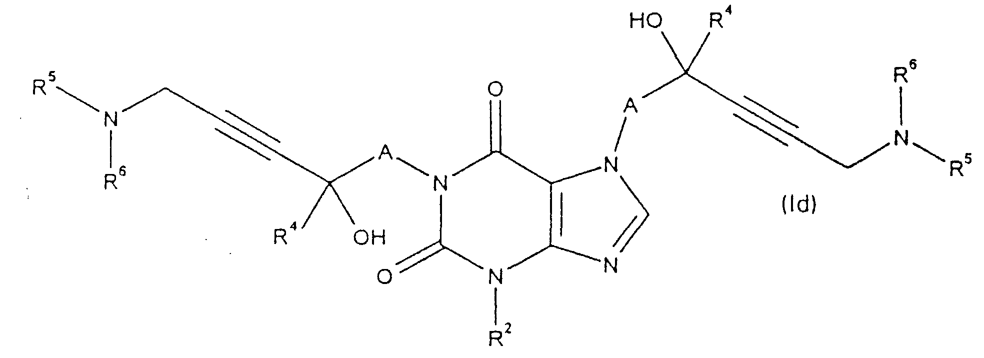 comportant deux radicaux alcynol identiques ou différents de formule la pour R¹ et R³ selon la formule I, soit
avec un composé de formule IV
R⁸-X (IV)
dans laquelle R⁸ est tel que défini dans la formule I et X est tel que défini dans la formule III, pour aboutir à un composé de formule le 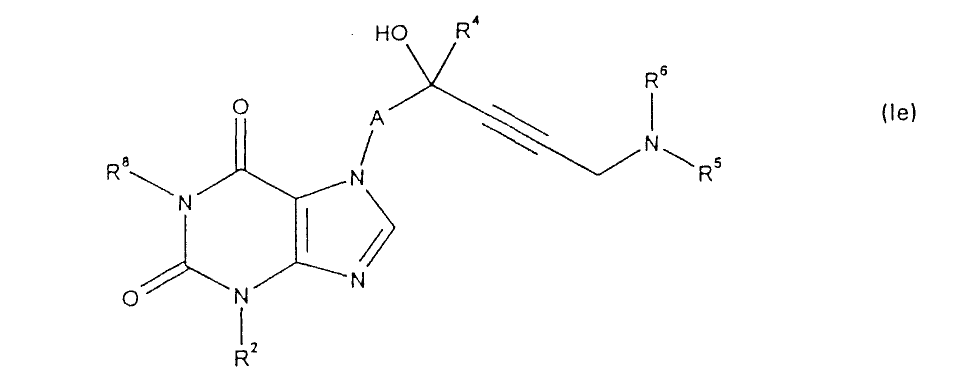 comportant le radical R⁸ pour R¹ et le radical alcynol de formule la pour R³ selon la formule I, ou on fait réagir une 1,3-dialkylxanthine de formule V 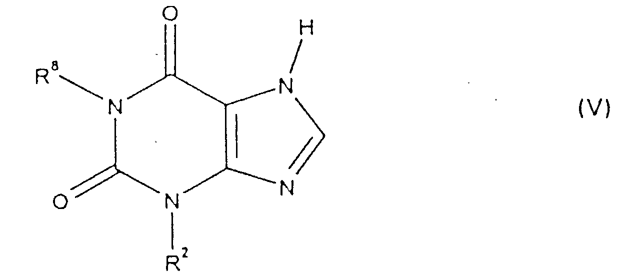 dans laquelle R² et R⁸ sont tels que définis dans la formule I, sans agent de condensation ou en présence d'un agent de condensation basique, ou bien un sel du composé de formule V, avec un composé de formule III, pour aboutir à un composé de formule le, ou
on fait réagir une 3,7-dialkylxanthine de formule VI 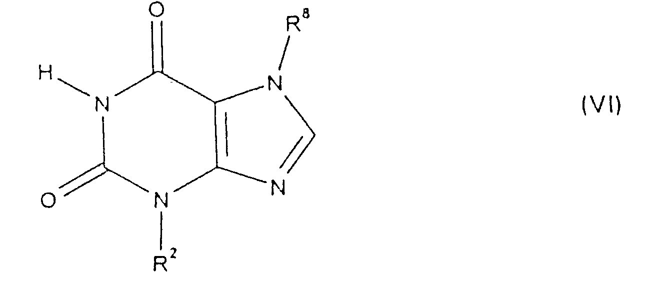 dans laquelle R² et R⁸ sont tels que définis dans la formule I, sans agent de condensation ou en présence d'un agent de condensation basique, ou bien un sel du composé de formule VI, avec un composé de formule III, pour aboutir à un composé de formule If 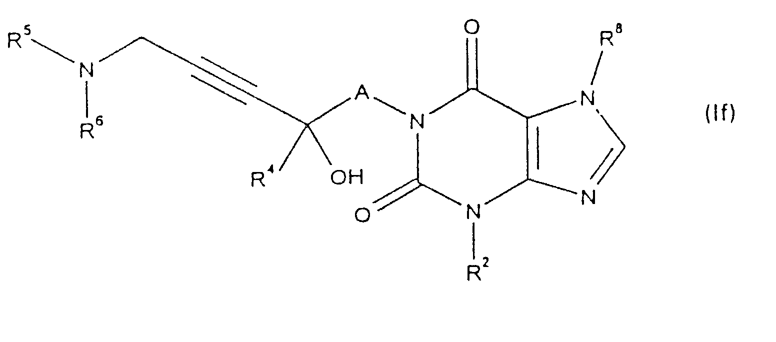 comportant un radical alcynol de formule la pour R¹ et le radical R⁸ pour R³ selon la formule I ; ou
selon la variante B du procédé, on soumet un composé de formule II, V ou VI, comme dans la variante A du procédé, à une alkylation avec un composé de formule VIII 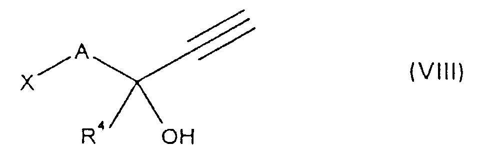 dans laquelle A et R⁴ sont tels que définis dans la formule I et X est tel que défini dans la formule III, pour aboutir à un composé de formule IX 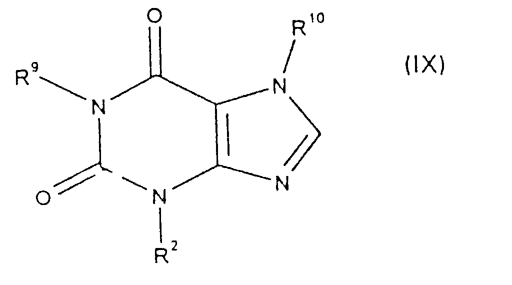 dans laquelle soit R⁹ et R¹⁰ représentent deux radicaux identiques ou différents de formule IXa 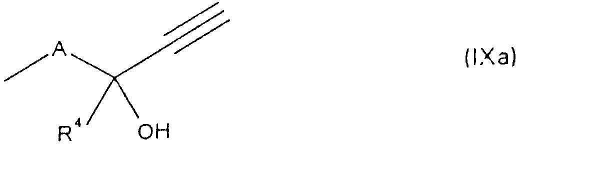 soit seul R⁹ ou R¹⁰ représente un radical de formule IXa et l'autre radical R¹⁰ ou R⁹ représente un atome d'hydrogène ou R⁸ ; R², A, R⁴ et R⁸ étant tels que définis dans la formule I, et
on soumet ensuite à une aminométhylation le composé de formule IX, dans les conditions de la réaction de Mannich, avec du formaldéhyde et une amine de formule X 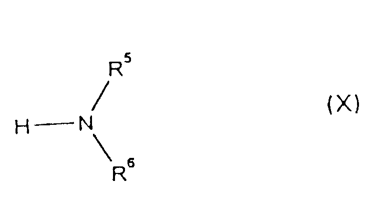 dans laquelle R⁵ et R⁶ sont tels que définis dans la formule I, au niveau du(ou des) groupe(s) éthynyle terminal(aux), pour aboutir à un composé de formule Ic, Id, le ou If ; ou
selon la variante C du procédé, on fait réagir une xanthine 1,3-substituée ou 3,7-disubstituée ou 1,3,7-trisubstituée de formule XI 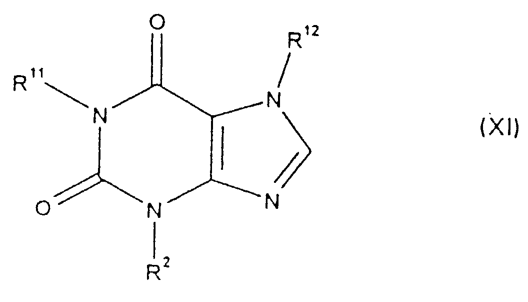 dans laquelle soit R¹ et R¹² représentent deux radicaux identiques ou différents de formule XIa 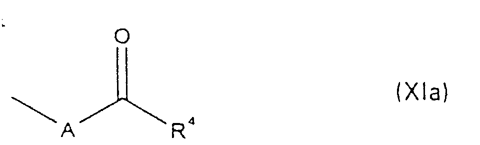 soit seul R¹¹ ou R¹² représente un radical de formule XIa et l'autre radical R¹² ou R¹¹ représente un atome d'hydrogène ou R⁸ ; R², A, R⁴ et R⁸ étant tels que définis dans la formule I,
avec un composé organométallique de formule XII 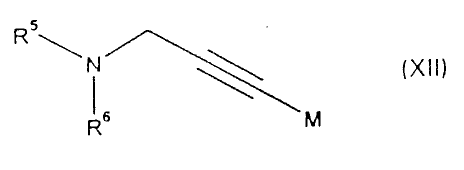 dans laquelle R⁵ et R⁶ sont tels que définis dans la formule I et M représente un métal alcalin, alcalino-terreux ou un métal lourd ; avec alcynylation réductrice du(des) groupe(s) carbonyle, pour aboutir à un composé de formule Ic, Id, Ie, If ou Ig 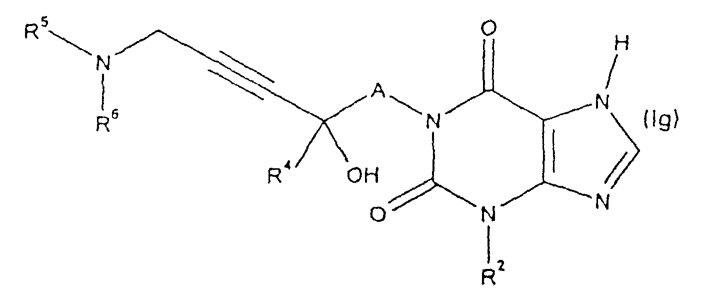 comportant un radical alcynol de formule Ia pour R¹ et un atome d'hydrogène pour R³ selon la formule I; ou
selon la variante D du procédé, on fait réagir une xanthine de formule XI, dans laquelle R¹¹ et/ou R¹² représentent le radical de formule XIa, selon le type de la réaction de Nef, soit avec un acétylure de formule XIII
HC≡C-M (XIII)
dans laquelle M est tel que défini dans la formule XII, soit
avec un acétylure protégé en bout de chaîne de formule XIV
R^{a}-C≡C-M (XIV)
dans laquelle M est tel que défini dans la formule XII et R^{a} représente un groupe partant pouvant être aisément éliminé ultérieurement, avec éthynylation du(des) groupe(s) carbonyle, pour aboutir à un composé de formule IX dans lequel R⁹ et/ou R¹⁰ représentent le radical de formule IXa, et on soumet ensuite à une aminométhylation le composé IX obtenu, au moyen d'une réaction de Mannich avec du formaldéhyde et une amine de formule X, comme dans la variante B du procédé, pour aboutir à un composé de formule Ic, Id, Ie, If ou Ig ; ou
selon la variante E du procédé, on soumet à une alkylation réductrice un composé de formule Ic, Id, Ie ou If, préparé selon les variantes A à D du procédé, ou un composé de formule Ig préparé selon la variante C ou D du procédé, dans lequel R⁵ et/ou R⁶ représentent chacun un atome d'hydrogène, une ou deux fois avec un dérivé oxo (aldéhyde ou cétone) d'alcanes en C₁-C₆, cycloalcanes en C₃-C₆, cycloalkylalcanes en C₄-C₈ ou aralcanes(C₁-C₂) ; ou
selon la variante F du procédé, on convertit un composé préparé selon les variantes A à E du procédé, à l'aide d'un acide organique ou minéral HZ physiologiquement acceptable, en un sel d'addition avec un acide de formule I, dans lequel R¹ et/ou R³ représentent le radical alcynol de formule Ib, dans lequel R⁷ représente un atome d'hydrogène, et R² est tel que défini dans la formule I ;
selon la variante G du procédé, on convertit un composé préparé selon les variantes A à E du procédé, à l'aide d'un agent d'alkylation de formule VII
R⁷-Z (VII)
dans laquelle R⁷ est tel que défini dans la formule I, à l'exception d'un atome d'hydrogène, et Z est tel que défini dans la formule III pour X, en un sel d'ammonium quaternaire de formule I, dans lequel R¹ et/ou R³ représentent le radical alcynol de formule Ib, et R² est tel que défini dans la formule I ; ou
selon la variante H du procédé, on scinde en les stéréoisomères purs, par chromatographie ou par cristallisation fractionnée, un composé préparé selon les variantes A à G du procédé.

8. Médicament, caractérisé par la teneur en une quantité thérapeutiquement efficace d'au moins un composé de formule I, sous forme de stéréoisomère pur ou de mélanges de stéréoisomères, selon une ou plusieurs des revendications 1 à 6 ou préparé selon la revendication 7.

9. Médicament selon la revendication 8, caractérisé en ce qu'il contient en outre une quantité efficace d'au moins une substance active choisie dans le groupe des fibrinolytiques, antagonistes du calcium, antagonistes d'aminoacides excitateurs, gangliosides, inhibiteurs de phospholipase, cyclo-oxygénase ou lipoxygénase, antagonistes de PAF (facteur d'activation des plaquettes), thromboxanes ou leucotriènes, capteurs de radicaux oxygénés, chélateurs de métaux lourds, substances actives anti-oedémateuses, anticoagulants, inhibiteurs de l'agrégation des thrombocytes, agonistes de la sérotonine 1A, modulateurs d'adénosine et facteurs de croissance neurotropes ou leurs activateurs de libération.

10. Utilisation du composé de formule I selon une ou plusieurs des revendications 1 à 6 ou préparé selon la revendication 7, pour la fabrication d'un médicament destiné au traitement curatif ou prophylactique de maladies cérébrovasculaires.

11. Utilisation selon la revendication 10, pour le traitement curatif et prophylactique d'une maladie choisie dans l'ensemble constitué par l'accident cérébral, les attaques ischémiques transitoires, la démence à infarctus multiples, la démence du type mixte à composante vasculaire et dégénérative (Alzheimer), les lésions de la moelle épinière et le traumatisme cérébral faisant suite à des blessures de la tête.

12. Utilisation selon la revendication 10, pour le traitement curatif et prophylactique d'altérations neuronales après arrêt du coeur, asphyxie, asphyxie néonatale, réanimation ou interventions de chirurgie vasculaire dans la région des artères principales alimentant le cerveau.

13. Utilisation selon une ou plusieurs des revendications 10 à 12, pour la prévention primaire, le traitement aigu et la prophylaxie secondaire de maladies cérébrovasculaires.

14. Utilisation selon une ou plusieurs des revendications 10 à 13, pour l'administration parentérale, orale, rectale ou transdermique.

15. Procédé pour la fabrication d'un médicament selon la revendication 8 ou 9, caractérisé en ce que l'on met sous une forme d'administration appropriée au moins un composé de formule I selon une ou plusieurs des revendications 1 à 6, avec des véhicules et additifs, diluants et/ou autres principes actifs et adjuvants pharmaceutiquement acceptables et physiologiquement compatibles.
